# EUROPEAN PATENT APPLICATION

(11) **EP 3 903 817 A1**
(43) Date of publication of application: **03.11.2021**
(21) Application number: 19905033.7
(22) Date of filing: 27.12.2019
(51) Int. Cl.: A61K 39/395, A61P 35/00, C07K 16/24, C07K 16/28, C07K 16/46, C12P 21/08, C12N 15/13, C12N 15/62, C12N 15/63

(54) **NOVEL ANTI-CCR8 ANTIBODY**

(30) Priority: 27.12.2018 JP 2018245044; 29.05.2019 JP 2019099923
(71) Applicant: Shionogi & Co., Ltd., Osaka 541-0045 (JP); Osaka University, Suita-shi, Osaka 565-0871 (JP)
(72) Inventor: YOSHIKAWA Mai, Toyonaka-shi, Osaka 561-0825 (JP); TAKAHASHI Tatsuya, Toyonaka-shi, Osaka 561-0825 (JP); ONODA Junji, Toyonaka-shi, Osaka 561-0825 (JP); IMAI Sunao, Toyonaka-shi, Osaka 561-0825 (JP); NAKAMURA Etsuo, Toyonaka-shi, Osaka 561-0825 (JP); FURUKAWA Kentaro, Toyonaka-shi, Osaka 561-0825 (JP); MIYAUCHI Tsuguo, Toyonaka-shi, Osaka 561-0825 (JP); YOSHIDA Tetsuya, Toyonaka-shi, Osaka 561-0825 (JP); NAGIRA Morio, Toyonaka-shi, Osaka 561-0825 (JP); TANAKA Atsushi, Suita-shi, Osaka 565-0871 (JP); OHKURA Naganari, Suita-shi, Osaka 565-0871 (JP); SAKAGUCHI Shimon, Suita-shi, Osaka 565-0871 (JP); WADA Hisashi, Suita-shi, Osaka 565-0871 (JP); KAWASHIMA Atsunari, Suita-shi, Osaka 565-0871 (JP)
(74) Representative: Mewburn Ellis LLP
(86) International application number: PCT/JP2019/051603
(87) International publication number: WO 2020/138489

(57) **Abstract**

Disclosed is a novel anti-CCR8 antibody. The antibody can be used for treating or preventing cancers or the like.

### [SEQUENCE LISTING]

SEQUENCE LISTING

<110>  SHIONOGI & CO., LTD.
       OSAKA UNIVERSITY

<120>  Novel anti-CCR8 antibody

<130>  19P00089WO

<150>  JP2018-245044
<151>  2018-12-27

<150>  JP2019-099923
<151>  2019-05-29

<160>  59

<170>  PatentIn version 3.5

<210>  1
<211>  355
<212>  PRT
<213>  Homo sapiens

<400>  1

Met Asp Tyr Thr Leu Asp Leu Ser Val Thr Thr Val Thr Asp Tyr Tyr
1               5                   10                  15

Tyr Pro Asp Ile Phe Ser Ser Pro Cys Asp Ala Glu Leu Ile Gln Thr
            20                  25                  30

Asn Gly Lys Leu Leu Leu Ala Val Phe Tyr Cys Leu Leu Phe Val Phe
        35                  40                  45

Ser Leu Leu Gly Asn Ser Leu Val Ile Leu Val Leu Val Val Cys Lys
    50                  55                  60

Lys Leu Arg Ser Ile Thr Asp Val Tyr Leu Leu Asn Leu Ala Leu Ser
65                  70                  75                  80

Asp Leu Leu Phe Val Phe Ser Phe Pro Phe Gln Thr Tyr Tyr Leu Leu
                85                  90                  95

Asp Gln Trp Val Phe Gly Thr Val Met Cys Lys Val Val Ser Gly Phe
            100                 105                 110

Tyr Tyr Ile Gly Phe Tyr Ser Ser Met Phe Phe Ile Thr Leu Met Ser
        115                 120                 125

Val Asp Arg Tyr Leu Ala Val Val His Ala Val Tyr Ala Leu Lys Val
    130                 135                 140

Arg Thr Ile Arg Met Gly Thr Thr Leu Cys Leu Ala Val Trp Leu Thr

145                 150                 155                 160

Ala Ile Met Ala Thr Ile Pro Leu Leu Val Phe Tyr Gln Val Ala Ser
                165                 170                 175

Glu Asp Gly Val Leu Gln Cys Tyr Ser Phe Tyr Asn Gln Gln Thr Leu
            180                 185                 190

Lys Trp Lys Ile Phe Thr Asn Phe Lys Met Asn Ile Leu Gly Leu Leu
        195                 200                 205

Ile Pro Phe Thr Ile Phe Met Phe Cys Tyr Ile Lys Ile Leu His Gln
    210                 215                 220

Leu Lys Arg Cys Gln Asn His Asn Lys Thr Lys Ala Ile Arg Leu Val
225                 230                 235                 240

Leu Ile Val Val Ile Ala Ser Leu Leu Phe Trp Val Pro Phe Asn Val
                245                 250                 255

Val Leu Phe Leu Thr Ser Leu His Ser Met His Ile Leu Asp Gly Cys
            260                 265                 270

Ser Ile Ser Gln Gln Leu Thr Tyr Ala Thr His Val Thr Glu Ile Ile
        275                 280                 285

Ser Phe Thr His Cys Cys Val Asn Pro Val Ile Tyr Ala Phe Val Gly
    290                 295                 300

Glu Lys Phe Lys Lys His Leu Ser Glu Ile Phe Gln Lys Ser Cys Ser
305                 310                 315                 320

Gln Ile Phe Asn Tyr Leu Gly Arg Gln Met Pro Arg Glu Ser Cys Glu
                325                 330                 335

Lys Ser Ser Ser Cys Gln Gln His Ser Ser Arg Ser Ser Ser Val Asp
            340                 345                 350

Tyr Ile Leu
        355

<210>  2
<211>  16
<212>  PRT
<213>  Artificial Sequence

<220>
<223>  CDR1 of 10A11 VL

<400>  2

Arg Ser Ser Lys Ser Leu Leu His Ser Asn Gly Asn Thr Tyr Leu Tyr
1               5                   10                  15


<210>  3
<211>  7
<212>  PRT
<213>  Artificial Sequence

<220>
<223>  CDR2 of 10A11 VL

<400>  3

Arg Met Ser Asn Leu Ala Ser
1               5


<210>  4
<211>  9
<212>  PRT
<213>  Artificial Sequence

<220>
<223>  CDR3 of 10A11 VL

<400>  4

Met Gln His Leu Glu Tyr Pro Leu Thr
1               5


<210>  5
<211>  5
<212>  PRT
<213>  Artificial Sequence

<220>
<223>  CDR1 of 10A11 VH

<400>  5

Thr Tyr Ala Leu Tyr
1               5


<210>  6
<211>  19
<212>  PRT
<213>  Artificial Sequence

<220>
<223>  CDR2 of 10A11 VH

<400>  6

Arg Ile Arg Ser Lys Ser Asn Asn Tyr Ala Thr Tyr Tyr Ala Asp Ser
1               5                   10                  15


Val Lys Asp

<210>  7
<211>  14
<212>  PRT
<213>  Artificial Sequence

<220>
<223>  CDR3 of 10A11 VH

<400>  7

Ala Arg Phe Tyr Tyr Ser Asp Tyr Gly Tyr Ala Met Asp Tyr
1               5                   10


<210>  8
<211>  5
<212>  PRT
<213>  Artificial Sequence

<220>
<223>  CDR2 of 27G1 VH

<400>  8

Thr Tyr Ala Met Tyr
1               5


<210>  9
<211>  7
<212>  PRT
<213>  Artificial Sequence

<220>
<223>  CDR2 of 19D7 VL

<400>  9

Arg Val Ser Asn Leu Ala Ser
1               5


<210>  10
<211>  9
<212>  PRT
<213>  Artificial Sequence

<220>
<223>  CDR3 of 19D7 VL

<400>  10

Met Gln His Leu Glu Tyr Pro Phe Thr
1               5


<210>  11
<211>  12
<212>  PRT
<213>  Artificial Sequence

<220>
<223>  CDR3 of 19D7 VH

<400>  11

Gly Gly Tyr Gly Asn Tyr Arg Tyr Ala Met Asp Tyr
1               5                   10


<210>  12
<211>  5
<212>  PRT
<213>  Artificial Sequence

<220>
<223>  CDR1 of 8F7 VH

<400>  12

Pro Tyr Ala Met Tyr
1               5


<210>  13
<211>  8
<212>  PRT
<213>  Artificial Sequence

<220>
<223>  CDR3 of 8F7 VH

<400>  13

Gly His Gln Gly Thr Met Asp Tyr
1               5


<210>  14
<211>  16
<212>  PRT
<213>  Artificial Sequence

<220>
<223>  CDR1 of 2C7 VL

<400>  14

Arg Ser Ser Gln Ser Leu Val His Ser Asn Gly Asn Thr Tyr Leu His
1               5                   10                  15


<210>  15
<211>  7
<212>  PRT
<213>  Artificial Sequence

<220>
<223>  CDR2 of 2C7 VL

<400>  15

Lys Val Ser Asn Arg Phe Ser

1               5


<210>  16
<211>  9
<212>  PRT
<213>  Artificial Sequence

<220>
<223>  CDR3 of 2C7 VL

<400>  16

Ser Gln Ser Thr Gln Val Pro Leu Thr
1               5


<210>  17
<211>  5
<212>  PRT
<213>  Artificial Sequence

<220>
<223>  CDR1 of 2C7 VH

<400>  17

Ala Tyr Gly Val His
1               5


<210>  18
<211>  16
<212>  PRT
<213>  Artificial Sequence

<220>
<223>  CDR2 of 2C7 VH

<400>  18

Val Ile Trp Arg Gly Gly Ser Thr Asp Tyr Asn Ala Ala Phe Met Ser
1               5                   10                  15


<210>  19
<211>  9
<212>  PRT
<213>  Artificial Sequence

<220>
<223>  CDR3 of 2C7 VH

<400>  19

Lys Gly Gly Ser Tyr Ala Met Asp Tyr
1               5


<210>  20
<211>  15
<212>  PRT
<213>  Artificial Sequence

<220>
<223>  CDR1 of 2-7B VL

<400>  20

Arg Ala Ser Gln Ser Val Ser Ile Ser Arg Tyr Asn Ile Met His
1               5                   10                  15


<210>  21
<211>  7
<212>  PRT
<213>  Artificial Sequence

<220>
<223>  CDR2 of 2-7B VL

<400>  21

Arg Ala Ser Asn Leu Ala Ser
1               5


<210>  22
<211>  9
<212>  PRT
<213>  Artificial Sequence

<220>
<223>  CDR3 of 2-7B VL

<400>  22

Gln Gln Ser Arg Glu Ser Pro Pro Thr
1               5


<210>  23
<211>  5
<212>  PRT
<213>  Artificial Sequence

<220>
<223>  CDR1 of 2-7B VH

<400>  23

Asp Tyr Tyr Met Ala
1               5


<210>  24
<211>  17
<212>  PRT
<213>  Artificial Sequence

<220>
<223>  CDR2 of 2-7B VH

<400>  24

Thr Ile Thr Tyr Asp Val Tyr Ser Thr Tyr Tyr Arg Asp Ser Val Lys

1               5                   10                  15

Gly

<210>  25
<211>  14
<212>  PRT
<213>  Artificial Sequence

<220>
<223>  CDR3 of 2-7B VH

<400>  25

His Gly Gly Asp Gly Tyr Asn Ser Gly Asn Trp Phe Ala Tyr
1               5                   10

<210>  26
<211>  113
<212>  PRT
<213>  Artificial Sequence

<220>
<223>  10A11 VL

<400>  26

Asp Ile Val Met Thr Gln Ala Ala Pro Ser Val Pro Val Thr Pro Gly
1               5                   10                  15

Glu Ser Val Ser Ile Ser Cys Arg Ser Ser Lys Ser Leu Leu His Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Leu Thr Phe Gly Ala Gly Thr Lys Leu Glu Leu Lys
            100                 105                 110

Arg

<210>  27
<211>  125
<212>  PRT
<213>  Artificial Sequence

<220>
<223>  10A11 VH

<400>  27

Glu Val Gln Leu Val Glu Ser Gly Gly Arg Leu Val Gln Pro Lys Gly
1               5                   10                  15

Ser Leu Lys Leu Ser Cys Ala Ala Ser Gly Phe Ala Phe Asn Thr Tyr
            20                  25                  30

Ala Leu Tyr Trp Ile Arg Gln Ala Pro Gly Lys Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Arg Ala Arg Phe Tyr Tyr Ser Asp Tyr Gly Tyr Ala Met
            100                 105                 110

Asp Tyr Trp Gly Gln Gly Thr Ser Val Thr Val Ser Ser
        115                 120                 125


<210>  28
<211>  125
<212>  PRT
<213>  Artificial Sequence

<220>
<223>  27G1 VH

<400>  28

Glu Val Gln Leu Val Glu Ser Gly Gly Arg Leu Val Gln Pro Lys Gly
1               5                   10                  15

Ser Leu Lys Leu Ser Cys Ala Ala Ser Gly Phe Ala Phe Asn Thr Tyr
            20                  25                  30

Ala Met Tyr Trp Ile Arg Gln Ala Pro Gly Lys Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Arg Ala Arg Phe Tyr Tyr Ser Asp Tyr Gly Tyr Ala Met
            100                 105                 110

Asp Tyr Trp Gly Gln Gly Thr Ser Val Thr Val Ser Ser
        115                 120                 125

<210>  29
<211>  113
<212>  PRT
<213>  Artificial Sequence

<220>
<223>  1H4 VL

<400>  29

Asp Ile Val Met Thr Gln Ala Ala Pro Ser Val Pro Val Thr Pro Gly
1               5                   10                  15

Glu Ser Val Ser Ile Ser Cys Arg Ser Ser Lys Ser Leu Leu His Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Ala Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Leu Thr Phe Gly Ala Gly Thr Lys Leu Glu Leu Lys
            100                 105                 110

Arg

<210>  30
<211>  125
<212>  PRT
<213>  Artificial Sequence

<220>
<223>  1H4 VH

<400>  30

Glu Val Gln Leu Val Glu Ser Gly Gly Arg Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Tyr Trp Ile Arg Gln Ala Pro Gly Lys Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Arg Ala Arg Phe Tyr Tyr Ser Asp Tyr Gly Tyr Ala Met
            100                 105                 110

Asp Tyr Trp Gly Gln Gly Thr Ser Val Thr Val Ser Ser
        115                 120                 125


<210>  31
<211>  113
<212>  PRT
<213>  Artificial Sequence

<220>
<223>  19D7 VL

<400>  31

Asp Ile Val Met Thr Gln Ala Ala Pro Ser Val Pro Val Thr Pro Gly
1               5                   10                  15

Glu Ser Val Ser Ile Ser Cys Arg Ser Ser Lys Ser Leu Leu His Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Val Ser Asn Leu Ala Ser Gly Val Pro
    50                  55                  60

Asp Arg Phe Ser Gly Ser Gly Ser Gly Ala Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Phe Thr Phe Gly Ser Gly Thr Lys Leu Glu Ile Lys
            100                 105                 110

Arg

<210>  32
<211>  123
<212>  PRT
<213>  Artificial Sequence

<220>
<223>  19D7 VH

<400>  32

Glu Val Gln Leu Val Glu Ser Gly Gly Arg Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Tyr Trp Ile Arg Gln Ala Pro Gly Lys Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Arg Gly Gly Tyr Gly Asn Tyr Arg Tyr Ala Met Asp Tyr
            100                 105                 110

Trp Gly Gln Gly Thr Ser Val Thr Val Ser Ser
        115                 120

<210>  33
<211>  113
<212>  PRT
<213>  Artificial Sequence

<220>
<223>  8F7 VL

<400>  33

Asp Ile Val Met Thr Gln Ala Ala Pro Ser Val Pro Val Thr Pro Gly
1               5                   10                  15

Glu Ser Val Ser Ile Ser Cys Arg Ser Ser Lys Ser Leu Leu His Ser
            20                  25                  30

Asn Gly Asn Thr Tyr Leu Tyr Trp Phe Leu Gln Arg Pro Gly Gln Ser
        35                  40                  45

Pro Gln Leu Leu Thr Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Phe Thr Phe Gly Gly Gly Thr Lys Leu Glu Ile Lys
            100                 105                 110

Arg

<210>  34
<211>  119
<212>  PRT
<213>  Artificial Sequence

<220>
<223>  8F7 VH

<400>  34

Glu Val Gln Leu Val Glu Ser Gly Gly Arg Leu Val Gln Pro Lys Arg
1               5                   10                  15

Ser Leu Lys Leu Ser Cys Ala Ala Ser Gly Phe Thr Phe Asn Pro Tyr
            20                  25                  30

Ala Met Tyr Trp Ile Arg Gln Ala Pro Gly Gln Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Arg Gly His Gln Gly Thr Met Asp Tyr Trp Gly Gln Gly
            100                 105                 110

Thr Ser Val Thr Val Ser Ser
        115

<210>  35
<211>  113
<212>  PRT
<213>  Artificial Sequence

<220>
<223>  2C7 VL

<400>  35

Asp Val Val Leu Thr Gln Thr Pro Leu Ser Leu Pro Val Ser Leu Gly
1               5                   10                  15

Gly Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr Gln Val Pro Leu Thr Phe Gly Ala Gly Thr Lys Leu Glu Leu Lys
            100                 105                 110

Arg

<210>  36
<211>  117
<212>  PRT
<213>  Artificial Sequence

<220>
<223>  2C7 VH

<400>  36

Gln Val Gln Leu Lys Gln Ser Gly Pro Ser Leu Val Gln Pro Ser Gln
1               5                   10                  15

Ser Leu Ser Ile Thr Cys Thr Val Ser Gly Phe Ser Leu Thr Ala Tyr
            20                  25                  30

Gly Val His Trp Ile Arg Gln Ser Pro Gly Lys Gly Gln Glu Trp Leu
        35                  40                  45

Gly Val Ile Trp Arg Gly Gly Ser Thr Asp Tyr Asn Ala Ala Phe Met
    50                  55                  60

Ser Arg Leu Thr Ile Thr Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Lys Lys Gly Gly Ser Tyr Ala Met Asp Tyr Trp Gly Gln Gly Thr Ser
            100                 105                 110

Val Thr Val Ser Ser
        115


<210>  37
<211>  111
<212>  PRT
<213>  Artificial Sequence

<220>
<223>  2-7B VL

<400>  37

Asp Ile Val Leu Thr Gln Ser Pro Ala Leu Ala Val Ser Leu Gly Gln
1               5                   10                  15

Arg Ala Thr Ile Ser Cys Arg Ala Ser Gln Ser Val Ser Ile Ser Arg
            20                  25                  30

Tyr Asn Ile Met His Trp Tyr Gln Gln Lys Pro Gly Gln Gln Pro Lys
        35                  40                  45

Leu Leu Ile Tyr Arg Ala Ser Asn Leu Ala Ser Gly Ile Pro Ala Arg
    50                  55                  60

Phe Ser Gly Ser Gly Ser Gly Thr Asp Phe Thr Leu Thr Ile Asn Pro
65                  70                  75                  80

Val Gln Ala Asp Asp Ile Ala Thr Tyr Tyr Cys Gln Gln Ser Arg Glu
                85                  90                  95

Ser Pro Pro Thr Phe Gly Gly Gly Thr Lys Leu Glu Leu Lys Arg
            100                 105                 110

<210>  38
<211>  123
<212>  PRT
<213>  Artificial Sequence

<220>
<223>  2-7B VH

<400>  38

Glu Val Gln Leu Val Glu Ser Asp Gly Gly Leu Val Gln Pro Gly Arg
1               5                   10                  15

Ser Leu Lys Leu Ser Cys Ala Ala Ser Gly Phe Thr Phe Ser Asp Tyr
            20                  25                  30

Tyr Met Ala Trp Val Arg Gln Ala Pro Thr Lys Gly Leu Glu Trp Val
        35                  40                  45

Ala Thr Ile Thr Tyr Asp Val Tyr Ser Thr Tyr Tyr Arg Asp Ser Val
    50                  55                  60

Lys Gly Arg Phe Thr Ile Ser Arg Asp Asn Ala Lys Ser Thr Leu Tyr
65                  70                  75                  80

Leu Gln Met Asp Ser Leu Arg Ser Glu Asp Thr Ala Thr Tyr Tyr Cys
                85                  90                  95

Ala Arg His Gly Gly Asp Gly Tyr Asn Ser Gly Asn Trp Phe Ala Tyr
            100                 105                 110

Trp Gly Gln Gly Thr Leu Val Thr Val Ser Ser
        115                 120

<210>  39
<211>  353
<212>  PRT
<213>  Mus musculus

<400>  39

Met Asp Tyr Thr Met Glu Pro Asn Val Thr Met Thr Asp Tyr Tyr Pro
1               5                   10                  15

Asp Phe Phe Thr Ala Pro Cys Asp Ala Glu Phe Leu Leu Arg Gly Ser
            20                  25                  30

Met Leu Tyr Leu Ala Ile Leu Tyr Cys Val Leu Phe Val Leu Gly Leu
        35                  40                  45

Leu Gly Asn Ser Leu Val Ile Leu Val Leu Val Gly Cys Lys Lys Leu
    50                  55                  60

Arg Ser Ile Thr Asp Ile Tyr Leu Leu Asn Leu Ala Ala Ser Asp Leu
65                  70                  75                  80

Leu Phe Val Leu Ser Ile Pro Phe Gln Thr His Asn Leu Leu Asp Gln
                85                  90                  95

Trp Val Phe Gly Thr Ala Met Cys Lys Val Val Ser Gly Leu Tyr Tyr
            100                 105                 110

Ile Gly Phe Phe Ser Ser Met Phe Phe Ile Thr Leu Met Ser Val Asp
        115                 120                 125

Arg Tyr Leu Ala Ile Val His Ala Val Tyr Ala Ile Lys Val Arg Thr
    130                 135                 140

Ala Ser Val Gly Thr Ala Leu Ser Leu Thr Val Trp Leu Ala Ala Val
145                 150                 155                 160

Thr Ala Thr Ile Pro Leu Met Val Phe Tyr Gln Val Ala Ser Glu Asp
                165                 170                 175

Gly Met Leu Gln Cys Phe Gln Phe Tyr Glu Glu Gln Ser Leu Arg Trp
            180                 185                 190

Lys Leu Phe Thr His Phe Glu Ile Asn Ala Leu Gly Leu Leu Leu Pro
        195                 200                 205

Phe Ala Ile Leu Leu Phe Cys Tyr Val Arg Ile Leu Gln Gln Leu Arg
    210                 215                 220

Gly Cys Leu Asn His Asn Arg Thr Arg Ala Ile Lys Leu Val Leu Thr
225                 230                 235                 240

Val Val Ile Val Ser Leu Leu Phe Trp Val Pro Phe Asn Val Ala Leu
                245                 250                 255

Phe Leu Thr Ser Leu His Asp Leu His Ile Leu Asp Gly Cys Ala Thr
            260                 265                 270

Arg Gln Arg Leu Ala Leu Ala Ile His Val Thr Glu Val Ile Ser Phe
        275                 280                 285

Thr His Cys Cys Val Asn Pro Val Ile Tyr Ala Phe Ile Gly Glu Lys
    290                 295                 300

Phe Lys Lys His Leu Met Asp Val Phe Gln Lys Ser Cys Ser His Ile
305                 310                 315                 320

Phe Leu Tyr Leu Gly Arg Gln Met Pro Val Gly Ala Leu Glu Arg Gln
                325                 330                 335

Leu Ser Ser Asn Gln Arg Ser Ser His Ser Ser Thr Leu Asp Asp Ile
            340                 345                 350

Leu

<210> 40
<211> 114
<212> PRT
<213> Artificial Sequence

<220>
<223> 10A11-IGKV3-20

<400> 40

Glu Ile Val Leu Thr Gln Ser Pro Gly Thr Leu Ser Leu Ser Pro Gly
1               5                   10                  15

Glu Arg Ala Thr Leu Ser Cys Arg Ser Ser Lys Ser Leu Leu His Ser
            20                  25                  30

Asn Gly Asn Thr Tyr Leu Tyr Trp Tyr Gln Gln Lys Pro Gly Gln Ala
        35                  40                  45

Pro Arg Leu Leu Ile Tyr Arg Met Ser Asn Leu Ala Ser Gly Ile Pro
    50                  55                  60

Asp Arg Phe Ser Gly Ser Gly Ser Gly Thr Asp Phe Thr Leu Thr Ile
65                  70                  75                  80

Ser Arg Leu Glu Pro Glu Asp Phe Ala Val Tyr Tyr Cys Met Gln His

85                  90                  95

Leu Glu Tyr Pro Leu Thr Phe Gly Gln Gly Thr Lys Val Glu Ile Lys
            100                 105                 110

Arg Thr

<210>  41
<211>  125
<212>  PRT
<213>  Artificial Sequence

<220>
<223>  10A11-IGHV3-15 T94R

<400>  41

Glu Val Gln Leu Val Glu Ser Gly Gly Ala Leu Val Lys Pro Gly Gly
1               5                   10                  15

Ser Leu Arg Leu Ser Cys Ala Ala Ser Gly Phe Thr Phe Ser Thr Tyr
            20                  25                  30

Ala Leu Tyr Trp Val Arg Gln Ala Pro Gly Lys Gly Leu Glu Trp Val
        35                  40                  45

Gly Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Leu Tyr Leu Gln Met Asn Ser Leu Lys Thr Glu Asp Thr Ala Val Tyr
                85                  90                  95

Tyr Cys Thr Arg Ala Arg Phe Tyr Tyr Ser Asp Tyr Gly Tyr Ala Met
            100                 105                 110

Asp Tyr Trp Gly Gln Gly Thr Leu Val Thr Val Ser Ser
        115                 120                 125

<210>  42
<211>  114
<212>  PRT
<213>  Artificial Sequence

<220>
<223>  10A11-IGKV4-1

<400>  42

Asp Ile Val Met Thr Gln Ser Pro Asp Ser Leu Ala Val Ser Leu Gly
1               5                   10                  15

Glu Arg Ala Thr Ile Asn Cys Arg Ser Ser Lys Ser Leu Leu His Ser
            20                  25                  30

Asn Gly Asn Thr Tyr Leu Tyr Trp Tyr Gln Gln Lys Pro Gly Gln Pro
        35                  40                  45

Pro Lys Leu Leu Ile Tyr Arg Met Ser Asn Leu Ala Ser Gly Val Pro
    50                  55                  60

Asp Arg Phe Ser Gly Ser Gly Ser Gly Thr Asp Phe Thr Leu Thr Ile
65                  70                  75                  80

Ser Ser Leu Gln Ala Glu Asp Val Ala Val Tyr Tyr Cys Met Gln His
                85                  90                  95

Leu Glu Tyr Pro Leu Thr Phe Gly Gln Gly Thr Lys Leu Glu Ile Lys
            100                 105                 110

Arg Thr

<210>  43
<211>  114
<212>  PRT
<213>  Artificial Sequence

<220>
<223>  10A11-IGKV1-39

<400>  43

Asp Ile Gln Met Thr Gln Ser Pro Ser Ser Leu Ser Ala Ser Val Gly
1               5                   10                  15

Asp Arg Val Thr Ile Thr Cys Arg Ser Ser Lys Ser Leu Leu His Ser
            20                  25                  30

Asn Gly Asn Thr Tyr Leu Tyr Trp Tyr Gln Gln Lys Pro Gly Lys Ala
        35                  40                  45

Pro Lys Leu Leu Ile Tyr Arg Met Ser Asn Leu Ala Ser Gly Val Pro
    50                  55                  60

Ser Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Tyr Cys Met Gln His

85                  90                  95

Leu Glu Tyr Pro Leu Thr Phe Gly Gln Gly Thr Lys Leu Glu Ile Lys
            100                 105                 110

Arg Thr

<210>  44
<211>  114
<212>  PRT
<213>  Artificial Sequence

<220>
<223>  10A11-IGKV2-40

<400>  44

Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Asn Thr Tyr Leu Tyr Trp Tyr Leu Gln Lys Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln His
                85                  90                  95

Leu Glu Tyr Pro Leu Thr Phe Gly Gln Gly Thr Lys Leu Glu Ile Lys
            100                 105                 110

Arg Thr

<210>  45
<211>  114
<212>  PRT
<213>  Artificial Sequence

<220>
<223>  10A11-IGKV1-16

<400>  45

Asp Ile Gln Met Thr Gln Ser Pro Ser Ser Leu Ser Ala Ser Val Gly
1               5                   10                  15

Asp Arg Val Thr Ile Thr Cys Arg Ser Ser Lys Ser Leu Leu His Ser
            20                  25                  30

Asn Gly Asn Thr Tyr Leu Tyr Trp Phe Gln Gln Lys Pro Gly Lys Ala
        35                  40                  45

Pro Lys Ser Leu Ile Tyr Arg Met Ser Asn Leu Ala Ser Gly Val Pro
    50                  55                  60

Ser Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Tyr Cys Met Gln His
                85                  90                  95

Leu Glu Tyr Pro Leu Thr Phe Gly Gln Gly Thr Lys Leu Glu Ile Lys
            100                 105                 110

Arg Thr

<210>  46
<211>  125
<212>  PRT
<213>  Artificial Sequence

<220>
<223>  10A11-IGHV3-73

<400>  46

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Ala Phe Asn Thr Tyr
            20                  25                  30

Ala Leu Tyr Trp Val Arg Gln Ala Ser Gly Lys Gly Leu Glu Trp Val
        35                  40                  45

Gly Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Ser Leu Lys Thr Glu Asp Thr Ala Val Tyr

85                  90                  95

Tyr Cys Thr Arg Ala Arg Phe Tyr Tyr Ser Asp Tyr Gly Tyr Ala Met
            100                 105                 110

Asp Tyr Trp Gly Gln Gly Thr Leu Val Thr Val Ser Ser
        115                 120                 125

<210>  47
<211>  114
<212>  PRT
<213>  Artificial Sequence

<220>
<223>  10A11-IGKV2-28

<400>  47

Asp Ile Val Met Thr Gln Ser Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Asn Thr Tyr Leu Tyr Trp Tyr Leu Gln Lys Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln His
                85                  90                  95

Leu Glu Tyr Pro Leu Thr Phe Gly Gln Gly Thr Lys Val Glu Ile Lys
            100                 105                 110

Arg Thr

<210>  48
<211>  360
<212>  PRT
<213>  Homo sapiens

<400>  48

Met Asn Pro Thr Asp Ile Ala Asp Thr Thr Leu Asp Glu Ser Ile Tyr
1               5                   10                  15

Ser Asn Tyr Tyr Leu Tyr Glu Ser Ile Pro Lys Pro Cys Thr Lys Glu
            20                  25                  30

Gly Ile Lys Ala Phe Gly Glu Leu Phe Leu Pro Pro Leu Tyr Ser Leu
        35                  40                  45

Val Phe Val Phe Gly Leu Leu Gly Asn Ser Val Val Val Leu Val Leu
    50                  55                  60

Phe Lys Tyr Lys Arg Leu Arg Ser Met Thr Asp Val Tyr Leu Leu Asn
65                  70                  75                  80

Leu Ala Ile Ser Asp Leu Leu Phe Val Phe Ser Leu Pro Phe Trp Gly
                85                  90                  95

Tyr Tyr Ala Ala Asp Gln Trp Val Phe Gly Leu Gly Leu Cys Lys Met
            100                 105                 110

Ile Ser Trp Met Tyr Leu Val Gly Phe Tyr Ser Gly Ile Phe Phe Val
        115                 120                 125

Met Leu Met Ser Ile Asp Arg Tyr Leu Ala Ile Val His Ala Val Phe
    130                 135                 140

Ser Leu Arg Ala Arg Thr Leu Thr Tyr Gly Val Ile Thr Ser Leu Ala
145                 150                 155                 160

Thr Trp Ser Val Ala Val Phe Ala Ser Leu Pro Gly Phe Leu Phe Ser
                165                 170                 175

Thr Cys Tyr Thr Glu Arg Asn His Thr Tyr Cys Lys Thr Lys Tyr Ser
            180                 185                 190

Leu Asn Ser Thr Thr Trp Lys Val Leu Ser Ser Leu Glu Ile Asn Ile
        195                 200                 205

Leu Gly Leu Val Ile Pro Leu Gly Ile Met Leu Phe Cys Tyr Ser Met
    210                 215                 220

Ile Ile Arg Thr Leu Gln His Cys Lys Asn Glu Lys Lys Asn Lys Ala
225                 230                 235                 240

Val Lys Met Ile Phe Ala Val Val Val Leu Phe Leu Gly Phe Trp Thr
                245                 250                 255

Pro Tyr Asn Ile Val Leu Phe Leu Glu Thr Leu Val Glu Leu Glu Val
            260                 265                 270

Leu Gln Asp Cys Thr Phe Glu Arg Tyr Leu Asp Tyr Ala Ile Gln Ala
        275                 280                 285

Thr Glu Thr Leu Ala Phe Val His Cys Cys Leu Asn Pro Ile Ile Tyr
    290                 295                 300

Phe Phe Leu Gly Glu Lys Phe Arg Lys Tyr Ile Leu Gln Leu Phe Lys
305                 310                 315                 320

Thr Cys Arg Gly Leu Phe Val Leu Cys Gln Tyr Cys Gly Leu Leu Gln
                325                 330                 335

Ile Tyr Ser Ala Asp Thr Pro Ser Ser Ser Tyr Thr Gln Ser Thr Met
            340                 345                 350

Asp His Asp Leu His Asp Ala Leu
        355                 360

<210>  49
<211>  7
<212>  PRT
<213>  Artificial Sequence

<220>
<223>  CDR2 consensus sequence of VL

<220>
<221>  misc_feature
<222>  (2)..(2)
<223>  Xaa can be any naturally occurring amino acid

<400>  49

Arg Xaa Ser Asn Leu Ala Ser
1               5

<210>  50
<211>  9
<212>  PRT
<213>  Artificial Sequence

<220>
<223>  CDR3 consensus sequence of VL

<220>
<221>  misc_feature
<222>  (8)..(8)
<223>  Xaa can be any naturally occurring amino acid

<400>  50

Met Gln His Leu Glu Tyr Pro Xaa Thr

1               5


<210>  51
<211>  5
<212>  PRT
<213>  Artificial Sequence

<220>
<223>  CDR1 consensus sequence of VH


<220>
<221>  misc_feature
<222>  (1)..(1)
<223>  Xaa can be any naturally occurring amino acid

<220>
<221>  misc_feature
<222>  (4)..(4)
<223>  Xaa can be any naturally occurring amino acid

<400>  51

Xaa Tyr Ala Xaa Tyr
1               5


<210>  52
<211>  105
<212>  PRT
<213>  Artificial Sequence

<220>
<223>  h10A11 CL

<400>  52

<210>  53
<211>  329
<212>  PRT
<213>  Artificial Sequence

<220>
<223>  h10A11 CH

<400>  53

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  54
<211>  112
<212>  PRT
<213>  Artificial Sequence

<220>
<223>  19D7-IGKV3-15

<400>  54

Glu Ile Val Met Thr Gln Ser Pro Ala Thr Leu Ser Val Ser Pro Gly
1               5                   10                  15

Glu Arg Ala Thr Leu Ser Cys Arg Ser Ser Lys Ser Leu Leu His Ser
            20                  25                  30

Asn Gly Asn Thr Tyr Leu Tyr Trp Tyr Gln Gln Lys Pro Gly Gln Ala
        35                  40                  45

Pro Arg Leu Leu Ile Tyr Arg Val Ser Asn Leu Ala Ser Gly Ile Pro
    50                  55                  60

Ala Arg Phe Ser Gly Ser Gly Ser Gly Thr Glu Phe Thr Leu Thr Ile
65                  70                  75                  80

Ser Ser Leu Gln Ser Glu Asp Phe Ala Val Tyr Tyr Cys Met Gln His
                85                  90                  95

Leu Glu Tyr Pro Phe Thr Phe Gly Gln Gly Thr Lys Leu Glu Ile Lys
            100                 105                 110

<210>  55
<211>  112
<212>  PRT
<213>  Artificial Sequence

<220>
<223>  19D7-IGKV2-18

<400>  55

Asp Ile Val Met Thr Gln Thr Pro Pro Ser Leu Pro Val Asn Pro Gly
1               5                   10                  15

Glu Pro Ala Ser Ile Ser Cys Arg Ser Ser Lys Ser Leu Leu His Ser
            20                  25                  30

Asn Gly Asn Thr Tyr Leu Tyr Trp Tyr Leu Gln Lys Pro Gly Gln Ser
        35                  40                  45

Pro Gln Leu Leu Ile Tyr Arg Val Ser Asn Leu Ala Ser Gly Val Pro
    50                  55                  60

Asp Arg Phe Ser Gly Ser Gly Ser Gly Ser Asp Phe Thr Leu Lys Ile
65                  70                  75                  80

Ser Trp Val Glu Ala Glu Asp Val Gly Val Tyr Tyr Cys Met Gln His
                85                  90                  95

Leu Glu Tyr Pro Phe Thr Phe Gly Gln Gly Thr Lys Leu Glu Ile Lys
            100                 105                 110

<210>  56
<211>  112
<212>  PRT
<213>  Artificial Sequence

<220>
<223>  19D7-IGKV3-20

<400>  56

Glu Ile Val Leu Thr Gln Ser Pro Gly Thr Leu Ser Leu Ser Pro Gly
1               5                   10                  15

Glu Arg Ala Thr Leu Ser Cys Arg Ser Ser Lys Ser Leu Leu His Ser
            20                  25                  30

Asn Gly Asn Thr Tyr Leu Tyr Trp Tyr Gln Gln Lys Pro Gly Gln Ala
        35                  40                  45

Pro Arg Leu Leu Ile Tyr Arg Val Ser Asn Leu Ala Ser Gly Ile Pro
    50                  55                  60

Asp Arg Phe Ser Gly Ser Gly Ser Gly Thr Asp Phe Thr Leu Thr Ile
65                  70                  75                  80

Ser Arg Leu Glu Pro Glu Asp Phe Ala Val Tyr Tyr Cys Met Gln His
                85                  90                  95

Leu Glu Tyr Pro Phe Thr Phe Gly Gln Gly Thr Lys Leu Glu Ile Lys
            100                 105                 110

<210>  57
<211>  123
<212>  PRT
<213>  Artificial Sequence

<220>
<223>  19D7-IGHV3-15 T94R

<400>  57

Glu Val Gln Leu Val Glu Ser Gly Gly Ala Leu Val Lys Pro Gly Gly
1               5                   10                  15

Ser Leu Arg Leu Ser Cys Ala Ala Ser Gly Phe Thr Phe Ser Thr Tyr
            20                  25                  30

Ala Met Tyr Trp Val Arg Gln Ala Pro Gly Lys Gly Leu Glu Trp Val
        35                  40                  45

Gly Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Leu Tyr Leu Gln Met Asn Ser Leu Lys Thr Glu Asp Thr Ala Val Tyr
                85                  90                  95

Tyr Cys Thr Arg Gly Gly Tyr Gly Asn Tyr Arg Tyr Ala Met Asp Tyr
            100                 105                 110

Trp Gly Gln Gly Thr Thr Val Thr Val Ser Ser
        115                 120

<210>  58
<211>  123
<212>  PRT
<213>  Artificial Sequence

<220>
<223>  19D7-IGHV3-73

<400>  58

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Thr Tyr
            20                  25                  30

Ala Met Tyr Trp Val Arg Gln Ala Ser Gly Lys Gly Leu Glu Trp Val
        35                  40                  45

Gly Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Ser Leu Lys Thr Glu Asp Thr Ala Val Tyr
                85                  90                  95

Tyr Cys Thr Arg Gly Gly Tyr Gly Asn Tyr Arg Tyr Ala Met Asp Tyr
            100                 105                 110

Trp Gly Gln Gly Thr Thr Val Thr Val Ser Ser
        115                 120


<210>  59
<211>  114
<212>  PRT
<213>  Artificial Sequence

<220>
<223>  10A11-IGKV4-1 N53Q+G29R

<400>  59

Asp Ile Val Met Thr Gln Ser Pro Asp Ser Leu Ala Val Ser Leu Gly
1               5                   10                  15

Glu Arg Ala Thr Ile Asn Cys Arg Ser Ser Lys Ser Leu Leu His Ser
            20                  25                  30

Asn Arg Asn Thr Tyr Leu Tyr Trp Tyr Gln Gln Lys Pro Gly Gln Pro
        35                  40                  45

Pro Lys Leu Leu Ile Tyr Arg Met Ser Gln Leu Ala Ser Gly Val Pro
    50                  55                  60

Asp Arg Phe Ser Gly Ser Gly Ser Gly Thr Asp Phe Thr Leu Thr Ile
65                  70                  75                  80

Ser Ser Leu Gln Ala Glu Asp Val Ala Val Tyr Tyr Cys Met Gln His
                85                  90                  95

Leu Glu Tyr Pro Leu Thr Phe Gly Gln Gly Thr Lys Leu Glu Ile Lys
            100                 105                 110

Arg Thr

## Description

### [TECHNICAL FIELD]

The present invention relates to a novel anti-CCR8 antibody and a pharmaceutical composition containing the antibody.

### [BACKGROUND ART]

Potent negative regulation mechanisms, including immunosuppression, mediated by regulatory T cells (Treg cells) in the tumor microenvironment are major obstacles to the treatment of tumors (Non-patent Document 1).

For example, CD4-positive Treg cells which infiltrate tumors may be able to strongly inhibit anti-tumor immune response and may become a major obstacle to effective cancer treatment.

Tumor immunosuppression mediated by CD4-positive FoxP3-positive Treg cells has been sufficiently demonstrated in animal tumor models. It has been reported that systemic (including intratumoral) Treg cell removal produces an anti-tumor effect, wherein the removal of approximately 50% tumor-infiltrating Treg cells is not effective (Non-patent Document 2).

It has been reported that the increased ratio of CD4-positive CD25-positive Treg cells (cell population including Treg cells) to the whole CD4-positive T cell population in humans is intratumorally detected in patients with various cancers including lung, breast, and ovary tumors, and the abundance ratio correlates negatively with the survival probabilities of the patients (Non-patent Documents 3 to 8).

The removal of CD4-positive CD25-positive Treg cells from tumors using an anti-CD25 antibody has been confirmed to produce an anti-tumor effect. However, this removal is not specific for the Treg cells because CD25 is expressed on the cell surface of the CD4-positive CD25-positive Treg cells as well as newly activated effector T cells. Furthermore, the administration of an anti-CD25 antibody to mice brings about a limited anti-tumor effect. It has been demonstrated in various tumor models that only the antibody administration before tumor inoculation exhibits a therapeutic effect, whereas the administration of the antibody after tumor engraftment in mice rarely produces a therapeutic effect. The anti-tumor effect was attenuated in the case of starting the administration of an anti-CD25 antibody at 1 day after tumor inoculation, and was rarely observed in the case of starting the administration of an anti-CD25 antibody at 2 days or later after tumor inoculation (Non-patent Document 9).

Drug efficacy tests have been carried out so far by administering antibodies to mice for the purpose of removing Treg cells. Nonetheless, there are few reports showing an anti-tumor effect. Thus, it is very difficult to confirm an anti-tumor therapeutic effect brought about by Treg cell removal by antibody administration before inoculation (Non-patent Document 10).

CCR8, also previously called CY6, CKR-L1 or TER1, is a G protein-coupled 7-transmembrane CC chemokine receptor protein expressed in the thymus, the spleen, etc. A gene encoding this protein resides on human chromosome 3p21. Human CCR8 consists of 355 amino acids (Non-patent Document 11). CCL1 is known as an endogenous ligand for CCR8 (Non-patent Document 12). Human CCR8 cDNA is constituted by the nucleotide sequence represented by GenBank ACC No. NM_005201.3, and mouse CCR8 cDNA is constituted by the nucleotide sequence represented by GenBank ACC No. NM_007720.2.

CCR8 is also specifically expressed in tumor-infiltrating Treg cellss, and it has been demonstrated that, when breast cancer cells were inoculated in CCR8-deficient and wild-type mice, breast cancer proliferation and metastasis were more suppressed in the CCR8-deficient mouse compared to the wild-type mice (Patent Document 1 and Non-patent Document 13). Furthermore, it has been disclosed that anti-CCR8 antibody administration to a cancer model animal showed an anti-tumor effect (Patent Documents 2 and 3).

Patent Document 4 discloses anti-CCR8 antibodies that are useful for treating allergic diseases and HIV infection. This document discloses anti-CCR8 antibodies represented by 414B, 414C, 414E, 433H, 459M, 464A, 464B, 433B, and 455AL. Of these, the anti-CCR8 antibody 433H is available from BD Biosciences. However, the CDR sequence of the antibody has not been disclosed. Furthermore, it has not been shown that anti-CCR8 antibodies have anti-tumor activity and are useful for cancer treatment.

Non-patent Document 14 discloses anti-CCR8 antibodies represented by 3B10, 2D10, and 5B11. An anti-CCR8 antibody of product number L263G8 is available from BioLegend. An anti-CCR8 antibody of product number 191704 is available from R&D Systems, Inc. However, the CDR sequences of these antibodies have not been disclosed. It has also not shown that anti-CCR8 antibodies have anti-tumor activity and are useful for cancer treatment.
Patent Document 5 and Non-patent Documents 15-19 describe that CCR8 is involved in the pathology of cancer.

### [PRIOR ART REFERENCES]

### [Patent Documents]

[Patent Document 1] US 10087259
[Patent Document 2] WO 2018/181425
[Patent Document 3] WO 2018/112032
[Patent Document 4] WO 2007/044756
[Patent Document 5] WO 2017/198631

### [Non-patent Documents]

[Non-patent Document 1] Nat. Rev. Immunol., 2006, Vol. 6, No. 4, p.295-307
[Non-patent Document 2] Eur. J. Immunol., 2010, Vol. 40, p.3325-3335
[Non-patent Document 3] J. Clin. Oncol., 2006, Vol. 24, p.5373-5380
[Non-patent Document 4] Nat. Med., 2004, Vol. 10, p.942-949
[Non-patent Document 5] J. Clin. Oncol., 2007, Vol. 25, p.2586-2593
[Non-patent Document 6] Cancer, 2006, Vol. 107, p.2866-2872
[Non-patent Document 7] Eur. J. Cancer, 2008, Vol. 44, p.1875-1882
[Non-patent Document 8] Cell. Mol. Immunol. 2011, Vol. 8, p.59-66
[Non-patent Document 9] Cancer Res., 1999, Vol. 59, No. 13, p. 3128-33
[Non-patent Document 10] Cancer Res., 2010, Vol. 70, No. 7, p. 2665-74
[Non-patent Document 11] J. Immunol., 1996, Vol. 157, No. 7, p. 2759-63
[Non-patent Document 12] J. Biol. Chem., 1997, Vol. 272, No. 28, p. 17251-4
[Non-patent Document 13] Cancer Res., 2016, Vol. 76, No. 4, Supp.1, P4-04-11
[Non-patent Document 14] J. Exp. Med., 2004, Vol. 200, No. 10, p1231-1241
[Non-patent Document 15] Immunity, 2016, Vol. 45, p1122-1134
[Non-patent Document 16] Immunity, 2016, Vol. 45, p1135-1147
[Non-patent Document 17] Proc. Natl. Acad. Sci. USA, 2018, Vol. 115, No. 45, p10672-10681
[Non-patent Document 18] Targeting of CCR8 induces antitumor activity as a monotherapy that is further enhanced in combination with a Listeria-based immunotherapy, ASCO 2018, Daniel O Villarreal, et al. https://www.advaxis.com/wp-content/uploads/2018/04/KeystonePosterCCR8-combo-posterFINAL.pdf
[Non-patent Document 19] Cancer Res., 2018, Vol. 78, No. 18, p5340-5348

### [SUMMARY OF THE INVENTION]

### [PROBLEMS TO BE SOLVED BY THE INVENTION]

An object of the present invention is to provide a novel anti-CCR8 antibody or an antibody fragment thereof. A further object of the present invention is to provide a novel anti-CCR8 antibody or an antibody fragment thereof that can be used as a therapeutic agent for cancer.

### [MEANS FOR SOLVING THE PROBLEMS]

The present inventors have found, as a result of intensive studies, a monoclonal antibody that specifically binds to human CCR8 to inhibit binding of CCR8 to a CCR8 ligand. The present inventors have also found that a monoclonal antibody or an antibody fragment thereof that recognizes tyrosine at position 17 in the amino acid sequence of human CCR8 can be used to inhibit binding of CCR8 to a CCR8 ligand. The present inventors have further found that the monoclonal antibody of the present invention has an anti-tumor activity and is useful for the treatment of cancer. The present inventors have also found that a monoclonal antibody or an antibody fragment thereof that recognizes tyrosine at position 17 in the amino acid sequence of human CCR8 has an anti-tumor activity and can be used to treat cancer. In other words, the monoclonal antibody or an antibody fragment thereof of the present invention can be used to inhibit binding of CCR8 to a CCR8 ligand. Furthermore, the monoclonal antibody or an antibody fragment thereof of the present invention can be used as a neutralizing antibody.

Specifically, the present invention relates to the following.
(1) A monoclonal antibody or an antibody fragment thereof that binds to CCR8, which recognizes tyrosine at position 17 in the amino acid sequence set forth in SEQ ID NO: 1.
(2) The antibody or an antibody fragment thereof according to (1), which is a neutralizing antibody.
(3) The antibody or an antibody fragment thereof according to (1) or (2), which further recognizes one or more of amino acids at positions 94 to 107 in the amino acid sequence set forth in SEQ ID NO: 1.
(4) The antibody or an antibody fragment thereof according to any one of (1) to (3), which further recognizes one or more of amino acids at positions 172 to 202 in the amino acid sequence set forth in SEQ ID NO: 1.
(5) The antibody or an antibody fragment thereof according to any one of (1) to (4), which recognizes one or more amino acids of isoleucine at position 20, serine at position 22, lysine at position 35, leucine at position 181, cysteine at position 183, and asparagine at position 188 in the amino acid sequence set forth in SEQ ID NO: 1.
(6) The antibody or an antibody fragment thereof according to any one of (1) to (5), which is a humanized monoclonal antibody or an antibody fragment thereof.
(7) The monoclonal antibody or an antibody fragment thereof that binds to CCR8, having:
   1) a light chain variable region including
      a CDR1 having the amino acid sequence of SEQ ID NO: 2 optionally having deletion, substitution, insertion and/or addition of one or two amino acids,
      a CDR2 having the amino acid sequence of SEQ ID NO: 3 optionally having deletion, substitution, insertion and/or addition of one or two amino acids, and
      a CDR3 having the amino acid sequence of SEQ ID NO: 4 optionally having deletion, substitution, insertion and/or addition of one or two amino acids, and
      a heavy chain variable region including
      a CDR1 having the amino acid sequence of SEQ ID NO: 5 optionally having deletion, substitution, insertion and/or addition of one or two amino acids,
      a CDR2 having the amino acid sequence of SEQ ID NO: 6 optionally having deletion, substitution, insertion and/or addition of one or two amino acids, and
      a CDR3 having the amino acid sequence of SEQ ID NO: 7 optionally having deletion, substitution, insertion and/or addition of one or two amino acids;
   2) a light chain variable region including
      a CDR1 having the amino acid sequence of SEQ ID NO: 2 optionally having deletion, substitution, insertion and/or addition of one or two amino acids,
      a CDR2 having the amino acid sequence of SEQ ID NO: 3 optionally having deletion, substitution, insertion and/or addition of one or two amino acids, and
      a CDR3 having the amino acid sequence of SEQ ID NO: 4 optionally having deletion, substitution, insertion and/or addition of one or two amino acids, and
      a heavy chain variable region including
      a CDR1 having the amino acid sequence of SEQ ID NO: 8 optionally having deletion, substitution, insertion and/or addition of one or two amino acids,
      a CDR2 having the amino acid sequence of SEQ ID NO: 6 optionally having deletion, substitution, insertion and/or addition of one or two amino acids, and
      a CDR3 having the amino acid sequence of SEQ ID NO: 7 optionally having deletion, substitution, insertion and/or addition of one or two amino acids;
   3) a light chain variable region including
      a CDR1 having the amino acid sequence of SEQ ID NO: 2 optionally having deletion, substitution, insertion and/or addition of one or two amino acids,
      a CDR2 having the amino acid sequence of SEQ ID NO: 9 optionally having deletion, substitution, insertion and/or addition of one or two amino acids, and
      a CDR3 having the amino acid sequence of SEQ ID NO: 10 optionally having deletion, substitution, insertion and/or addition of one or two amino acids, and
      a heavy chain variable region including
      a CDR1 having the amino acid sequence of SEQ ID NO: 8 optionally having deletion, substitution, insertion and/or addition of one or two amino acids,
      a CDR2 having the amino acid sequence of SEQ ID NO: 6 optionally having deletion, substitution, insertion and/or addition of one or two amino acids, and
      a CDR3 having the amino acid sequence of SEQ ID NO: 11 optionally having deletion, substitution, insertion and/or addition of one or two amino acids;
      wherein one or more of the following substitutions are optionally present:
         A) substitution of serine with threonine at position 2 in the amino acid sequence of SEQ ID NO: 2,
         B) substitution of serine with threonine at position 3 in the amino acid sequence of SEQ ID NO: 2,
         C) substitution of serine with threonine at position 5 in the amino acid sequence of SEQ ID NO: 2,
         D) substitution of glutamine with asparagine at position 2 in the amino acid sequence of SEQ ID NO: 10,
         E) substitution of threonine with serine at position 1 in the amino acid sequence of SEQ ID NO: 8,
         F) substitution of alanine with valine at position 14 in the amino acid sequence of SEQ ID NO: 6,
         G) substitution of lysine with arginine at position 18 in the amino acid sequence of SEQ ID NO: 6,
         H) substitution of aspartic acid with glutamic acid at position 19 in the amino acid sequence of SEQ ID NO: 6,
         I) substitution of asparagine with glutamine at position 5 in the amino acid sequence of SEQ ID NO: 11, and
         J) substitution of tyrosine with phenylalanine at position 12 in the amino acid sequence of SEQ ID NO: 11;
   4) a light chain variable region including
      a CDR1 having the amino acid sequence of SEQ ID NO: 2 optionally having deletion, substitution, insertion and/or addition of one or two amino acids,
      a CDR2 having the amino acid sequence of SEQ ID NO: 3 optionally having deletion, substitution, insertion and/or addition of one or two amino acids, and
      a CDR3 having the amino acid sequence of SEQ ID NO: 10 optionally having deletion, substitution, insertion and/or addition of one or two amino acids, and
      a heavy chain variable region including
      a CDR1 having the amino acid sequence of SEQ ID NO: 12 optionally having deletion, substitution, insertion and/or addition of one or two amino acids,
      a CDR2 having the amino acid sequence of SEQ ID NO: 6 optionally having deletion, substitution, insertion and/or addition of one or two amino acids, and
      a CDR3 having the amino acid sequence of SEQ ID NO: 13 optionally having deletion, substitution, insertion and/or addition of one or two amino acids;
   5) a light chain variable region including
      a CDR1 having the amino acid sequence of SEQ ID NO: 14 optionally having deletion, substitution, insertion and/or addition of one or two amino acids,
      a CDR2 having the amino acid sequence of SEQ ID NO: 15 optionally having deletion, substitution, insertion and/or addition of one or two amino acids, and
      a CDR3 having the amino acid sequence of SEQ ID NO: 16 optionally having deletion, substitution, insertion and/or addition of one or two amino acids, and
      a heavy chain variable region including
      a CDR1 having the amino acid sequence of SEQ ID NO: 17 optionally having deletion, substitution, insertion and/or addition of one or two amino acids,
      a CDR2 having the amino acid sequence of SEQ ID NO: 18 optionally having deletion, substitution, insertion and/or addition of one or two amino acids, and
      a CDR3 having the amino acid sequence of SEQ ID NO: 19 optionally having deletion, substitution, insertion and/or addition of one or two amino acids; or
   6) a light chain variable region including
      a CDR1 having the amino acid sequence of SEQ ID NO: 20 optionally having deletion, substitution, insertion and/or addition of one or two amino acids,
      a CDR2 having the amino acid sequence of SEQ ID NO: 21 optionally having deletion, substitution, insertion and/or addition of one or two amino acids, and
      a CDR3 having the amino acid sequence of SEQ ID NO: 22 optionally having deletion, substitution, insertion and/or addition of one or two amino acids, and
      a heavy chain variable region including
      a CDR1 having the amino acid sequence of SEQ ID NO: 23 optionally having deletion, substitution, insertion and/or addition of one or two amino acids,
      a CDR2 having the amino acid sequence of SEQ ID NO: 24 optionally having deletion, substitution, insertion and/or addition of one or two amino acids, and
      a CDR3 having the amino acid sequence of SEQ ID NO: 25 optionally having deletion, substitution, insertion and/or addition of one or two amino acids.
(8) The antibody or an antibody fragment thereof according to (7), having:
   1) a light chain variable region including
      a CDR1 having the amino acid sequence of SEQ ID NO: 2,
      a CDR2 having the amino acid sequence of SEQ ID NO: 3, and
      a CDR3 having the amino acid sequence of SEQ ID NO: 4, and
      a heavy chain variable region including
      a CDR1 having the amino acid sequence of SEQ ID NO: 5,
      a CDR2 having the amino acid sequence of SEQ ID NO: 6, and
      a CDR3 having the amino acid sequence of SEQ ID NO: 7,
      wherein one or more of the following substitutions are optionally present:
         A) substitution of asparagine with lysine at position 10 in the amino acid sequence of SEQ ID NO: 2,
         B) substitution of glycine with glutamine, threonine, alanine, lysine, leucine or arginine at position 11 in the amino acid sequence of SEQ ID NO: 2,
         C) substitution of asparagine with glutamine at position 4 in the amino acid sequence of SEQ ID NO: 3,
         D) substitution of leucine with isoleucine at position 5 in the amino acid sequence of SEQ ID NO: 3,
         E) substitution of leucine with isoleucine at position 4 in the amino acid sequence of SEQ ID NO: 4,
         F) substitution of tyrosine with phenylalanine at position 6 in the amino acid sequence of SEQ ID NO: 4,
         G) substitution of serine with threonine at position 16 in the amino acid sequence of SEQ ID NO: 6, and
         H) substitution of aspartic acid with glutamic acid at position 19 in the amino acid sequence of SEQ ID NO: 6;
   2) a light chain variable region including
      a CDR1 having the amino acid sequence of SEQ ID NO: 2,
      a CDR2 having the amino acid sequence of SEQ ID NO: 3, and
      a CDR3 having the amino acid sequence of SEQ ID NO: 4, and
      a heavy chain variable region including
      a CDR1 having the amino acid sequence of SEQ ID NO: 8,
      a CDR2 having the amino acid sequence of SEQ ID NO: 6, and
      a CDR3 having the amino acid sequence of SEQ ID NO: 7;
   3) a light chain variable region including
      a CDR1 having the amino acid sequence of SEQ ID NO: 2,
      a CDR2 having the amino acid sequence of SEQ ID NO: 9, and
      a CDR3 having the amino acid sequence of SEQ ID NO: 10, and
      a heavy chain variable region including
      a CDR1 having the amino acid sequence of SEQ ID NO: 8,
      a CDR2 having the amino acid sequence of SEQ ID NO: 6, and
      a CDR3 having the amino acid sequence of SEQ ID NO: 11;
   4) a light chain variable region including
      a CDR1 having the amino acid sequence of SEQ ID NO: 2,
      a CDR2 having the amino acid sequence of SEQ ID NO: 3, and
      a CDR3 having the amino acid sequence of SEQ ID NO: 10, and
      a heavy chain variable region including
      a CDR1 having the amino acid sequence of SEQ ID NO: 12,
      a CDR2 having the amino acid sequence of SEQ ID NO: 6, and
      a CDR3 having the amino acid sequence of SEQ ID NO: 13;
   5) a light chain variable region including
      a CDR1 having the amino acid sequence of SEQ ID NO: 14,
      a CDR2 having the amino acid sequence of SEQ ID NO: 15, and
      a CDR3 having the amino acid sequence of SEQ ID NO: 16, and
      a heavy chain variable region including
      a CDR1 having the amino acid sequence of SEQ ID NO: 17,
      a CDR2 having the amino acid sequence of SEQ ID NO: 18, and
      a CDR3 having the amino acid sequence of SEQ ID NO: 19; or
   6) a light chain variable region including
      a CDR1 having the amino acid sequence of SEQ ID NO: 20,
      a CDR2 having the amino acid sequence of SEQ ID NO: 21, and
      a CDR3 having the amino acid sequence of SEQ ID NO: 22, and
      a heavy chain variable region including
      a CDR1 having the amino acid sequence of SEQ ID NO: 23,
      a CDR2 having the amino acid sequence of SEQ ID NO: 24, and
      a CDR3 having the amino acid sequence of SEQ ID NO: 25.
(9) The antibody or an antibody fragment thereof according to (8), having:
   a light chain variable region including
   a CDR1 having the amino acid sequence of SEQ ID NO: 2,
   a CDR2 having the amino acid sequence of SEQ ID NO: 3, and
   a CDR3 having the amino acid sequence of SEQ ID NO: 4, and
   a heavy chain variable region including
   a CDR1 having the amino acid sequence of SEQ ID NO: 5,
   a CDR2 having the amino acid sequence of SEQ ID NO: 6, and
   a CDR3 having the amino acid sequence of SEQ ID NO: 7,
   wherein one or more of the following substitutions are present:
      A) substitution of asparagine with lysine at position 10 in the amino acid sequence of SEQ ID NO: 2;
      B) substitution of glycine with glutamine, threonine, alanine, lysine, leucine or arginine at position 11 in the amino acid sequence of SEQ ID NO: 2;
      C) substitution of asparagine with glutamine at position 4 in the amino acid sequence of SEQ ID NO: 3;
      D) substitution of leucine with isoleucine at position 5 in the amino acid sequence of SEQ ID NO: 3;
      E) substitution of leucine with isoleucine at position 4 in the amino acid sequence of SEQ ID NO: 4;
      F) substitution of tyrosine with phenylalanine at position 6 in the amino acid sequence of SEQ ID NO: 4;
      G) substitution of serine with threonine at position 16 in the amino acid sequence of SEQ ID NO: 6; and
      H) substitution of aspartic acid with glutamic acid at position 19 in the amino acid sequence of SEQ ID NO: 6.
(10) The antibody or an antibody fragment thereof according to (9), wherein one or more of the following substitutions are present:
   A) substitution of asparagine with lysine at position 10 in the amino acid sequence of SEQ ID NO: 2;
   B) substitution of glycine with leucine or arginine at position 11 in the amino acid sequence of SEQ ID NO: 2; and
   C) substitution of asparagine with glutamine at position 4 in the amino acid sequence of SEQ ID NO: 3.
(11) The antibody or an antibody fragment thereof according to (9) or (10), wherein asparagine at position 4 in the amino acid sequence of SEQ ID NO: 3 is substituted with glutamine; and further, any one of the following substitutions are optionally present:
   A) substitution of asparagine with lysine at position 10 in the amino acid sequence of SEQ ID NO: 2; and
   B) substitution of glycine with leucine or arginine at position 11 in the amino acid sequence of SEQ ID NO: 2.
(12) The antibody or an antibody fragment thereof according to (11), wherein asparagine at position 4 in the amino acid sequence of SEQ ID NO: 3 is substituted with glutamine; and glycine at position 11 in the amino acid sequence of SEQ ID NO: 2 is substituted with arginine.
(13) The antibody or an antibody fragment thereof according to (8), having
   a light chain variable region including
   a CDR1 having the amino acid sequence of SEQ ID NO: 2,
   a CDR2 having the amino acid sequence of SEQ ID NO: 9, and
   a CDR3 having the amino acid sequence of SEQ ID NO: 10, and
   a heavy chain variable region including
   a CDR1 having the amino acid sequence of SEQ ID NO: 8,
   a CDR2 having the amino acid sequence of SEQ ID NO: 6, and
   a CDR3 having the amino acid sequence of SEQ ID NO: 11,
   wherein one or more of the following substitutions are present:
      A) substitution of serine with threonine at position 2 in the amino acid sequence of SEQ ID NO: 2;
      B) substitution of serine with threonine at position 3 in the amino acid sequence of SEQ ID NO: 2;
      C) substitution of serine with threonine at position 5 in the amino acid sequence of SEQ ID NO: 2;
      D) substitution of glutamine with asparagine at position 2 in the amino acid sequence of SEQ ID NO: 10;
      E) substitution of threonine with serine at position 1 in the amino acid sequence of SEQ ID NO: 8;
      F) substitution of alanine with valine at position 14 in the amino acid sequence of SEQ ID NO: 6;
      G) substitution of lysine with arginine at position 18 in the amino acid sequence of SEQ ID NO: 6;
      H) substitution of aspartic acid with glutamic acid at position 19 in the amino acid sequence of SEQ ID NO: 6;
      I) substitution of asparagine with glutamine at position 5 in the amino acid sequence of SEQ ID NO: 11; and
      J) substitution of tyrosine with phenylalanine at position 12 in the amino acid sequence of SEQ ID NO: 11.
(14) The antibody or an antibody fragment thereof according to any one of (7) to (13), which is a humanized monoclonal antibody or an antibody fragment thereof.
(15) The humanized monoclonal antibody or an antibody fragment thereof according to (14), having
   a light chain variable region having
   the amino acid sequence of SEQ ID NO: 40, 42, 43, 44, 45, or 47, or
   an amino acid sequence having 95% or more identity to the amino acid sequence of SEQ ID NO: 40, 42, 43, 44, 45, or 47, and
   a heavy chain variable region having
   the amino acid sequence of SEQ ID NO: 41 or 46, or
   an amino acid sequence having 95% or more identity to the amino acid sequence of SEQ ID NO: 41 or 46.
(16) The humanized monoclonal antibody or an antibody fragment thereof according to (14) or (15), having:
   1) a light chain variable region having the amino acid sequence of SEQ ID NO: 40, and a heavy chain variable region having the amino acid sequence of SEQ ID NO: 41;
   2) a light chain variable region having the amino acid sequence of SEQ ID NO: 42, and a heavy chain variable region having the amino acid sequence of SEQ ID NO: 41;
   3) a light chain variable region having the amino acid sequence of SEQ ID NO: 43, and a heavy chain variable region having the amino acid sequence of SEQ ID NO: 41;
   4) a light chain variable region having the amino acid sequence of SEQ ID NO: 44, and a heavy chain variable region having the amino acid sequence of SEQ ID NO: 41;
   5) a light chain variable region having the amino acid sequence of SEQ ID NO: 45, and a heavy chain variable region having the amino acid sequence of SEQ ID NO: 41;
   6) a light chain variable region having the amino acid sequence of SEQ ID NO: 47, and
      a heavy chain variable region having the amino acid sequence of SEQ ID NO: 41;
   7) a light chain variable region having the amino acid sequence of SEQ ID NO: 40, and
      a heavy chain variable region having the amino acid sequence of SEQ ID NO: 46;
   8) a light chain variable region having the amino acid sequence of SEQ ID NO: 42, and
      a heavy chain variable region having the amino acid sequence of SEQ ID NO: 46;
   9) a light chain variable region having the amino acid sequence of SEQ ID NO: 43, and
      a heavy chain variable region having the amino acid sequence of SEQ ID NO: 46;
   10) a light chain variable region having the amino acid sequence of SEQ ID NO: 44, and
      a heavy chain variable region having the amino acid sequence of SEQ ID NO: 46;
   11) a light chain variable region having the amino acid sequence of SEQ ID NO: 45, and
      a heavy chain variable region having the amino acid sequence of SEQ ID NO: 46; or
   12) a light chain variable region having the amino acid sequence of SEQ ID NO: 47, and
      a heavy chain variable region having the amino acid sequence of SEQ ID NO: 46, wherein one or more of the following substitutions are optionally present:
      A) substitution of asparagine with lysine at position 33 in the amino acid sequence of SEQ ID NO: 40, 42, 43, 44, 45 or 47;
      B) substitution of glycine with glutamine, threonine, alanine, lysine, leucine or arginine at position 34 in the amino acid sequence of SEQ ID NO: 40, 42, 43, 44, 45 or 47;
      C) substitution of asparagine with glutamine at position 58 in the amino acid sequence of SEQ ID NO: 40, 42, 43, 44, 45 or 47;
      D) substitution of leucine with isoleucine at position 59 in the amino acid sequence of SEQ ID NO: 40, 42, 43, 44, 45 or 47;
      E) substitution of leucine with isoleucine at position 97 in the amino acid sequence of SEQ ID NO: 40, 42, 43, 44, 45 or 47;
      F) substitution of tyrosine with phenylalanine at position 99 in the amino acid sequence of SEQ ID NO: 40, 42, 43, 44, 45 or 47;
      G) substitution of serine with threonine at position 65 in the amino acid sequence of SEQ ID NO: 41 or 46; and
      H) substitution of aspartic acid with glutamic acid at position 68 in the amino acid sequence of SEQ ID NO: 41 or 46.
(17) The humanized monoclonal antibody or an antibody fragment thereof according to (16), wherein one or more of the following substitutions are present:
   A) substitution of asparagine with lysine at position 33 in the amino acid sequence of SEQ ID NO: 40, 42, 43, 44, 45 or 47;
   B) substitution of glycine with glutamine, threonine, alanine, lysine, leucine or arginine at position 34 in the amino acid sequence of SEQ ID NO: 40, 42, 43, 44, 45 or 47;
   C) substitution of asparagine with glutamine at position 58 in the amino acid sequence of SEQ ID NO: 40, 42, 43, 44, 45 or 47;
   D) substitution of leucine with isoleucine at position 59 in the amino acid sequence of SEQ ID NO: 40, 42, 43, 44, 45 or 47;
   E) substitution of leucine with isoleucine at position 97 in the amino acid sequence of SEQ ID NO: 40, 42, 43, 44, 45 or 47;
   F) substitution of tyrosine with phenylalanine at position 99 in the amino acid sequence of SEQ ID NO: 40, 42, 43, 44, 45 or 47;
   G) substitution of serine with threonine at position 65 in the amino acid sequence of SEQ ID NO: 41 or 46; and
   H) substitution of aspartic acid with glutamic acid at position 68 in the amino acid sequence of SEQ ID NO: 41 or 46.
(18) The humanized monoclonal antibody or an antibody fragment thereof according to (17), having
   a light chain variable region having the amino acid sequence of SEQ ID NO: 40 or 42, and
   a heavy chain variable region having the amino acid sequence of SEQ ID NO: 41, wherein one or more of the following substitutions are present:
      A) substitution of asparagine with lysine at position 33 in the amino acid sequence of SEQ ID NO: 40 or 42;
      B) substitution of glycine with glutamine, threonine, alanine, lysine, leucine or arginine at position 34 in the amino acid sequence of SEQ ID NO: 40 or 42;
      C) substitution of asparagine with glutamine at position 58 in the amino acid sequence of SEQ ID NO: 40 or 42;
      D) substitution of leucine with isoleucine at position 59 in the amino acid sequence of SEQ ID NO: 40 or 42;
      E) substitution of leucine with isoleucine at position 97 in the amino acid sequence of SEQ ID NO: 40 or 42;
      F) substitution of tyrosine with phenylalanine at position 99 in the amino acid sequence of SEQ ID NO: 40 or 42;
      G) substitution of serine with threonine at position 65 in the amino acid sequence of SEQ ID NO: 41; and
      H) substitution of aspartic acid with glutamic acid at position 68 in the amino acid sequence of SEQ ID NO: 41.
(19) The humanized monoclonal antibody or an antibody fragment thereof according to (17) or (18), having:
   a light chain variable region having the amino acid sequence of SEQ ID NO: 40 or 42, and
   a heavy chain variable region having the amino acid sequence of SEQ ID NO: 41,
   wherein one or more of the following substitutions are present:
      A) substitution of asparagine with lysine at position 33 in the amino acid sequence of SEQ ID NO: 40 or 42;
      B) substitution of glycine with leucine or arginine at position 34 in the amino acid sequence of SEQ ID NO: 40 or 42; and
      C) substitution of asparagine with glutamine at position 58 in the amino acid sequence of SEQ ID NO: 40 or 42.
(20) The humanized monoclonal antibody or an antibody fragment thereof according to any one of (17) to (19), having
   a light chain variable region having the amino acid sequence of SEQ ID NO: 40 or 42, and
   a heavy chain variable region having the amino acid sequence of SEQ ID NO: 41, wherein
   asparagine at position 58 in the amino acid sequence of SEQ ID NO: 40 or 42 is substituted with glutamine, and
   further, any one of the following substitutions are optionally present:
      A) substitution of asparagine with lysine at position 33 in the amino acid sequence of SEQ ID NO: 40 or 42; and
      B) substitution of glycine with leucine or arginine at position 34 in the amino acid sequence of SEQ ID NO: 40 or 42.
(21) The humanized monoclonal antibody or an antibody fragment thereof according to (20), having
   a light chain variable region having the amino acid sequence of SEQ ID NO: 42, and a heavy chain variable region having the amino acid sequence of SEQ ID NO: 41, wherein asparagine at position 58 in the amino acid sequence of SEQ ID NO: 42 is substituted with glutamine, and glycine at position 34 in the amino acid sequence of SEQ ID NO: 42 is substituted with arginine.
(22) The humanized monoclonal antibody or an antibody fragment thereof according to (20), having
   a light chain variable region having the amino acid sequence of SEQ ID NO: 59, and a heavy chain variable region having the amino acid sequence of SEQ ID NO: 41.
(23) The humanized monoclonal antibody or an antibody fragment thereof according to (14), having:
   a light chain variable region having
   the amino acid sequence of SEQ ID NO: 54, 55, or, 56, or
   an amino acid sequence having 95% or more identity to the amino acid sequence of SEQ ID NO: 54, 55, or, 56; and
   a heavy chain variable region having
   the amino acid sequence of SEQ ID NO: 57 or 58, or
   an amino acid sequence having 95% or more identity to the amino acid sequence of SEQ ID NO: 57 or 58.
(24) The humanized monoclonal antibody or an antibody fragment thereof according to (14) or (23), having:
   1) a light chain variable region having the amino acid sequence of SEQ ID NO: 54, and
      a heavy chain variable region having the amino acid sequence of SEQ ID NO: 57;
   2) a light chain variable region having the amino acid sequence of SEQ ID NO: 55, and
      a heavy chain variable region having the amino acid sequence of SEQ ID NO: 57;
   3) a light chain variable region having the amino acid sequence of SEQ ID NO: 56, and
      a heavy chain variable region having the amino acid sequence of SEQ ID NO: 57;
   4) a light chain variable region having the amino acid sequence of SEQ ID NO: 54, and
      a heavy chain variable region having the amino acid sequence of SEQ ID NO: 58;
   5) a light chain variable region having the amino acid sequence of SEQ ID NO: 55, and
      a heavy chain variable region having the amino acid sequence of SEQ ID NO: 58;
   6) a light chain variable region having the amino acid sequence of SEQ ID NO: 56, and
      a heavy chain variable region having the amino acid sequence of SEQ ID NO: 58,
      wherein one or more of the following substitutions are optionally present:
      A) substitution of serine with threonine at position 25 in the amino acid sequence of SEQ ID NO: 54, 55 or 56;
      B) substitution of serine with threonine at position 26 in the amino acid sequence of SEQ ID NO: 54, 55 or 56;
      C) substitution of serine with threonine at position 28 in the amino acid sequence of SEQ ID NO: 54, 55 or 56;
      D) substitution of glutamine with asparagine at position 95 in the amino acid sequence of SEQ ID NO: 54, 55 or 56;
      E) substitution of threonine with serine at position 31 in the amino acid sequence of SEQ ID NO: 57 or 58;
      F) substitution of alanine with valine at position 63 in the amino acid sequence of SEQ ID NO: 57 or 58;
      G) substitution of lysine with arginine at position 67 in the amino acid sequence of SEQ ID NO: 57 or 58;
      H) substitution of aspartic acid with glutamic acid at position 68 in the amino acid sequence of SEQ ID NO: 57 or 58;
      I) substitution of asparagine with glutamine at position 99 in the amino acid sequence of SEQ ID NO: 57 or 58; and
      J) substitution of tyrosine with phenylalanine at position 105 in the amino acid sequence of SEQ ID NO: 57 or 58.
(25) The humanized monoclonal antibody or an antibody fragment thereof according to (24), having a light chain variable region having the amino acid sequence of SEQ ID NO: 56, and a heavy chain variable region having the amino acid sequence of SEQ ID NO: 57, wherein one or more of the following substitutions are optionally present:
   A) substitution of serine with threonine at position 25 in the amino acid sequence of SEQ ID NO: 56;
   B) substitution of serine with threonine at position 26 in the amino acid sequence of SEQ ID NO: 56;
   C) substitution of serine with threonine at position 28 in the amino acid sequence of SEQ ID NO: 56;
   D) substitution of glutamine with asparagine at position 95 in the amino acid sequence of SEQ ID NO: 56;
   E) substitution of threonine with serine at position 31 in the amino acid sequence of SEQ ID NO: 57;
   F) substitution of alanine with valine at position 63 in the amino acid sequence of SEQ ID NO: 57;
   G) substitution of lysine with arginine at position 67 in the amino acid sequence of SEQ ID NO: 57;
   H) substitution of aspartic acid with glutamic acid at position 68 in the amino acid sequence of SEQ ID NO: 57;
   I) substitution of asparagine with glutamine at position 99 in the amino acid sequence of SEQ ID NO: 57; and
   J) substitution of tyrosine with phenylalanine at position 105 in the amino acid sequence of SEQ ID NO: 57.
(26) The humanized monoclonal antibody or an antibody fragment thereof according to any one of (14) to (25), further having:
   a light chain constant region having the amino acid sequence of SEQ ID NO: 52, and
   a heavy chain constant region having the amino acid sequence of SEQ ID NO: 53,
   wherein lysine is optionally added to the C-terminal end of SEQ ID NO: 53.
(27) A pharmaceutical composition comprising the antibody or an antibody fragment thereof according to any one of (1) to (26).
(28) A pharmaceutical composition comprising the antibody or an antibody fragment thereof according to any one of (1) to (26), for use in inhibiting a binding of CCR8 to a CCR8 ligand.
(29) A pharmaceutical composition comprising the antibody or an antibody fragment thereof according to any one of (1) to (26), for use as a neutralizing antibody.
(30) A pharmaceutical composition comprising the monoclonal antibody or an antibody fragment thereof according to any one of (1) to (26) that binds to CCR8, for use in recognizing tyrosine at position 17 in the amino acid sequence set forth in SEQ ID NO: 1.
(31) The pharmaceutical composition according to (30), for use in inhibiting a binding of CCR8 to a CCR8 ligand.
(32) The pharmaceutical composition according to any one of (27) to (31), wherein the antibody has ADCC activity.
(33) The pharmaceutical composition according to any one of (27) to (31), wherein the antibody is an IgG antibody.
(34) The pharmaceutical composition according to any one of (27) to (33), for use in treating cancer.
(35) A polynucleotide encoding a light chain variable region or a heavy chain variable region of the antibody according to any one of (7) to (18).
(36) An expression vector comprising the polynucleotide according to (35).

(51) A monoclonal antibody or an antibody fragment thereof that binds to CCR8, which recognizes a site on an antigen recognized by the antibody according to any one of (7) to (19).
(52) The antibody or an antibody fragment thereof according to (51), which is a neutralizing antibody.
(53) The antibody or an antibody fragment according to (51) or (52), which is a humanized monoclonal antibody or an antibody fragment thereof.

(54) A monoclonal antibody or an antibody fragment thereof that has neutralizing activity against human CCR8 and competes with the antibody according to any one of (7) to (26) for binding to human CCR8.
(55) A monoclonal antibody or an antibody fragment thereof that has neutralizing activity against human CCR8 and competes for binding to human CCR8 with an antibody having a light chain variable region having the amino acid sequence of SEQ ID NO: 42 and a heavy chain variable region having an amino acid sequence of SEQ ID NO: 41.
(56) A monoclonal antibody or an antibody fragment thereof that recognizes an epitope that is entirely or partially identical to an epitope of an antibody having a light chain variable region having the amino acid sequence of SEQ ID NO: 42 and a heavy chain variable region having the amino acid sequence of SEQ ID NO: 41.
(57) A monoclonal antibody or an antibody fragment thereof that has neutralizing activity against human CCR8 and competes for binding to human CCR8 with an antibody having a light chain variable region having the amino acid sequence of SEQ ID NO: 42 and a heavy chain variable region having the amino acid sequence of SEQ ID NO: 41 wherein asparagine at position 58 in the amino acid sequence of SEQ ID NO: 42 is substituted with glutamine, and glycine at position 34 in the amino acid sequence of SEQ ID NO: 42 is substituted with arginine.
(58) A monoclonal antibody or an antibody fragment thereof that recognizes an epitope that is entirely or partially identical to an epitope of an antibody having a light chain variable region having the amino acid sequence of SEQ ID NO: 42 and a heavy chain variable region having the amino acid sequence of SEQ ID NO: 41 wherein asparagine at position 58 in the amino acid sequence of SEQ ID NO: 42 is substituted with glutamine, and glycine at position 34 in the amino acid sequence of SEQ ID NO: 42 is substituted with arginine.
(59) A monoclonal antibody or an antibody fragment thereof according to any one of (54) to (58), which is an antibody or an antibody fragment thereof that recognizes tyrosine at position 17 in the amino acid sequence set forth in SEQ ID NO: 1.
(60) The antibody or an antibody fragment thereof according to (59), which further recognizes one or more of amino acids at positions 94 to 107 in the amino acid sequence set forth in SEQ ID NO: 1.
(61) The antibody or an antibody fragment thereof according to (59) or (60), which further recognizes one or more of amino acids at positions 172 to 202 in the amino acid sequence set forth in SEQ ID NO: 1.
(62) The antibody or an antibody fragment thereof according to any one of (59) to (61), wherein the antibody or an antibody fragment thereof recognizes one or more amino acids of isoleucine at position 20, serine at position 22, lysine at position 35, leucine at position 181, cysteine at position 183, and asparagine at position 188 in the amino acid sequence set forth in SEQ ID NO: 1.
(63) The monoclonal antibody or an antibody fragment thereof according to any one of (54) to (62), which is a humanized monoclonal antibody or an antibody fragment thereof.
(64) A pharmaceutical composition comprising the monoclonal antibody or an antibody fragment thereof according to any one of (54) to (63).

(71) The antibody or an antibody fragment thereof according to any one of (1) to (5) or (7) to (13), which is produced by an antibody-producing hybridoma produced by using a human CCR8 gene as an antigen.

(81) A monoclonal antibody or an antibody fragment thereof that binds to human CCR8, which recognizes tyrosine at position 17 in the amino acid sequence set forth in SEQ ID NO: 1, wherein the monoclonal antibody or an antibody fragment thereof excludes the following antibodies:
a) an anti-CCR8 antibody shown by 414B, 414C, 414E, 433H, 459M, 464A, 464B, 433B and 455AL, as described in WO 2007/044756;
b) an anti-CCR8 antibody shown by 3B10, 2D10, and 5B11, as described in the article by Qu et al. (J. Exp. Med., (2004), 200(10), 1231-1241);
c) an anti-CCR8 antibody of product number L263G8 by BioLegend;
d) an anti-CCR8 antibody of product number 191704 by R&D Systems, Inc.

(91) A method for inhibiting a binding of CCR8 to a CCR8 ligand, comprising using the antibody or an antibody fragment thereof according to any one of (1) to (26).
(92) A method for treating cancer, comprising administering the antibody or an antibody fragment thereof according to any one of (1) to (26).
(93) A method for recognizing tyrosine at position 17 in the amino acid sequence set forth in SEQ ID NO: 1, using the monoclonal antibody or an antibody fragment thereof that binds to CCR8 according to any one of (7) to (26).
(94) Use of the monoclonal antibody or an antibody fragment thereof according to any one of (1) to (26), for producing a medicament that inhibits a binding of CCR8 to a CCR8 ligand.
(95) Use of the antibody or an antibody fragment thereof according to any one of (1) to (26), for producing a therapeutic agent for cancer.
(96) Use of the monoclonal antibody or an antibody fragment thereof according to any one of (1) to (26) that binds to CCR8, for producing an agent that recognizes tyrosine at position 17 in the amino acid sequence set forth in SEQ ID NO: 1.
(97) An antibody or an antibody fragment thereof according to any one of (1) to (26), for use in inhibiting a binding of CCR8 to a CCR8 ligand.
(98) An antibody or an antibody fragment thereof according to any one of (1) to (26), for use in treating cancer.
(99) A monoclonal antibody or an antibody fragment thereof according to any one of (1) to (26) that binds to CCR8, for use in recognizing tyrosine at position 17 in the amino acid sequence set forth in SEQ ID NO: 1.

### [EFFECTS OF THE INVENTION]

The monoclonal antibody or an antibody fragment thereof of the present invention specifically binds to human CCR8 and can thus be used for detecting human CCR8 in a biological sample. Furthermore, the monoclonal antibody or an antibody fragment thereof of the present invention has the activity to selectively inhibit human CCR8. Thus, a pharmaceutical composition comprising the monoclonal antibody or an antibody fragment thereof of the present invention is very useful as a medicament, particularly as a medicament for the treatment or prevention of human CCR8-related diseases.

### [BRIEF DESCRIPTION OF DRAWINGS]

Figure 1 shows an amino acid sequence comparison of human CCR8, human CCR4, and each chimera in which the N-terminal region, loop1 region, loop2 region, or loop3 region, which are the extracellular domains of human CCR8, is replaced with a corresponding N-terminal region, loop1 region, loop2 region, or loop3 region of human CCR4, respectively.
Figure 2 shows the Kabat numbering and alignment results for light chain variable regions of 10A11, 27G1, 1H4, 19D7, 8F7 and 2C7.
Figure 3 shows the Kabat numbering and alignment results for heavy chain variable regions of 10A11, 27G1, 1H4, 19D7, 8F7 and 2C7.
Figure 4 is the Kabat numbering and alignment results for the light chain variable region of 10A11, IGKV4-1, IGKV3-20, IGKV1-39, IGKV2-40, IGKV2-28, and IGKV1-16.
Figure 5 is the Kabat numbering and alignment results for the heavy chain variable region of 10A11, IGHV3-15 T94R and IGHV3-73.
Figure 6 is the Kabat numbering and alignment results for the light chain variable region of 19D7, IGKV3-15, IGKV2-18 and IGKV3-20.
Figure 7 is the Kabat numbering and alignment results for the heavy chain variable region of 19D7, IGHV3-15 T94R and IGHV3-73.
Figure 8 is the anti-tumor activity of anti-human CCR8 antibodies which was evaluated in human CCR8 knock-in mice inoculated with colon cancer-derived CT26 cells by measuring the tumor volume after inoculation. For significance levels, ** indicates p < 0.01 and *** indicates p < 0.001 by Welch's t test.

### [MODE FOR CARRYING OUT THE INVENTION]

The term used in the present description is used in the meaning usually used in the art, unless specifically mentioned.

Antibody-producing techniques known in the art can be used in the present invention. Examples of the techniques include a method described in Immunochemistry in Practice (Blackwell Scientific Publiations).

Genetically engineered techniques known in the art can also be used. Examples of the techniques include methods described in Molecular Cloning, A Laboratory Manual, Forth Edition, Cold Spring Harbor Laboratory Press (2012), and Current Protocols Essential Laboratory Techniques, Current Protocols (2012).

The amino acid sequence of human CCR8 is shown in UniProtKB/Swiss-Prot: P51685 (SEQ ID NO: 1). The extramembrane domains of human CCR8 correspond to the N-terminal region consisting of amino acids positions 1-35, the loop1 region consisting of amino acids positions 94-107, the loop2 region consisting of amino acids positions 172-202, and the loop3 region consisting of amino acids positions 264-280. The amino acid at position 17 in the amino acid sequence of human CCR8 is tyrosine. In the amino acid sequence of mouse CCR8, the amino acid at position 17 is lacking. The monoclonal antibody or an antibody fragment thereof of the present invention has no binding activity to mouse CCR8, as shown in Table 2 of Example 3.

As a monoclonal antibody to mouse CCR8, an antibody SA214G2 is available from BioLegend. The antibody has neutralizing activity against mouse CCR8. The present inventors have confirmed that this antibody does not exhibit binding activity to human CCR8 and does not recognize tyrosine at position 17 in the amino acid sequence of human CCR8. The present inventors have also confirmed that the antibody recognizes phenylalanine at position 27 in the amino acid sequence of mouse CCR8, and the amino acid is an important amino acid for expression of neutralizing activity against mouse CCR8. The amino acid at position 27 in the amino acid sequence of mouse CCR8 corresponds to the amino acid at position 29 in the amino acid sequence of human CCR8, when these sequences are aligned. The amino acid at position 29 in the amino acid sequence of human CCR8 is leucine. It is considered that, due to this difference, the monoclonal antibody having neutralizing activity against mouse CCR8 has no binding activity to human CCR8.

The hybridoma producing the anti-CCR8 antibody of the present invention can be produced by using, as an immunogen, a human CCR8 protein, a gene encoding the full length of human CCR8, a human CCR8 expressing cell, or the like. When a gene encoding the full length of human CCR8 is used an immunogen, a hybridoma producing the anti-CCR8 antibody can be prepared, for example, by fusing a spleen cell of a mouse which has been DNA-immunized with the gene as an antigen into a mouse myeloma cell.

One aspect of the monoclonal antibody or an antibody fragment thereof of the present invention includes a monoclonal antibody or a fragment thereof having a CDR or a heavy/light chain variable region described in the present description. The antibody or an antibody fragment may be from any class or subclass of immunoglobulin molecule (e.g., IgG, IgE, IgM, IgD, or IgA, preferably, IgG). The antibody or antibody fragment may also be obtained from any species, such as mouse, rat, shark, rabbit, pig, hamster, camel, llama, goat, or human. The antibody or antibody fragment thereof is preferably a humanized monoclonal antibody or an antibody fragment of a humanized monoclonal antibody.

In the present description, an epitope refers to a region of an antigen that is bound by an antibody targeting the antigen. When the antigen is a protein, the epitope comprises a particular amino acid that directly contact with the antibody. The present invention encompasses not only monoclonal antibodies or antibody fragments thereof that recognize exactly the same epitope as the monoclonal antibody or an antibody fragment thereof of the present invention, but also monoclonal antibodies or antibody fragments thereof that recognize partially the same epitope as that.

The monoclonal antibody or an antibody fragment thereof of the present invention is a monoclonal antibody or an antibody fragment thereof that binds to human CCR8, which recognizes tyrosine at position 17 in the amino acid sequence of human CCR8.

Of the above monoclonal antibody or an antibody fragment thereof, the following monoclonal antibodies or antibody fragments thereof are preferred:
a monoclonal antibody or an antibody fragment thereof that binds to human CCR8, which recognizes one or more of amino acids at positions 94 to 107 in the amino acid sequence, which correspond to loop1 of human CCR8;
a monoclonal antibody or an antibody fragment thereof that binds to human CCR8, which recognizes one or more of amino acids at positions 172 to 202 in the amino acid sequence, which correspond to loop2 of human CCR8; and
a monoclonal antibody or an antibody fragment thereof that binds to human CCR8, which recognizes one or more amino acids of isoleucine at position 20, serine at position 22, lysine at position 35, leucine at position 181, cysteine at position 183, and asparagine at position 188 in the amino acid sequence of human CCR8.

Here, in the above preferred cases, a plurality of optionally selected epitopes in the epitopes described above may be recognized. Furthermore, the monoclonal antibody or an antibody fragment thereof of the present invention may further recognize an amino acid other than the above amino acids in the amino acid sequence of human CCR8.

The monoclonal antibody of the present invention is not particularly limited as long as it recognizes tyrosine at position 17 in the amino acid sequence of human CCR8, but in one aspect the CDR sequence of the monoclonal antibodies of the present invention preferably has the following sequence:
(1) light chain CDR1: SEQ ID NO: 2, 14 or 20;
(2) light chain CDR2: SEQ ID NO: 3, 9, 15, or 21;
(3) light chain CDR3: SEQ ID NO: 4, 10, 16, or 22;
(4) heavy chain CDR1: SEQ ID NO: 5, 8, 12, 17, or 23;
(5) heavy chain CDR2: SEQ ID NO: 6, 18, or 24;
(6) heavy chain CDR3: SEQ ID NO: 7, 11, 13, 19, or 25.

More preferably, the CDR sequence has the following sequence:
(1) light chain CDR1: SEQ ID NO: 2;
(2) light chain CDR2: SEQ ID NO: 3, or 9;
(3) light chain CDR3: SEQ ID NO: 4, or 10;
(4) heavy chain CDR1: SEQ ID NO: 5, 8, or 12;
(5) heavy chain CDR2: SEQ ID NO: 6;
(6) heavy chain CDR3: SEQ ID NO: 7, 11, or 13.

Most preferably, the CDR sequence has the following sequence:
(1) light chain CDR1: SEQ ID NO: 2;
(2) light chain CDR2: SEQ ID NO: 3;
(3) light chain CDR3: SEQ ID NO: 4;
(4) heavy chain CDR1: SEQ ID NO: 5;
(5) heavy chain CDR2: SEQ ID NO: 6;
(6) heavy chain CDR3: SEQ ID NO: 7.

Each of the amino acid sequences (SEQ ID NO: 2 to 25) optionally has deletion, substitution, insertion and/or addition of one or two amino acid.

Herein, in the CDR sequence of the monoclonal antibody of the present invention, asparagine may be substituted with glutamine or lysine, aspartic acid may be substituted with glutamic acid, leucine may be substituted with isoleucine, tyrosine may be substituted with phenylalanine, serine may be substituted with threonine, and glycine may be substituted with glutamine, threonine, alanine, lysine, leucine, or arginine.

Preferably, one or more of the following substitutions are optionally present:
A) substitution of asparagine with lysine at position 10 in the amino acid sequence of SEQ ID NO: 2;
B) substitution of glycine with glutamine, threonine, alanine, lysine, leucine or arginine at position 11 in the amino acid sequence of SEQ ID NO: 2;
C) substitution of asparagine with glutamine at position 4 in the amino acid sequence of SEQ ID NO: 3;
D) substitution of leucine with isoleucine at position 5 in the amino acid sequence of SEQ ID NO: 3;
E) substitution of leucine with isoleucine at position 4 in the amino acid sequence of SEQ ID NO: 4;
F) substitution of tyrosine with phenylalanine at position 6 in the amino acid sequence of SEQ ID NO: 4;
G) substitution of serine with threonine at position 16 in the amino acid sequence of SEQ ID NO: 6; and
H) substitution of aspartic acid with glutamic acid at position 19 in the amino acid sequence of SEQ ID NO: 6.

More preferably, one or more of the following substitutions are optionally present:
A) substitution of asparagine with lysine at position 10 in the amino acid sequence of SEQ ID NO: 2;
B) substitution of glycine with leucine or arginine at position 11 in the amino acid sequence of SEQ ID NO: 2; and
C) substitution of asparagine with glutamine at position 4 in the amino acid sequence of SEQ ID NO: 3.

Further preferably, asparagine at position 4 in the amino acid sequence of SEQ ID NO: 3 is substituted with glutamine; and further, any one of the following substitutions are optionally present:
A) substitution of asparagine with lysine at position 10 in the amino acid sequence of SEQ ID NO: 2; and
B) substitution of glycine with leucine or arginine at position 11 in the amino acid sequence of SEQ ID NO: 2.

When the monoclonal antibody of the present invention has the following sequence:
(1) light chain CDR1: SEQ ID NO: 2;
(2) light chain CDR2: SEQ ID NO: 9;
(3) light chain CDR3: SEQ ID NO: 10;
(4) heavy chain CDR1: SEQ ID NO: 8;
(5) heavy chain CDR2: SEQ ID NO: 6;
(6) heavy chain CDR3: SEQ ID NO: 11,
   one or more of the following substitutions are optionally present:
   A) substitution of serine with threonine at position 2 in the amino acid sequence of SEQ ID NO: 2;
   B) substitution of serine with threonine at position 3 in the amino acid sequence of SEQ ID NO: 2;
   C) substitution of serine with threonine at position 5 in the amino acid sequence of SEQ ID NO: 2;
   D) substitution of glutamine with asparagine at position 2 in the amino acid sequence of SEQ ID NO: 10;
   E) substitution of threonine with serine at position 1 in the amino acid sequence of SEQ ID NO: 8;
   F) substitution of alanine with valine at position 14 in the amino acid sequence of SEQ ID NO: 6;
   G) substitution of lysine with arginine at position 18 in the amino acid sequence of SEQ ID NO: 6;
   H) substitution of aspartic acid with glutamic acid at position 19 in the amino acid sequence of SEQ ID NO: 6;
   I) substitution of asparagine with glutamine at position 5 in the amino acid sequence of SEQ ID NO: 11; and
   J) substitution of tyrosine with phenylalanine at position 12 in the amino acid sequence of SEQ ID NO: 11.

In the present invention, the "antibody fragment of a monoclonal antibody" means a portion of the monoclonal antibody of the present invention and an fragment that specifically binds to human CCR8 and selectively inhibits human CCR8 in the same manner as the monoclonal antibody. The antibody fragment of the monoclonal antibody of the present invention recognizes tyrosine at position 17 in the amino acid sequence of human CCR8.

Specifically, examples of the antibody fragment include Fab (fragment of antigen binding), Fab', F(ab')₂, a single chain antibody (single chain Fv; hereinafter referred to as scFv), a disulfide stabilized antibody (disulfide stabilized Fv; hereinafter referred to as dsFv), a dimerized V-region fragment (hereinafter referred to as diabody), a peptide comprising CDR, and the like, which specifically bind to human CCR8 (Expert Opinion on Therapeutic Patents, vol. 6, No. 5, pp. 441-456, 1996).

Fab is an antibody fragment having an antigen-binding activity of about 50,000 molecular weight, composed of about half of the N-terminal end side of the H-chain and the entire L-chain obtained by degrading the peptide portion of the upper part of the two disulfide bonds (S-S bonds) crosslinking the two H-chains in the hinge region of IgG with an enzyme papain. Fab used in the present invention can be obtained by treating the monoclonal antibody of the present invention with papain. Fab can also be produced by inserting DNA encoding Fab of the monoclonal antibody of the present invention into a cell expression vector,and introducing the obtained vector into a cell to express Fab.

Fab' is an antibody fragment having an antigen-binding activity of about 50,000 molecular weight, obtained by cleaving S-S bonds in the hinge of F(ab')₂. Fab' used in the present invention can be obtained by treating F(ab')₂ of the monoclonal antibody of the present invention with a reducing agent dithiothreitol. Fab' can also be produced by inserting DNA encoding Fab' of the monoclonal antibody of the present invention into a cell expression vector, and introducing the obtained vector into E. coli, yeast, or an animal cell to express Fab'.

F(ab')₂ is an antibody fragment having an antigen-binding activity of about 100,000 molecular weight, composed of two Fab' regions bonded at the hinge portion obtained by degrading the lower part of the two S-S bonds in the hinge region of IgG with an enzyme pepsin. F(ab')₂ used in the present invention can be obtained by treating the monoclonal antibody of the present invention with pepsin. F(ab')₂ can also be produced by inserting DNA encoding F(ab')₂ of the monoclonal antibody of the present invention into a cell expression vector, and introducing the obtained vector into E. coli, yeast, or an animal cell to express F(ab')2.

scFv is a VH-P-VL or VL-P-VH polypeptide in which one VH and one VL are linked with an appropriate peptide linker (hereinafter referred to as P), and is an antibody fragment having antigen-binding activity. The VH and VL contained in scFv used in the present invention may be those of the monoclonal antibody of the present invention. scFv used in the present invention can also be produced by constructing a scFv expression vector using cDNA encoding the VH and VL of the monoclonal antibody of the present invention and introducing the obtained vector into E. coli, yeast, or an animal cell to express scFv.

dsFv refers to an antibody fragment obtained by bonding polypeptides in which 1 amino acid residue in VH and VL is substituted with a cysteine residue, respectively, via an S-S bond. The amino acid residues to be substituted with cysteine residues can be selected based on stereostructural predictions of the antibody according to the method shown by Reiter et al. (Protein Engineering, 7, 697 (1994)). The VH or VL contained in the dsFv used in the present invention may be those of the monoclonal antibody of the present invention. dsFv used in the present invention can also be produced by constructing a dsFv expression vector by inserting cDNA encoding the VH and VL of the monoclonal antibody of the present invention into an appropriate expression vector and introducing the obtained vector into E. coli, yeast, or an animal cell to express dsFv.

The diabody is an antibody fragment in which scFvs having the same or different antigen-binding specificity form a dimer, and is an antibody fragment having divalent antigen-binding activity for the same antigen or two different specific antigen-binding activities for different antigens. For example, a divalent diabody that specifically reacts to the monoclonal antibody of the present invention can be produced by using cDNA encoding the VH and VL of the monoclonal antibody of the present invention to construct a DNA encoding scFv having a peptide linker of 3 to 10 residues, inserting the DNA into a cell expression vector, introducing the obtained expression vector into E. coli, yeast, or an animal cell to express the diabody.

A peptide comprising a CDR is composed of at least one or more regions of a CDR of VH or VL. The plurality of CDRs can be bound directly or via an appropriate peptide linker. The peptide comprising a CDR used in the present invention can be produced by constructing a CDR-encoding DNA using cDNA encoding the VH and VL of the monoclonal antibody of the present invention, inserting the DNA into an animal cell expression vector, and introducing the obtained vector into E. coli, yeast, or an animal cell to express the peptide. The peptide comprising a CDR can also be produced by chemical synthesis methods such as the Fmoc method (fluorenylmethyloxycarbonyl method), the tBoc method (t-butyloxycarbonyl method), and the like.

The monoclonal antibody and an antibody fragment thereof of the present invention are characterized in that they bind to human CCR8. In particular, those that specifically bind to human CCR8 are preferred.

The specific binding can be characterized by an equilibrium dissociation constant of at least about 1 × 10⁻⁶ M or less (for example, the smaller Kd represents the closer binding). The Kd value is preferably 1 x 10⁻⁷ M or less, more preferably 1 x 10⁻⁸ M or less, still more preferably 1 x 10⁻⁹ M or less. Methods for determining whether two molecules specifically bind are well known in the art, and examples thereof include competitive ELISA methods, surface plasmon resonance, and the like other method.

The present invention includes a monoclonal antibody or an antibody fragment thereof competing with the monoclonal antibody or an antibody fragment thereof described in the present description for binding to human CCR8.

The "monoclonal antibody or an antibody fragment thereof competing with" means an antibody or an antibody fragment thereof that inhibits a specific binding to human CCR8 of the monoclonal antibody or an antibody fragment thereof of the present invention (preferably an antibody described in Examples of the present description, particularly preferably, 10A11. For example, an antibody comprising a light chain variable region having the amino acid sequence of SEQ ID NO: 42 and a heavy chain variable region having the amino acid sequence of SEQ ID NO: 41. An antibody comprising a light chain variable region having the amino acid sequence of SEQ ID NO: 42 and a heavy chain variable region having the amino acid sequence of SEQ ID NO: 41, wherein asparagine at position 58 in the amino acid sequence of SEQ ID NO: 42 is substituted with glutamine, and glycine at position 34 in the amino acid sequence of SEQ ID NO: 42 is substituted with arginine. Whether a monoclonal antibody or antibody fragment thereof competes with the monoclonal antibody or an antibody fragment thereof of the present invention is determined. Among antibodies for which binding to an antigen (human CCR8) could be confirmed under the coexistence of the isotype control antibody, the antibody for which the binding signal is significantly reduced under the coexistence of the monoclonal antibody or an antibody fragment thereof of the present invention can be identified as the antibody competing with the monoclonal antibody or an antibody fragment thereof of the present invention. The reduction in the binding signal is preferably 50%, more preferably 70%. The Ki value of the antibody competing with the monoclonal antibody or an antibody fragment thereof of the present invention for binding of the monoclonal antibody or an antibody fragment thereof of the present invention to an antigen is preferably 1 × 10⁻⁷ M or less, more preferably 1 × 10⁻⁸ M or less, still more preferably 1 × 10⁻⁹ M or less.

The neutralizing activity against human CCR8 of the antibody competing with the monoclonal antibody or an antibody fragment thereof of the present invention thus obtained is preferably an IC50 value of 10 nM or less.

The monoclonal antibody or an antibody fragment thereof of the present invention are characterized in that they inhibit a binding of CCR8 to a CCR8 ligand. The "CCR8 ligand" is not particularly limited as long as it is a substance that binds to CCR8, such as CCL1, CCL8, or CCL18, but is preferably CCL1, CCL18, and particularly preferably CCL1.

The inhibitory ability of binding of CCR8 to a CCR8 ligand can be determined, when the CCR8 ligand is human CCL1, for example, by using human CCR8-expressing 293 cells, determining Ca²⁺ influx by human CCL1 addition, and calculating an IC50 value with setting that the signal when human CCL1 is not added as the inhibition rate of 100% and the signal when human CCL1 is added and the antibody is not added as the inhibition rate of 0%. The inhibitory ability of binding to other CCR8 ligands can also be determined in a similar manner to human CCL1 as described above.

Human CCL1 has an amino acid sequence shown by UniProtKB/Swiss-Prot No. P22362 or the like. Human CCL8 has the amino acid sequence shown by GenBank No. AAI 26243.1 or the like. Human CCL18 has the amino acid sequence shown by GenBank No. EAW80102.1 or the like.

The monoclonal antibody of the present invention can be produced by a conventional method in the art using a CDR or heavy chain variable region/light chain variable region described in the present description.

The monoclonal antibody of the present invention also includes a chimeric antibody, a humanized antibody, a fully human antibody, an antibody-drug conjugate (ADC) and a bispecific antibody.
The humanized monoclonal antibody is useful when administered to humans for therapeutic purposes or the like, because it is less antigenic in humans. A humanized monoclonal antibody is an antibody in which a complementarity determining region (CDR) of a non-human mammal antibody, such as a mouse antibody, is implanted into a framework region (FR) of a human antibody. The FR of a humanized monoclonal antibody is thus derived from a human. Suitable FR can be selected by referring to the documents of Kabat E.A. et al. As the FR in this case, one with which the CDR can form an appropriate antigen binding site is selected. As necessary, amino acids of the FR of a variable region of the antibody may be substituted so that the CDR of the reconstituted humanized monoclonal antibody form an appropriate antigen binding site (Sato, K. et al., Cancer Res. 1993, vol. 53, p. 851). The percentage of amino acids of the FR to be substituted is from 0 to 15%, preferably from 0 to 5%, of the total FR region.

The humanized monoclonal antibody of the present invention preferably comprises:
a light chain variable region consisting of
the amino acid sequence of SEQ ID NO: 40, 42, 43, 44, 45, or 47, or
an amino acid sequence having 95% or more identity to the amino acid sequence of SEQ ID NO: 40, 42, 43, 44, 45, or 47; and
a heavy chain variable region consisting of
the amino acid sequence of SEQ ID NO: 41 or 46, or
an amino acid sequence having 95% or more identity to the amino acid sequence of SEQ ID NO: 41 or 46.

More preferably, the humanized monoclonal antibody of the present invention has:
1) a light chain variable region having the amino acid sequence of SEQ ID NO: 40, and
   a heavy chain variable region having the amino acid sequence of SEQ ID NO: 41;
2) a light chain variable region having the amino acid sequence of SEQ ID NO: 42, and
   a heavy chain variable region having the amino acid sequence of SEQ ID NO: 41;
3) a light chain variable region having the amino acid sequence of SEQ ID NO: 43, and
   a heavy chain variable region having the amino acid sequence of SEQ ID NO: 41;
4) a light chain variable region having the amino acid sequence of SEQ ID NO: 44, and
   a heavy chain variable region having the amino acid sequence of SEQ ID NO: 41;
5) a light chain variable region having the amino acid sequence of SEQ ID NO: 45, and
   a heavy chain variable region having the amino acid sequence of SEQ ID NO: 41;
6) a light chain variable region having the amino acid sequence of SEQ ID NO: 47, and
   a heavy chain variable region having the amino acid sequence of SEQ ID NO: 41;
7) a light chain variable region having the amino acid sequence of SEQ ID NO: 40, and
   a heavy chain variable region having the amino acid sequence of SEQ ID NO: 46;
8) a light chain variable region having the amino acid sequence of SEQ ID NO: 42, and
   a heavy chain variable region having the amino acid sequence of SEQ ID NO: 46;
9) a light chain variable region having the amino acid sequence of SEQ ID NO: 43, and
   a heavy chain variable region having the amino acid sequence of SEQ ID NO: 46;
10) a light chain variable region having the amino acid sequence of SEQ ID NO: 44, and
   a heavy chain variable region having the amino acid sequence of SEQ ID NO: 46;
11) a light chain variable region having the amino acid sequence of SEQ ID NO: 45, and
   a heavy chain variable region having the amino acid sequence of SEQ ID NO: 46; or
12) a light chain variable region having the amino acid sequence of SEQ ID NO: 47, and
   a heavy chain variable region having the amino acid sequence of SEQ ID NO: 46, wherein one or more of the following substitutions are optionally present:
   A) substitution of asparagine with lysine at position 33 in the amino acid sequence of SEQ ID NO: 40, 42, 43, 44, 45 or 47;
   B) substitution of glycine with glutamine, threonine, alanine, lysine, leucine or arginine at position 34 in the amino acid sequence of SEQ ID NO: 40, 42, 43, 44, 45 or 47;
   C) substitution of asparagine with glutamine at position 58 in the amino acid sequence of SEQ ID NO: 40, 42, 43, 44, 45 or 47;
   D) substitution of leucine with isoleucine at position 59 in the amino acid sequence of SEQ ID NO: 40, 42, 43, 44, 45 or 47;
   E) substitution of leucine with isoleucine at position 97 in the amino acid sequence of SEQ ID NO: 40, 42, 43, 44, 45 or 47;
   F) substitution of tyrosine with phenylalanine at position 99 in the amino acid sequence of SEQ ID NO: 40, 42, 43, 44, 45 or 47;
   G) substitution of serine with threonine at position 65 in the amino acid sequence of SEQ ID NO: 41 or 46; and
   H) substitution of aspartic acid with glutamic acid at position 68 in the amino acid sequence of SEQ ID NO: 41 or 46.

Further preferably, the humanized monoclonal antibody of the present invention has:
1) a light chain variable region having the amino acid sequence of SEQ ID NO: 40, and
   a heavy chain variable region having the amino acid sequence of SEQ ID NO: 41; or
2) a light chain variable region having the amino acid sequence of SEQ ID NO: 42, and
   a heavy chain variable region having the amino acid sequence of SEQ ID NO: 41, wherein one or more of the following substitutions are optionally present:
   A) substitution of asparagine with lysine at position 33 in the amino acid sequence of SEQ ID NO: 40 or 42;
   B) substitution of glycine with glutamine, threonine, alanine, lysine, leucine or arginine at position 34 in the amino acid sequence of SEQ ID NO: 40 or 42;
   C) substitution of asparagine with glutamine at position 58 in the amino acid sequence of SEQ ID NO: 40 or 42;
   D) substitution of leucine with isoleucine at position 59 in the amino acid sequence of SEQ ID NO: 40 or 42;
   E) substitution of leucine with isoleucine at position 97 in the amino acid sequence of SEQ ID NO: 40 or 42;
   F) substitution of tyrosine with phenylalanine at position 99 in the amino acid sequence of SEQ ID NO: 40 or 42;
   G) substitution of serine with threonine at position 65 in the amino acid sequence of SEQ ID NO: 41; and
   H) substitution of aspartic acid with glutamic acid at position 68 in the amino acid sequence of SEQ ID NO: 41.

Particularly preferably, the humanized monoclonal antibody of the present invention has:
1) a light chain variable region having the amino acid sequence of SEQ ID NO: 40, and
   a heavy chain variable region having the amino acid sequence of SEQ ID NO: 41; or
2) a light chain variable region having the amino acid sequence of SEQ ID NO: 42, and
   a heavy chain variable region having the amino acid sequence of SEQ ID NO: 41, wherein one or more of the following substitutions are optionally present:
   A) substitution of asparagine with lysine at position 33 in the amino acid sequence of SEQ ID NO: 40 or 42;
   B) substitution of glycine with leucine or arginine at position 34 in the amino acid sequence of SEQ ID NO: 40 or 42; and
   C) substitution of asparagine with glutamine at position 58 in the amino acid sequence of SEQ ID NO: 40 or 42.

Most preferably, the humanized monoclonal antibody of the present invention has:
1) a light chain variable region having the amino acid sequence of SEQ ID NO: 40, and
   a heavy chain variable region having the amino acid sequence of SEQ ID NO: 41; or
2) a light chain variable region having the amino acid sequence of SEQ ID NO: 42, and
   a heavy chain variable region having the amino acid sequence of SEQ ID NO: 41, wherein
   asparagine at position 58 in the amino acid sequence of SEQ ID NO: 40 or 42 is substituted with glutamine, and
   further, any one of the following substitutions are optionally present:
      A) substitution of asparagine with lysine at position 33 in the amino acid sequence of SEQ ID NO: 40 or 42; and
      B) substitution of glycine with leucine or arginine at position 34 in the amino acid sequence of SEQ ID NO: 40 or 42.

The humanized monoclonal antibody having a light chain variable region having the amino acid sequence of SEQ ID NO: 42 and a heavy chain variable region having the amino acid sequence of SEQ ID NO: 41, wherein asparagine at position 58 in the amino acid sequence of SEQ ID NO: 42 is substituted with glutamine, and glycine at position 34 in the amino acid sequence of SEQ ID NO: 42 is substituted with arginine is most preferable.

Examples of sequences of light and heavy chain variable regions in the humanized monoclonal antibodies of the present invention include combinations in Table 1.

**[Table 1]**

| Combination | Light chain variable region SEQ ID NO: | Type of substitution | Heavy chain variable region SEQ ID NO: | Type of substitution |
|---|---|---|---|---|
| 1 | 42 | C | 41 | None |
| 2 | 42 | C+A | 41 | None |
| 3 | 42 | C+A | 41 | H |
| 4 | 42 | C+B5 | 41 | None |
| 5 | 42 | C+B5 | 41 | H |
| 6 | 42 | C+B6 | 41 | None |
| 7 | 42 | C+B6 | 41 | H |
| 8 | 40 | C | 41 | None |
| 9 | 40 | C+A | 41 | None |
| 10 | 40 | C+A | 41 | H |
| 11 | 40 | C+B5 | 41 | None |
| 12 | 40 | C+B5 | 41 | H |
| 13 | 40 | C+B6 | 41 | None |
| 14 | 40 | C+B6 | 41 | H |
| 15 | 42 | D | 41 | None |
| 16 | 42 | E | 41 | None |
| 17 | 42 | F | 41 | None |
| 18 | 42 | B1 | 41 | None |
| 19 | 42 | B6 | 41 | None |
| 20 | 42 | B2+C | 41 | None |
| 21 | 42 | B3+C+D | 41 | None |
| 22 | 42 | None | 41 | H |
| 23 | 42 | C | 41 | H |
| 24 | 42 | None | 41 | G+H |
| 25 | 42 | A | 41 | G+H |
| 26 | 40 | D | 41 | None |
| 27 | 40 | E | 41 | None |
| 28 | 40 | F | 41 | None |
| 29 | 40 | B4 | 41 | None |
| 30 | 40 | B5 | 41 | None |
| 31 | 40 | None | 41 | H |
| 32 | 40 | A | 41 | H |

Herein each symbol in the "Type of substitution" in Table 1 respectively means the following substitutions.
(1) Light chain variable region having the amino acid sequence of SEQ ID NO: 40 or 42
   A) Substitution of asparagine with lysine at position 33 in the amino acid sequence.

   B1) Substitution of glycine with glutamine at position 34 in the amino acid sequence.
   B2) Substitution of glycine with threonine at position 34 in the amino acid sequence.
   B3) Substitution of glycine with alanine at position 34 in the amino acid sequence.
   B4) Substitution of glycine with lysine at position 34 in the amino acid sequence.
   B5) Substitution of glycine with leucine at position 34 in the amino acid sequence.
   B6) Substitution of glycine with arginine at position 34 in the amino acid sequence.
   C) Substitution of asparagine with glutamine at position 58 in the amino acid sequence.
   D) Substitution of leucine with isoleucine at position 59 in the amino acid sequence.
   E) Substitution of leucine with isoleucine at position 97 in the amino acid sequence.
   F) Substitution of tyrosine with phenylalanine at position 99 in the amino acid sequence.
(2) Heavy chain variable region having the amino acid sequence of SEQ ID NO: 41
   G) substitution of serine with threonine at position 65 in the amino acid sequence.
   H) Substitution of aspartic acid with glutamic acid at position 68 in the amino acid sequence.

Another aspect of the humanized monoclonal antibody of the present invention comprises:
a light chain variable region having
the amino acid sequence of SEQ ID NO: 40, 42, 43, 44, 45, or 47, or
an amino acid sequence having 95% or more identity to the amino acid sequence of SEQ ID NO: 40, 42, 43, 44, 45, or 47, wherein the amino acids at positions 26, 27, 30 and 98 in the amino acid sequence are serine, lysine, leucine, and glutamic acid,
respectively; and
a heavy chain variable region having
the amino acid sequence of SEQ ID NO: 41 or 46, or
an amino acid sequence having 95% or more identity to the amino acid sequence of SEQ ID NO: 41 or 46, wherein the amino acids at positions 33, 35, 52, 56, 62, 102, 103, 104, 109, and 113 in the amino acid sequence are alanine, tyrosine, arginine, asparagine, tyrosine, arginine, phenylalanine, tyrosine, glycine, and aspartic acid, respectively.

More preferably, the humanized monoclonal antibody of the present invention comprises:
a light chain variable region having
the amino acid sequence of SEQ ID NO: 40 or 42, or
an amino acid sequence having 95% or more identity to the amino acid sequence of SEQ ID NO: 40 or 42, wherein the amino acids at positions 26, 27, 30 and 98 in the amino acid sequence are serine, lysine, leucine, and glutamic acid, respectively; and a heavy chain variable region having
the amino acid sequence of SEQ ID NO: 41 or 46, or
an amino acid sequence having 95% or more identity to the amino acid sequence of SEQ ID NO: 41 or 46, wherein the amino acids at positions 33, 35, 52, 56, 62, 102, 103, 104, 109, and 113 in the amino acid sequence are alanine, tyrosine, arginine, asparagine, tyrosine, arginine, phenylalanine, tyrosine, glycine, and aspartic acid, respectively.

Further preferably, the humanized monoclonal antibody of the present invention comprises:
a light chain variable region having
the amino acid sequence of SEQ ID NO: 42, or
an amino acid sequence having 95% or more identity to the amino acid sequence of SEQ ID NO: 42, wherein the amino acids at positions 26, 27, 30 and 98 in the amino acid sequence are serine, lysine, leucine, and glutamic acid, respectively; and a heavy chain variable region having
the amino acid sequence of SEQ ID NO: 41 or 46, or
an amino acid sequence having 95% or more identity to the amino acid sequence of SEQ ID NO: 41 or 46, wherein the amino acids at positions 33, 35, 52, 56, 62, 102, 103, 104, 109, and 113 in the amino acid sequence are alanine, tyrosine, arginine, asparagine, tyrosine, arginine, phenylalanine, tyrosine, glycine, and aspartic acid, respectively.

The humanized monoclonal antibody of the present invention also preferably comprises:
a light chain variable region having
the amino acid sequence of SEQ ID NO: 54, 55, or, 56, or
an amino acid sequence having 95% or more identity to the amino acid sequence of SEQ ID NO: 54, 55, or, 56; and
a heavy chain variable region having
the amino acid sequence of SEQ ID NO: 57 or 58, or
an amino acid sequence having 95% or more identity to the amino acid sequence of SEQ ID NO: 57 or 58.

More preferably, the humanized monoclonal antibody of the present invention has:
1) a light chain variable region having the amino acid sequence of SEQ ID NO: 54, and
   a heavy chain variable region having the amino acid sequence of SEQ ID NO: 57;
2) a light chain variable region having the amino acid sequence of SEQ ID NO: 55, and
   a heavy chain variable region having the amino acid sequence of SEQ ID NO: 57;
3) a light chain variable region having the amino acid sequence of SEQ ID NO: 56, and
   a heavy chain variable region having the amino acid sequence of SEQ ID NO: 57;
4) a light chain variable region having the amino acid sequence of SEQ ID NO: 54, and
   a heavy chain variable region having the amino acid sequence of SEQ ID NO: 58;
5) a light chain variable region having the amino acid sequence of SEQ ID NO: 55, and
   a heavy chain variable region having the amino acid sequence of SEQ ID NO: 58;
6) a light chain variable region having the amino acid sequence of SEQ ID NO: 56, and
   a heavy chain variable region having the amino acid sequence of SEQ ID NO: 58, wherein one or more of the following substitutions are optionally present:
   A) substitution of serine with threonine at position 25 in the amino acid sequence of SEQ ID NO: 54, 55 or 56;
   B) substitution of serine with threonine at position 26 in the amino acid sequence of SEQ ID NO: 54, 55 or 56;
   C) substitution of serine with threonine at position 28 in the amino acid sequence of SEQ ID NO: 54, 55 or 56;
   D) substitution of glutamine with asparagine at position 95 in the amino acid sequence of SEQ ID NO: 54, 55 or 56;
   E) substitution of threonine with serine at position 31 in the amino acid sequence of SEQ ID NO: 57 or 58;
   F) substitution of alanine with valine at position 63 in the amino acid sequence of SEQ ID NO: 57 or 58;
   G) substitution of lysine with arginine at position 67 in the amino acid sequence of SEQ ID NO: 57 or 58;
   H) substitution of aspartic acid with glutamic acid at position 68 in the amino acid sequence of SEQ ID NO: 57 or 58;
   I) substitution of asparagine with glutamine at position 99 in the amino acid sequence of SEQ ID NO: 57 or 58; and
   J) substitution of tyrosine with phenylalanine at position 105 in the amino acid sequence of SEQ ID NO: 57 or 58.

Further preferably, the humanized monoclonal antibody of the present invention has:
a light chain variable region having the amino acid sequence of SEQ ID NO: 56, and a heavy chain variable region having the amino acid sequence of SEQ ID NO: 57, wherein one or more of the following substitutions are optionally present:
A) substitution of serine with threonine at position 25 in the amino acid sequence of SEQ ID NO: 56;
B) substitution of serine with threonine at position 26 in the amino acid sequence of SEQ ID NO: 56;
C) substitution of serine with threonine at position 28 in the amino acid sequence of SEQ ID NO: 56;
D) substitution of glutamine with asparagine at position 95 in the amino acid sequence of SEQ ID NO: 56;
E) substitution of threonine with serine at position 31 in the amino acid sequence of SEQ ID NO: 57;
F) substitution of alanine with valine at position 63 in the amino acid sequence of SEQ ID NO: 57;
G) substitution of lysine with arginine at position 67 in the amino acid sequence of SEQ ID NO: 57;
H) substitution of aspartic acid with glutamic acid at position 68 in the amino acid sequence of SEQ ID NO: 57;
I) substitution of asparagine with glutamine at position 99 in the amino acid sequence of SEQ ID NO: 57; and
J) substitution of tyrosine with phenylalanine at position 105 in the amino acid sequence of SEQ ID NO: 57.

It should be noted that the humanized monoclonal antibody of the present invention uses a constant region of a human antibody. Preferred examples of the constant region of a human antibody include Cy, such as Cγ1, Cy2, Cy3, or Cy4, as the heavy chain, and Cκ and Cλ, as the light chain. The C-region of the human antibody may be modified to improve stability of the antibody or its production. The human antibody used in producing the humanized antibody may be any isotype of human antibody, such as IgG, IgM, IgA, IgE or IgD, and, in the present invention, IgG is preferably used, and IgG1 or IgG4 is further preferably used.

In the humanized monoclonal antibodies of the present invention, lysine may or may not be added to the C-terminal end of the heavy chain constant region. Preferably, the humanized monoclonal antibodies of the present invention has a light chain constant region of the amino acid sequence of SEQ ID NO: 52 and a heavy chain constant region of the amino acid sequence of SEQ ID NO: 53, wherein lysine may or may not be added to the C-terminal end of SEQ ID NO: 53.

The humanized monoclonal antibody can be made by general production methods (see, e.g., WO 95/14041, WO 96/02576). Specifically, at first, a DNA sequence encoding a variable region designed to link a CDR of a mouse antibody to a FR of a human antibody is synthesized by PCR method from several oligonucleotides which are made to have moieties overlapping the terminal ends (see, WO 98/13388). The obtained DNA is linked to a DNA encoding a constant region of a human antibody, and then incorporated into an expression vector. Alternatively, a DNA encoding a variable region of an antibody may be incorporated into an expression vector comprising a DNA of a constant region of an antibody. To produce an antibody used in the present invention, the antibody gene is incorporated into an expression vector such that it is expressed under the control of an expression control region, e.g., an enhancer/promoter. The expression vector can then be used to transform a host cell and express the antibody.

Examples of the host cell of the transformant include a vertebrate cell such as a COS cell or a CHO cell, a prokaryotic cell, and a yeast. The transformant can be cultured according to a method well known to those skilled in the art, and the monoclonal antibody of the present invention is produced intracellularly or extracellularly from the transformant. The medium used for the culture can be selected as appropriate depending on the adopted host cell from various types of media commonly used. When the host cell is a COS cell, examples of the medium include a medium such as RPMI-1640 medium or Dulbecco's Modified Eagle Minimum Essential Medium (DMEM) supplemented with serum components such as bovine fetal serum (FBS), as necessary. The culture temperature during culture of the transformant may be any temperature that does not significantly reduce protein synthesis capacity in the cell, and is preferably 32 to 42°C, most preferably 37°C. As necessary, the transformant can be cultured in an atmosphere containing 1 to 10% (v/v) of carbon dioxide.

Fractions comprising the monoclonal antibody of the present invention produced intracellularly or extracellularly from the transformant as described above can be separated and purified by various known separation methods utilizing the physical and chemical properties or the like of the proteins. Specific examples of such methods include usual treatment with a protein precipitant, ultrafiltration, various chromatography such as molecular sieve chromatography (gel filtration), adsorption chromatography, ion exchange chromatography, affinity chromatography, or high-performance liquid chromatography (HPLC), a dialysis method, and a combination of these. With the methods, the monoclonal antibody of the present invention can be readily produced in high yield and high purity.

The monoclonal antibody or an antibody fragment thereof of the present invention may be further modified by various molecules such as polyethylene glycol (PEG), radioactive materials, toxins, and the like. As the method for modifying the antibody, known methods in the art can be used.

Furthermore, other proteins may be fused to the N- or C-terminal end of the monoclonal antibody of the present invention (Clinical Cancer Research, 2004, 10, 1274-1281). The protein to be fused can be appropriately selected by those skilled in the art.

The monoclonal antibody of the present invention includes an antibody having an N-glycoside-linked glycochain attached to the Fc region of the antibody. Note that fucose may not be attached to N-acetylglucosamine at the reducing end of the N-glycoside-linked glycochain. Examples of the antibody in which an N-glycoside-linked glycochain attached to the Fc region of the antibody and no fucose is attached to N-acetylglucosamine at the reducing end of the N-glycoside-linked glycochain include an antibody produced using a CHO cell lacking an α1,6-fucose transferase gene (WO 2005/035586, WO 02/31140). The antibody of the present invention in which an N-glycoside-linked glycochain attached to the Fc region of the antibody and no fucose is attached to N-acetylglucosamine at the reducing end of the N-glycoside-linked glycochain has high ADCC activity.

It is preferable, in terms of removing Treg cells or macrophage cells, that the monoclonal antibody or an antibody fragment thereof of the present invention has antibody-dependent cell mediated cytotoxicity (ADCC) activity against cells expressing CCR8. ADCC activity means activity in vivo in which an antibody bound to a surface antigen of a cell, such as a target cell, activates an effector cell via a binding of the Fc region of the antibody to an Fc receptor present on the surface of the effector cell, and injures the target cell or the like. Examples of the effector cell include a natural killer cell and an activated macrophage. In the present invention, ADCC activity means activity in vivo in which an antibody bound to a surface antigen (CCR8) of a cell such as a Treg cell or macrophage cell, activates an effector cell via a binding of the Fc region of the antibody to an Fc receptor present on the surface of the effector cell, injures the Treg cell or macrophage cell or the like, and consequently injures a tumor cell or the like.

It is preferred that the monoclonal antibody or an antibody fragment thereof of the present invention is a neutralizing antibody or a neutralizing antibody fragment of CCR8. The neutralizing antibody or neutralizing antibody fragment of CCR8 means an antibody or antibody fragment having neutralizing activity against CCR8. Whether or not an antibody has neutralizing activity against CCR8 can be determined, for example, by measuring whether or not the antibody inhibits the physiological action of a CCR8 ligand (e.g., CCL1) against CCR8. Examples thereof include, but are not limited to, measuring the binding of CCL1 to CCR8, the migration or intracellular Ca²⁺ increase of CCR8-expressing cells by CCL1, or the expression variation of genes susceptible to CCL1 stimulation. It can also be measured by the method described in Examples below.

The neutralizing activity of the monoclonal antibody or an antibody fragment thereof of the present invention for binding of CCR8 to CCL1 can be measured by addition of an antibody dilution diluted with a medium to human CCR8 expressing 293 cells in which a Ca²⁺ indicator is incorporated beforehand. The affinity of CCR8 to a CCR8 ligand is highest when the CCR8 ligand is CCL1, thus antibodies having high inhibitory activity against CCL1 are useful.

It is preferred that the neutralizing activity of the monoclonal antibody or an antibody fragment thereof of the present invention is an IC50 value of 10 nM or less. More preferably, the neutralizing activity is IC50 value of 5 nM or less, even more preferably 2 nM or less, especially preferably 1 nM or less, most preferably 0.5 nM or less.

Among the anti-CCR8 antibodies or antibody fragments thereof that recognize human CCR8, an antibody or an antibody fragment thereof that strongly recognizes human CCR8 is preferred. When selecting the antibody or an antibody fragment thereof that strongly recognizes human CCR8, an antibody or an antibody fragment thereof that more strongly recognizes human CCR8 can be selected by selecting the antibody or an antibody fragment thereof using the strength of neutralizing activity as index.

The anti-CCR8 antibody or an antibody fragment thereof that recognizes tyrosine at position 17 in the amino acid sequence of human CCR8 inhibits a binding of CCR8 to CCL1, and is useful as an anti-CCR8 antibody or an antibody fragment thereof having neutralizing activity. The antibody or an antibody fragment thereof having higher neutralizing activity is preferred in terms of selecting the antibody or an antibody fragment thereof that more strongly recognizes human CCR8. For example, the following antibodies or antibody fragments thereof are preferred.
An antibody or an antibody fragment thereof that recognizes, in addition to tyrosine at position 17 in the amino acid sequence of human CCR8, one or more of amino acids at positions 94 to 107 in the amino acid sequence which correspond to loop1 of human CCR8 and/or one or more of amino acids at positions 172 to 202 in the amino acid sequence which correspond to loop2 of human CCR8 is more preferred.

An anti-CCR8 antibody or an antibody fragment thereof that recognizes, in addition to tyrosine at position 17 in the amino acid sequence of human CCR8, one or more amino acids of isoleucine at position 20, serine at position 22, lysine at position 35, leucine at position 181, cysteine at position 183, and asparagine at position 188 in the amino acid sequence of human CCR8 is more preferred.

As the antibodies that recognize human CCR8, the following antibodies are known.
a) an anti-CCR8 antibody represented by 414B, 414C, 414E, 433H, 459M, 464A, 464B, 433B and 455AL, as described in WO 2007/044756.
b) an anti-CCR8 antibody represented by 3B10, 2D10, and 5B11, as described in the article by Qu et al. (J. Exp. Med., (2004), 200(10), 1231-1241).
c) an anti-CCR8 antibody of product number L263G8 by BioLegend.
d) an anti-CCR8 antibody of product number 191704 by R&D Systems, Inc.

For the anti-CCR8 antibody in a), it is disclosed in WO 2007/044756 that the antibody binds positions 1 to 39 in the amino acid sequence of human CCR8 in an epitope analysis. However, detailed epitope sites have not been analyzed or disclosed at all.

For the anti-CCR8 antibodies in b) to d), epitope sites are not disclosed at all. In other words, no documents disclose or suggest that a monoclonal antibody or an antibody fragment thereof that recognizes tyrosine at position 17 in the amino acid sequence of human CCR8 can be used to inhibit binding of CCR8 to a CCR8 ligand (e.g., CCL1).

The monoclonal antibody or an antibody fragment thereof of the present invention preferably has an tumor-infiltrating Treg cells removal action. Whether or not the monoclonal antibody of the present invention has an tumor-infiltrating Treg cells removal action can be measured, for example, by the method described in Examples of Patent Document 2.

The monoclonal antibody or an antibody fragment thereof of the present invention preferably has an intratumoral infiltrating macrophage cell removal action. Whether or not the antibody or an antibody fragment thereof of the present invention has an intratumoral infiltrating macrophage cell removal action can be measured, for example, by the method described in Examples of Patent Document 2.

The monoclonal antibody or an antibody fragment thereof of the present invention is useful for a pharmaceutical composition. In particular, the monoclonal antibody or an antibody fragment thereof that recognizes tyrosine at position 17 in the amino acid sequence of human CCR8 is very useful for a pharmaceutical composition. Thus, a pharmaceutical composition comprising the monoclonal antibody or an antibody fragment thereof of the present invention can be administered systemically or topically, orally or parenterally. Examples of the parenteral administration include intravenous injection, such as infusion, intramuscular injection, intraperitoneal injection, subcutaneous injection, intranasal administration, and inhalation.

The pharmaceutical composition of the present invention is very useful as a medicament for the treatment and/or prevention of a CCR8-related disease. In particular, it is very useful as a medicament for treating and/or preventing cancer in which intratumoral infiltration of CCR8-expressing Treg cells has occurred. For example, the pharmaceutical composition of the present invention is very useful as a medicament for treating and/or preventing cancer such as breast cancer, uterine corpus cancer, cervical cancer, ovary cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colorectal cancer, kidney cancer, non-Hodgkin lymphoma, urothelial cancer, sarcoma, blood cell carcinoma (leukemia, lymphoma, etc.), bile duct carcinoma, gallbladder carcinoma, thyroid carcinoma, testicular cancer, thymic carcinoma, hepatocarcinoma, and the like, preferably breast cancer, uterine corpus cancer, ovary cancer, lung cancer, colorectal cancer, kidney cancer and sarcoma, more preferably breast cancer, colorectal cancer, kidney cancer and sarcoma.

The "cancer" in the "pharmaceutical composition for treating cancer" of the present invention includes all solid cancers and hematological cancers. Specific examples of the cancer include breast cancer, uterine corpus cancer, cervical cancer, ovary cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colorectal cancer, kidney cancer, non-Hodgkin lymphoma, urothelial cancer, sarcoma, blood cell carcinoma (leukemia, lymphoma, etc.), bile duct carcinoma, gallbladder carcinoma, thyroid carcinoma, testicular cancer, thymic carcinoma, hepatocarcinoma. Preferred examples thereof include breast cancer, uterine corpus cancer, ovary cancer, lung cancer, colorectal cancer, kidney cancer and sarcoma, and more preferred examples thereof include breast cancer, lung cancer, colorectal cancer, kidney cancer and sarcoma.

Furthermore, the "cancer" in the "pharmaceutical composition for treating cancer" of the present invention is preferably a cancer expressing a tumor-specific antigen.

Note that the "cancer" as described in the present description shall mean not only epithelial malignancies such as ovarian cancer, gastric cancer, and the like, but also non-epithelial malignancies including hematopoietic cancer such as chronic lymphocytic leukemia and Hodgkin lymphoma. Furthermore, in the present description, the terms such as "cancer," "carcinoma," "tumor," and "neoplasm" are not distinguishable from one another and used interchangeably.

The monoclonal antibody or an antibody fragment thereof of the present invention may be used in combination with other agents and administered as a concomitant agent, for
(1) supplementing and/or enhancing the therapeutic effect of the pharmaceutical composition of the present invention,
(2) improving in kinetics or absorption or reducing in dosage of the pharmaceutical composition of the present invention, and/or
(3) reducing the side effects of the pharmaceutical composition of the present invention.

The concomitant agent of the monoclonal antibody or an antibody fragment thereof of the present invention in combination with other agents may be administered in the form of a combination drug containing both components in one formulation, or may be administered in separate formulations. When administered in separate formulations, they can be co-administered or administered with time difference. The administration with time difference may be performed by administering the monoclonal antibody or an antibody fragment thereof of the present invention, then the other agent, or by administering the other agent, then the monoclonal antibody or an antibody fragment thereof of the present invention, in which each administration method may be the same or different.

Examples of the other agents that may be used in combination with the monoclonal antibody or an antibody fragment thereof of the present invention include an anti-PD-1 antibody, an anti-PD-L1 antibody, and an anti-CTLA-4 antibody. The anti-PD-1 antibody and the anti-PD-L1 antibody are preferred, and the anti-PD-1 antibody is more preferred.

Examples of the anti-PD-1 antibody in the present invention include Nivolumab and Pembrolizumab.

Examples of the anti-PD-L1 antibody in the present invention include Atezolizumab, Avelumab, and Durvalumab.

Examples of the anti-CTLA-4 antibody in the present invention include Ipilimumab.

It is contemplated that a patient to be subjected to the pharmaceutical composition of the present invention is or is suspected of being a cancer patient. The effective dosage of the pharmaceutical composition according to the present invention is selected from the range of 0.01 mg to 100 mg per kg of body weight per dose. Alternatively, it can be selected from a dosage of 5 to 5000 mg, preferably 10 to 500 mg, per patient. However, the dosage of the pharmaceutical composition comprising the monoclonal antibody or an antibody fragment thereof of the present invention are not limited to these dosages. The administration period can also be appropriately selected depending on the age or symptoms of the patient. The pharmaceutical composition of the present invention may further comprise a pharmaceutically acceptable carrier or additive, depending on the route of administration. Examples of the carrier and additive include water, a pharmaceutically acceptable organic solvent, collagen, polyvinyl alcohol, polyvinylpyrrolidone, sodium alginate, watersoluble dextran, pectin, methylcellulose, ethylcellulose, casein, diglycerin, propylene glycol, polyethylene glycol, petrolatum, human serum albumin (HSA), mannitol, sorbitol, lactose, and a surfactant acceptable as a pharmaceutical additive. The additives used are selected as appropriate or in combination from the above, depending on the dosage form, but are not limited thereto.

The present invention encompasses a polynucleotide encoding a light chain variable region or a heavy chain variable region of the monoclonal antibody of the present invention. The present invention further encompasses an expression vector comprising the polynucleotide.

The polynucleotide is not particularly limited as long as it encodes a light chain variable region or a heavy chain variable region of the monoclonal antibody of the present invention, and it is a polymer consisting of nucleotides such as a plurality of deoxyribonucleic acids (DNA) or ribonucleic acids (RNA). It may contain a non-natural nucleotide. The polynucleotide of the present invention can be used for producing antibodies by genetically engineered methods. The polynucleotide of the present invention can also be used as a probe for screening an antibody having the same function as the monoclonal antibody of the present invention. That is, a polynucleotide encoding the monoclonal antibody of the present invention or a portion thereof can be used as a probe in techniques such as hybridization, gene amplification techniques (e.g., PCR) or the like to obtain a DNA that hybridizes with the polynucleotide under stringent conditions and encodes an antibody having equivalent activity to the monoclonal antibody of the present invention. Such DNA is also included in the polynucleotide of the present invention.

Hybridization techniques (Sambrook, J et al., Molecular Cloning 2nd ed., 9.47-9.58, Cold Spring Harbor Lab. press, 1989) are techniques well known to those skilled in the art. Examples of conditions for hybridization include low stringent conditions. A low stringent condition is, for example, a condition of 42°C, 0.1 x SSC, 0.1% SDS, preferably a condition of 50°C, 0.1 x SSC, 0.1% SDS in washing after hybridization. Examples of more preferred hybridization conditions include high stringent conditions. A high stringent condition is, for example, a condition of 65°C, 5 x SSC and 0.1% SDS. In these conditions, it can be expected that polynucleotides having high homology are efficiently obtained as the temperature increases. However, multiple factors such as temperature and salt concentration are considered to affect the stringency of hybridization. Those skilled in the art can achieve the same stringency by selecting these factors as appropriate.

Antibodies that are functionally equivalent to the monoclonal antibody of the present invention, encoded by the polynucleotides obtained by these hybridization and gene amplification techniques, typically have high homology with the antibody in amino acid sequence. The monoclonal antibody of the present invention also includes an antibody that is functionally equivalent to the monoclonal antibody of the present invention and has high homology with the antibody in amino acid sequence. High homology usually refers to at least 75% or more identity, preferably 85% or more identity, further preferably 95% or more identity in amino acid level. The homology of a polypeptide can be determined according to the algorithm described in the document (Wilbur, W. J. and Lipman, D. J. Proc. Natl. Acad. Sci. USA (1983) 80, 726-730).

The monoclonal antibody or an antibody fragment thereof of the present invention specifically binds to CCR8 and can thus be used for detecting CCR8 in a biological sample. Examples of the biological sample include blood, plasma, serum, urine, an organ, a tissue, bone marrow, and a lymph node. Accordingly, a kit comprising the monoclonal antibody of the present invention is available as a kit for detecting CCR8. The kit comprises the monoclonal antibody or an antibody fragment thereof of the present invention, and may further comprise a secondary antibody for labelling, a substrate necessary for detection of the labelling, a carrier, a wash buffer, a sample dilution, an enzyme substrate, a reaction stop solution, a CCR8 protein as a purified standard substance, an instruction for use, and the like.

The present invention also includes a monoclonal antibody or an antibody fragment thereof that binds to CCR8, which recognizes a site on the antigen recognized by the monoclonal antibody or an antibody fragment thereof of the present invention. That is, monoclonal antibodies or antibody fragments thereof competing with the monoclonal antibody or an antibody fragment thereof of the present invention for binding to CCR8 are also included in the present invention. Such antibodies can be selected, for example, by performing competitive experiments with antibodies such as clone No. 10A11 in Example 3 or antibodies having the CDR sequences described in the present description in light and long chain variable regions.

### [EXAMPLES]

Hereinafter, the present invention will be explained in more detail by way of Examples of the present invention, but the present invention is not limited by them.

### Example 1: Production of anti-human CCR8 antibody-producing mouse hybridoma

The gene encoding the full length of human CCR8 (UniProtKB/Swiss-Prot: P51685) was used as an antigen and DNA-immunized to A/J Jms Slc female mice. DNA immunization was repeated two or three times at two-week intervals and boosted by intraperitoneally administering human CCR8-expressing Expi293 cells one week after final immunization. After three days, spleen was removed, and splenocytes and mouse myeloma cells (p3x6363-Ag8., Tokyo Oncology Institute) were fused by the PEG method, and selection was made in a medium containing hypoxanthine, aminopterin and thymidine. With the obtained culture supernatant, anti-human CCR8 antibodies and anti-human CCR8 neutralizing antibodies were selected by the following method.

For anti-human CCR8 antibodies, the culture supernatant was reacted with human CCR8-expressing Expi293 cells and human CCR4-expressing Expi293 cells, respectively, and clones that specifically bind only to human CCR8 were selected by detecting with Alexa488-labeled anti-mouse IgG antibodies (manufactured by Thermo Fisher Scientific).

For anti-human CCR8 neutralizing antibodies, the culture supernatant was sufficiently reacted with human CCR8 expressing 293 cells in which a Ca²⁺ indicator was incorporated beforehand, then Ca²⁺ influx by addition of 200 nM hCCL1 (manufactured by BioLegend) was measured with FLIPR, and clones that inhibit Ca²⁺ influx by hCCL1 stimulation were selected.

Clones that exhibited particularly strong neutralizing activity (% inhibition > 80%) and clones that had no neutralizing activity were respectively cloned to establish hybridomas.

### Example 2: Production of anti-human CCR8 antibody-producing rat hybridoma

The immunogen, a human CCR8-expressing Rat-1 cell, was produced by transfecting Rat-1 cells with an expression vector in which the human CCR8 gene was cloned into pQCXIP (Clontech Laboratories, Inc.), then subjecting the cells to drug selection with puromycin (1 ug/ml) for one month.

The human CCR8-expressing Rat-1 cells were used to immunize rats once. About two weeks later, the lymph nodes were collected and hybridomas were produced by a routine method.

For anti-human CCR8 antibodies, the culture supernatant of the hybridomas was reacted with human CCR8-expressing 293 cells and 293 cells, respectively, and clones that are capable of specifically binding to human CCR8 were selected by detecting with Alexa647-labeled anti-rat IgG antibodies (manufactured by Thermo Fisher Scientific).

### Example 3: Epitope analysis of anti-human CCR8 neutralizing and non-neutralizing antibodies

The culture supernatants of the anti-human CCR8 antibody producing hybridoma established in Example 1 cultured in serum-free medium was subjected to Protein G purification and gel filtration purification to obtain purified antibodies of 40 clones. Of the obtained 40 clones, purified antibodies of 27 neutralizing antibodies and 13 non-neutralizing antibodies were used to perform an antigen-binding test by the method described below to identify epitopes important for neutralizing activity. For the neutralizing activity of each clone, inhibitory activity against Ca²⁺ influx by 100 nM hCCL1 (manufactured by BioLegend) addition was measured with FLIPR by the method described in Example 1. Each neutralizing antibody had an IC50 value of 2 nM or less of Ca²⁺ influx inhibitory activity by 100 nM hCCL1 stimulation.

The gene encoding the full length of human CCR8 (UniProtKB/Swiss-Prot: P51685, SEQ ID NO: 1), the gene encoding the full length of human CCR4 (UniProtKB/Swiss-Prot: P51679, SEQ ID NO: 48) and the gene encoding the full length of mouse CCR8 (UniProtKB/Swiss-Prot: P56484, SEQ ID NO: 39) were cloned into pcDNA 3.4 vectors, respectively. These vectors were used for transfection to produce Expi293 cells transiently expressing human CCR8, human CCR4, or mouse CCR8. These cells were reacted with a dilution series of the purified antibodies of each clone, and the binding properties thereof were evaluated by detection with Alexa488-labeled anti-mouse IgG antibody (manufactured by Thermo Fisher Scientific). As a result, every antibody bound to human CCR8, but not to human CCR4 and mouse CCR8 at all.

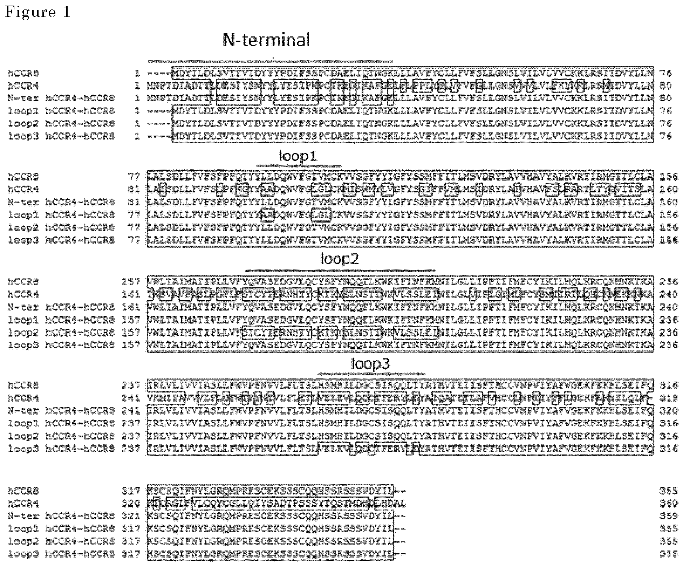

Then, chimeras (Figure 1) in which the extracellular domains of human CCR8, the N-terminal region (amino acids 1-35 of SEQ ID NO: 1), the loop1 region (amino acids 94-107 of SEQ ID NO: 1), the loop2 region (amino acids 172-202 of SEQ ID NO: 1) and the loop3 region (amino acids 264-280 of SEQ ID NO: 1) are substituted with the corresponding regions of human CCR4, the N-terminal region (amino acids 1-39 of SEQ ID NO: 48), the loop1 region (amino acids 98-111 of SEQ ID NO: 48), the loop2 region (amino acids 176-206 of SEQ ID NO: 48) and the loop3 region (amino acids 268-284 of SEQ ID NO: 48), respectively, were produced, and binding of each antibody was evaluated by a method similar to that described above.

Binding at 5 ug/mL, 0.5 ug/mL, and 0.05 ug/mL was evaluated, and, in comparison to wild-type human CCR8 (hCCR8), an antibody having significantly reduced binding activity was indicated as Δ and an antibody having binding activity of the detection limit or less was indicated as ×. The blank column means that it exhibits the same binding activity as the wild-type human CCR8 (hCCR8).

As a result, as shown in Table 2, all antibodies having neutralizing activity had the binding activity of the detection limit or less by substituting the N-terminal region with human CCR4 (N-ter hCCR4-hCCR8), thus it was revealed that the N-terminal region is an important epitope for exerting neutralizing activity. In addition, the binding activities to loop 1-human CCR4-substituted human CCR8 and loop2-human CCR4-substituted human CCR8 were also reduced. From the above, it is considered that anti-CCR8 antibodies having strong neutralizing activity have stereo-structural recognition to strongly recognize the N-terminal region and bind to loop1 and loop2.

Note that although an antibody that does not recognize any of human CCR8, human CCR4, and mouse CCR8 was used as a control, the control antibody did not bind to each antigen. It was also confirmed that expression of hCCR8 has no reduction due to mutation by attaching a tag to the N-terminal of each antigen and detecting it with an anti-tag antibody.

Furthermore, to analyze in more detail the N-terminal region that is an epitope region common to all clones having strong neutralization, the human CCR8 point mutants shown in Table 2 were produced. As a result of evaluating the binding activity of each clone to the mutants by a similar method to the method described above, all 27 neutralizing antibodies described above had significantly reduction to the detection limit or less in their binding activities to the mutant in which Y at position 17 of human CCR8 is substituted for A (hCCR8 (Y17A)). It is thus considered that the neutralizing antibodies recognize Y at position 17. In addition, although clone No. 8F7 is only shown in Table 2, plural other neutralizing antibodies had reduced binding activity to the mutant in which I at position 20 of human CCR8 is substituted with A (hCCR8 (I20A)). It is thus considered that some neutralizing antibodies have stereo-structural recognition to bind to isoleucine at position 20.

Meanwhile, 13 non-neutralizing antibodies described above did not reduce their binding activities to hCCR8 (Y17A), thus it is considered that they recognize the same N-terminal region, but not Y at position 17. From the above, it is considered that Y at position 17 is an amino acid that is extremely important for exerting neutralizing activity. The results for representative neutralizing antibodies (clone Nos. 10A11, 27G1, 1H4, 8F7, 2C7) and non-neutralizing antibodies (clone No. 5B5) are shown in Table 2.

**[Table 2]**

| clone No. | 10A11 | 27G1 | 1H4 | 8F7 | 2C7 | 5B5* |
|---|---|---|---|---|---|---|
| Neutralizing Activity IC50 (nM) | 0.08 | 0.09 | 0.32 | 1.56 | 0.40 | >1000 |
| hCCR8 | | | | | | |
| hCCR4 | × | × | × | × | × | × |
| mCCR8 | × | × | × | × | × | × |
| N-ter hCCR4-hCCR8 | × | × | × | × | × | Δ |
| loop1 hCCR4-hCCR8 | Δ | Δ | Δ | × | × | |
| loop2 hCCR4-hCCR8 | Δ | Δ | Δ | Δ | Δ | |
| loop3 hCCR4-hCCR8 | | | | | | |
| hCCR8 (L5A) | | | Δ | | Δ | |
| hCCR8 (D6A) | | | | | | |
| hCCR8 (L7A) | | | | | | |
| hCCR8 (S8A) | | | | | | |
| hCCR8 (T10A) | | | | | Δ | |
| hCCR8 (T11A) | | | Δ | | | |
| hCCR8 (V12A) | | | | | | |
| hCCR8 (T13A) | | | | | | |
| hCCR8 (Y15A) | | | | | | |
| hCCR8 (Y16A) | | | | | | |
| hCCR8 (Y17A) | × | × | × | × | × | |
| hCCR8 (120A) | | | | Δ | | |
| hCCR8 (S22A) | | | | | Δ | |
| hCCR8 (S23A) | | | | | | |
| hCCR8 (L29A) | | | | | | |
| hCCR8 (I30A) | | | | Δ | | |
| hCCR8 (Q31A) | | | | | | |
| hCCR8 (T32A) | | | | | | |
| hCCR8 (N33A) | | | | | | |
| hCCR8 (G34A) | | | | | | |
| hCCR8 (K35A) | | | × | Δ | | |

| | | | | | | |
|---|---|---|---|---|---|---|
| *: non-neutralizing antibodies | | | | | | |

### Example 4: Determination of antibody sequences

Of the established clones, for the mouse antibody shown in Table 3 and the rat antibody shown in Table 4, the amino acid sequences of the light and heavy chain variable regions of the antibodies were determined with the hybridoma cells according to a routine method.

**[Table 3]**

| mAb | | SEQ ID NO: | | SEQ ID NO: | | SEQ ID NO: | | SEQ ID NO: |
|---|---|---|---|---|---|---|---|---|
| 10A11 | Light chain | 26 | CDR1 | 2 | Heavy chain | 27 | CDR1 | 5 |
| | | | CDR2 | 3 | | | CDR2 | 6 |
| | | | CDR3 | 4 | | | CDR3 | 7 |
| 27G1 | Light chain | 26 | CDR1 | 2 | Heavy chain | 28 | CDR1 | 8 |
| | | | CDR2 | 3 | | | CDR2 | 6 |
| | | | CDR3 | 4 | | | CDR3 | 7 |
| 1H4 | Light chain | 29 | CDR1 | 2 | Heavy chain | 30 | CDR1 | 8 |
| | | | CDR2 | 3 | | | CDR2 | 6 |
| | | | CDR3 | 4 | | | CDR3 | 7 |
| 19D7 | Light chain | 31 | CDR1 | 2 | Heavy chain | 32 | CDR1 | 8 |
| | | | CDR2 | 9 | | | CDR2 | 6 |
| | | | CDR3 | 10 | | | CDR3 | 11 |
| 8F7 | Light chain | 33 | CDR1 | 2 | Heavy chain | 34 | CDR1 | 12 |
| | | | CDR2 | 3 | | | CDR2 | 6 |
| | | | CDR3 | 10 | | | CDR3 | 13 |
| 2C7 | Light chain | 35 | CDR1 | 14 | Heavy chain | 36 | CDR1 | 17 |
| | | | CDR2 | 15 | | | CDR2 | 18 |
| | | | CDR3 | 16 | | | CDR3 | 19 |

**[Table 4]**

| mAb | | SEQ ID NO: | | SEQ ID NO: | | SEQ ID NO: | | SEQ ID NO: |
|---|---|---|---|---|---|---|---|---|
| 2-7B | Light chain | 37 | CDR1 | 20 | Heavy chain | 38 | CDR1 | 23 |
| | | | CDR2 | 21 | | | CDR2 | 24 |
| | | | CDR3 | 22 | | | CDR3 | 25 |

### Example 5: Alignment of Antibody Sequences

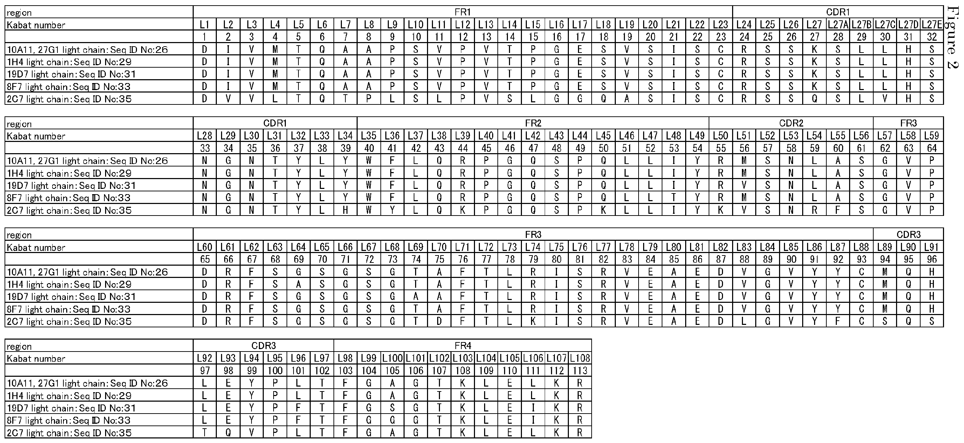

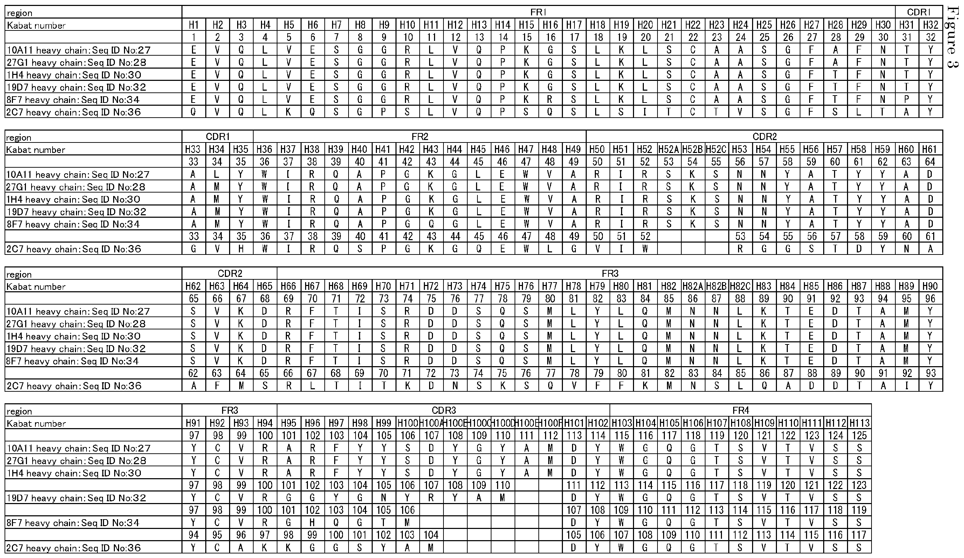

The amino acid sequences of the light and heavy chain of anti-human CCR8 neutralizing antibodies from mouse hybridoma cells described in Example 4 were aligned to Kabat numbering with an antibody sequence analysis software abYsis (Figures 2 and 3). As a result, the amino acid sequences of 10A11, 27G1, 1H4, 19D7, and 8F7 comprised sequences similar to CDRs as described below.
CDR1 of the light chain consisted of 16 amino acids of SEQ ID NO: 2.
CDR2 of the light chain consisted of 7 amino acids of R-Xaa1-S-N-L-A-S (wherein Xaa1 is M or V: SEQ ID NO: 49) (SEQ ID NO: 3 or 9).
CDR3 of the light chain consisted of 9 amino acids of M-Q-H-L-E-Y-P-Xaa1-T (wherein Xaa1 is L or F: SEQ ID NO: 50) (SEQ ID NO: 4 or 10).
CDR1 of the heavy chain consisted of 5 amino acids of Xaal-Y-A-Xaa2-Y (wherein Xaa1 is T or P and Xaa2 is L or M: SEQ ID NO: 51) (SEQ ID NO: 5, 8 or 12).
CDR2 of the heavy chain consisted of 19 amino acids of SEQ ID NO: 6.
CDR3 of the heavy chain, which were common to 10A11, 27G1, 1H4, consisted of 14 amino acids of SEQ ID NO: 7.

### Example 6: Evaluation of Neutralizing Activity

The established hybridomas were cultured in serum-free medium and the culture supernatant was subjected to Protein G affinity purification and gel filtration purification to give a purified antibody. Neutralizing activity of the purified antibody was measured by the method described below.

The antibody dilution diluted with a medium was added to human CCR8 expressing 293 cells in which a Ca²⁺ indicator was incorporated beforehand, and Ca²⁺ influx by addition of 200 nM HCCL1 (manufactured by BioLegend) was measured with FLIPR. The inhibition rate was calculated by setting the signal when HCCL1 was not added as the inhibition rate of 100% and the signal when hCCL1 was added and the antibody was not added as the inhibition rate of 0%, and the antibody concentration showing the inhibition rate of 50% was taken as IC50. The evaluation was performed at least three times, and IC50 was expressed as average ± SD. (Table 5)

**[Table 5]**

| Clone | IC50 (nM) |
|---|---|
| 10A11 | 0.23±0.10 |
| 27G1 | 0.27±0.06 |
| 1H4 | 0.36±0.10 |
| 19D7 | 0.18±0.06 |
| 8F7 | 3.03±0.91 |
| 2C7 | 1.19±0.64 |
| 2-7B | 0.23±0.10 |

### Example 7: Humanization of Antibodies (10A11, 2C7)

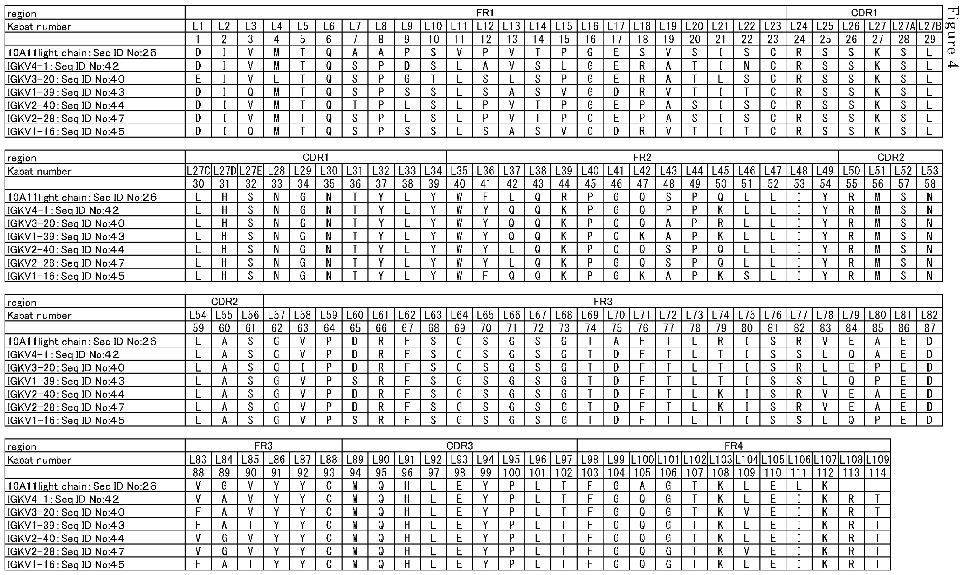

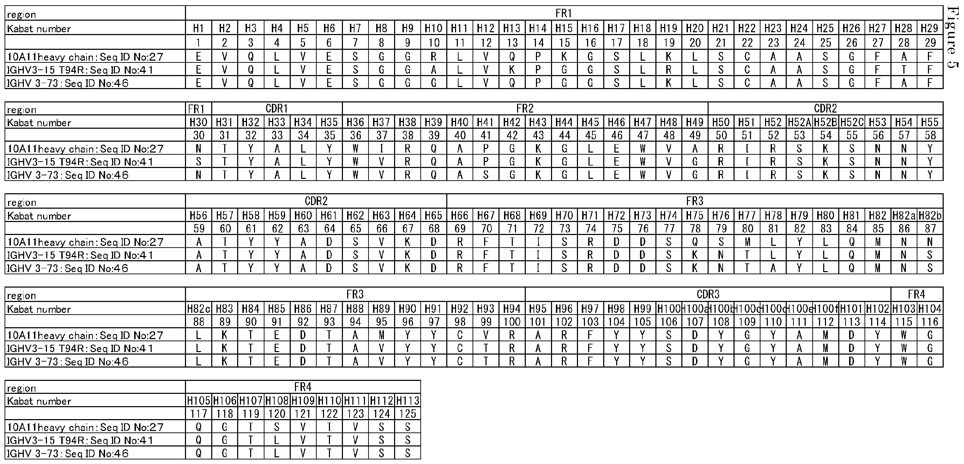

Humanization was performed for 10A11, 2C7 by the methods described below. The definition of Kabat numbering and CDR was made with the antibody sequence analysis software abYsis. Human reproductive system acceptor sequences analogous to the V gene region sequences of the heavy and light chains of the mouse antibody amino acid sequences were searched and selected with the sequence analysis software Absis. For the J-chain region, sequences that are highly homologous to mouse antibody DNA sequences were searched with IMGT (http://www.imgt.org/) and used as the human framework sequences. To this human framework sequences, mouse antibody heavy chains CDR1, CDR2, CDR3 and mouse antibody light chains CDR1, CDR2, CDR3 as defined by Kabat Numbering (Wu, T. T. and Kabat, E.A., J Exp. Med. Aug 1; 132 (2): 211-50. (1970)) were implanted to design a humanized monoclonal antibody sequence (light chain; Figure 4, heavy chain; Figure 5). The neutralizing activity was measured by the method described in Example 6, resulting that the humanized monoclonal antibody shown in Table 6 exhibited affinity equal to or greater than the mouse antibody.

**[Table 6]**

| mAb | Light chain SEQ ID NO: | Heavy chain SEQ ID NO: | IC50 (nM) |
|---|---|---|---|
| h10A11 | 40 | 41 | 0.16±0.08 |
| (IGKV3-20/IGHV3-15 T94R) | | | |
| h10A11 | 42 | 41 | 0.15±0.10 |
| (IGKV4-1/IGHV3-15 T94R) | | | |
| h10A11 | 43 | 41 | 0.20±0.08 |
| (IGKV1-39/IGHV3-15 T94R) | | | |
| h10A11 | 44 | 41 | 0.30±0.12 |
| (IGKV2-40/IGHV3-15 T94R) | | | |
| h10A11 | 45 | 41 | 0.70±0.36 |
| (IGKV1-16/IGHV3-15 T94R) | | | |
| h10A11 | 40 | 46 | 0.22±0.07 |
| (IGKV3-20/IGHV3-73) | | | |
| h10A11 | 42 | 46 | 0.17±0.09 |
| (IGKV 4-1/IGHV3-73) | | | |
| h10A11 | 43 | 46 | 0.16±0.08 |
| (IGKV1-39/IGHV3-73) | | | |
| h10A11 | 44 | 46 | 0.21±0.06 |
| (IGKV2-40/IGHV3-73) | | | |
| h10A11 | 47 | 46 | 0.20±0.05 |
| (IGKV2-28/IGHV3-73) | | | |
| h10A11 | 45 | 46 | 0.31±0.08 |
| (IGKV1-16/IGHV3-73) | | | |
| m10A11 | 26 | 27 | 0.23±0.10 |

### Example 7-2: Humanization of antibody (19D7)

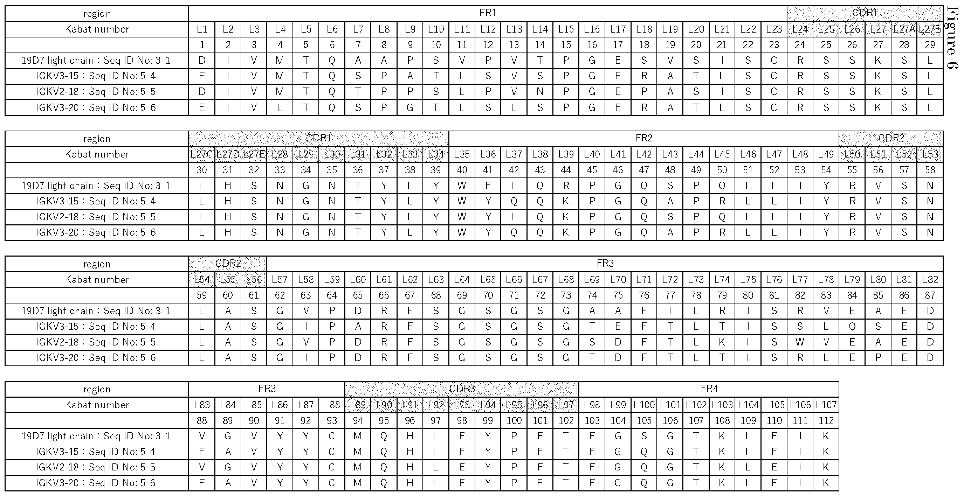

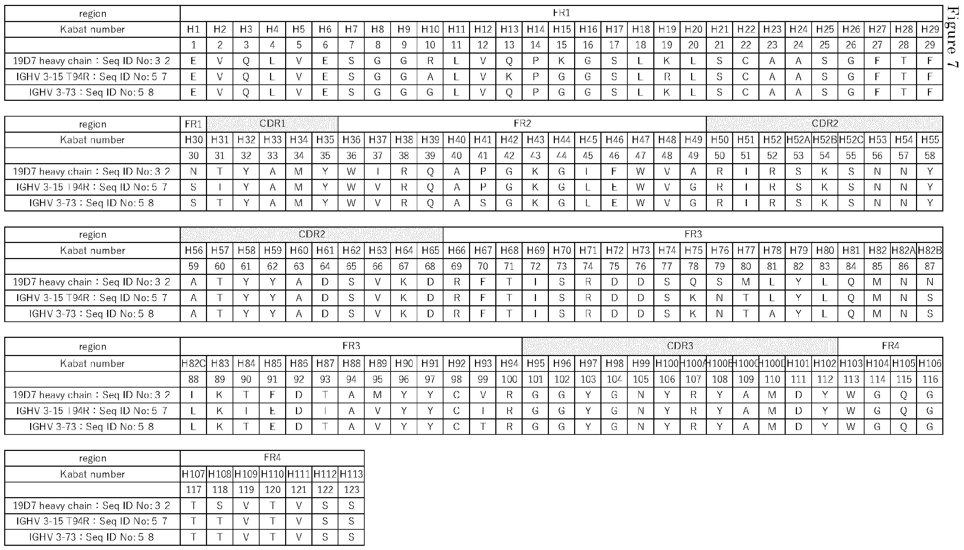

For 19D7, humanization was performed in a similar manner to Example 7 (light chain; Figure 6, heavy chain; Figure 7). The neutralizing activity was measured by the method described in Example 6, resulting that the humanized monoclonal antibody shown in Table 7 exhibited affinity equal to or greater than the mouse antibody.

**[Table 7]**

| mAb | Light chain SEQ ID NO: | Heavy chain SEQ ID NO: | IC50 (nM) |
|---|---|---|---|
| h19D7 | 54 | 57 | 0.18±0.04 |
| (IGKV3-15/IGHV3-15 T94R) | | | |
| h19D7 | 55 | 57 | 0.27±0.05 |
| (IGKV2-18/IGHV3-15 T94R) | | | |
| h19D7 | 56 | 57 | 0.24±0.04 |
| (IGKV3-20/IGHV3-15 T94R) | | | |
| h19D7 | 54 | 58 | 0.27±0.06 |
| (IGKV3-15/IGHV3-73) | | | |
| h19D7 | 55 | 58 | 0.46±0.12 |
| (IGKV2-18/IGHV3-73) | | | |
| h19D7 | 56 | 58 | 0.31±0.04 |
| (IGKV3-20/IGHV3-73) | | | |
| m19D7 | 31 | 32 | 0.18±0.06 |

### Example 8: Identification of amino acids important for activity of humanized 10A11

For humanized 10A11 shown in Figures 4 and 5, mutants in which point mutations were introduced into amino acids corresponding to CDRs were produced, and the neutralizing activity of each mutant was calculated by the method described in Example 6. Evaluation of neutralizing activity was performed multiple times for each mutant, and the IC50 value was expressed by IC50 ratio (WT IC50/mutant IC50) which is the ratio to the IC50 value of WT.

The results are shown in Tables 8 and 9. The n.d. (= not detectable) indicates that the IC50 value of the mutant is 10 nM or more, the limit value of the measurement system, and the activity is reduced to the detection limit or less. As a result of the neutralizing activity evaluation, for light chains, the activities of each mutant of S26T, K27R, L27cI and E93D having mutations at L26, L27, L27c, and L93 amino acid positions by Kabat Numbering, respectively, were reduced by 10-fold or more (Table 8). For heavy chains, the activities of each mutant of A33V, Y35F, R52K, N53Q, Y59F, R96K, F97L, Y98F, G100cA, D101E having mutations at the H33, H35, H52, H53, H59, H96, H97, H98, H100c, H101 amino acid position by Kabat Numbering, respectively, were reduced by 10-fold or more (Table 9). These amino acids are extremely important for the activity.

**[Table 8]**

| Light chain frame | Heavy chain frame | Light chain CDR mutation | IC50 ratio |
|---|---|---|---|
| IGKV4-1 mutant | IGHV3-15 T94R | R24K | 0.16 |
| | | S26T | 0.05 |
| | | K27R | n.d. |
| | | L27cI | 0.05 |
| | | H27dR | 0.18 |
| | | N28Q | 0.79 |
| | | G29A | 0.18 |
| | | N30Q | 0.19 |
| | | T31S | 0.50 |
| | | L33I | 0.70 |
| | | Y34F | 0.25 |
| | | M51L | 0.24 |
| | | N53Q | 2.10 |
| | | L54I | 1.18 |
| | | M89L | 0.49 |
| | | H91R | 0.15 |
| | | L92I | 1.27 |
| | | E93D | n.d. |
| | | Y94F | 0.87 |
| | | P95G | 0.20 |
| | | L96I | 0.56 |
| | | wt | 1.00 |

| | | | |
|---|---|---|---|
| * The amino acid position is the position by Kabat numbering. | | | |

**[Table 9]**

| Light chain frame | Heavy chain frame | Heavy chain CDR mutation | IC50 ratio |
|---|---|---|---|
| IGKV4-1 | IGHV3-73 mutant | T31A | 0.17 |
| | | Y32F | 0.44 |
| | | A33V | n.d. |
| | | L34I | 0.43 |
| | | Y35F | n.d. |
| | | R50K | 0.12 |
| | | I51L | 0.22 |
| | | R52K | n.d. |
| | | S52aT | 0.22 |
| | | K52bR | 0.47 |
| | | S52cT | 0.53 |
| | | N53Q | 0.03 |
| | | Y55F | 0.47 |
| | | A56V | 0.55 |
| | | T57S | 0.49 |
| | | Y58F | 0.50 |
| | | Y59F | 0.03 |
| | | A60V | 0.67 |
| | | D61E | 0.25 |
| | | S62T | 1.60 |
| | | V63L | 0.56 |
| | | K64R | 0.54 |
| | | D65E | 1.30 |
| | | R96K | n.d. |
| | | F97L | n.d. |
| | | Y98F | 0.07 |
| | | Y99F | 0.80 |
| | | S100T | 0.64 |
| | | D100aE | 0.30 |
| | | Y100bF | 0.75 |
| | | G100cA | 0.07 |
| | | Y100dF | 0.55 |
| | | A100eV | 0.49 |
| | | M100fL | 0.72 |
| | | D101E | n.d. |
| | | Y102F | 0.99 |
| | | wt | 1.00 |

| | | | |
|---|---|---|---|
| * The amino acid position is the position by Kabat numbering. | | | |

### Example 8-2: Identification of amino acids important for activity of humanized 19D7

For humanized 19D7 shown in Figures 6 and 7, mutants in which point mutations were introduced into amino acids corresponding to CDRs were produced, and the neutralizing activity of each mutant was calculated by the method described in Example 6. Evaluation of neutralizing activity was performed multiple times for each mutant, and the IC50 value was expressed by IC50 ratio (WT IC50/mutant IC50) which is the ratio to the IC50 value of WT.

The results are shown in Tables 10 and 11. The n.d. (= not detectable) indicates that the IC50 value of the mutant is 10 nM or more, the limit value of the measurement system, and the activity is reduced to the detection limit or less.

**[Table 10]**

| Light chain frame | Heavy chain frame | Light chain CDR mutation | IC50 ratio |
|---|---|---|---|
| IGKV3-20 mutant | IGHV3-15 T94R | R24K | 0.94 |
| | | S25T | 0.63 |
| | | S26T | 0.79 |
| | | K27R | 0.77 |
| | | S27aT | 1.01 |
| | | L27bI | 0.26 |
| | | L27cI | 0.68 |
| | | H27dR | n.d. |
| | | S27eT | 0.58 |
| | | N28Q | 0.02 |
| | | G29A | 0.75 |
| | | N30Q | 0.64 |
| | | T31S | 0.61 |
| | | Y32F | 0.33 |
| | | L33I | 0.20 |
| | | Y34F | 0.78 |
| | | R50K | 0.52 |
| | | V51L | 0.67 |
| | | S52T | 0.60 |
| | | N53Q | 0.49 |
| | | L54I | 0.85 |
| | | A55V | 0.66 |
| | | S56T | 0.69 |
| | | M89L | 0.85 |
| | | Q90N | 1.01 |
| | | H91R | 0.05 |
| | | L92I | 0.43 |
| | | E93D | 0.58 |
| | | Y94F | 0.69 |
| | | P95G | 0.34 |
| | | F96L | 0.10 |
| | | T97S | 0.53 |
| | | N28A | 0.05 |
| | | N28E | n.d. |
| | | N28F | n.d. |
| | | N28G | n.d. |
| | | N28I | n.d. |
| | | N28K | n.d. |
| | | N28L | n.d. |
| | | N28P | n.d. |
| | | N28R | n.d. |
| | | N28S | n.d. |
| | | N28T | n.d. |
| | | N28Y | n.d. |
| | | N28V | 0.14 |

| | | | |
|---|---|---|---|
| * The amino acid position is the position by Kabat numbering. | | | |

**[Table 11]**

| Light chain frame | Heavy chain frame | Heavy chain CDR mutation | IC50 ratio |
|---|---|---|---|
| IGKV3-20 | IGHV3-15 T94R mutant | T31S | 1.22 |
| | | Y32F | 0.77 |
| | | A33V | 0.32 |
| | | M34L | 0.64 |
| | | Y35F | n.d. |
| | | R50K | n.d. |
| | | I51L | 0.79 |
| | | R52K | n.d. |
| | | S52aT | 0.58 |
| | | K52bR | 0.69 |
| | | S52cT | 0.25 |
| | | N53Q | n.d. |
| | | N54Q | 0.48 |
| | | Y55F | 0.98 |
| | | A56V | 0.60 |
| | | T57S | 0.61 |
| | | Y58F | 0.67 |
| | | Y59F | 0.50 |
| | | A60V | 0.74 |
| | | D61E | 0.65 |
| | | S62T | 0.74 |
| | | V63L | 0.73 |
| | | K64R | 1.45 |
| | | D65E | 0.88 |
| | | G95A | 0.53 |
| | | G96A | 0.02 |
| | | Y97F | 0.80 |
| | | G98A | 0.73 |
| | | N99Q | 0.85 |
| | | Y100F | 0.60 |
| | | R100aK | 0.11 |
| | | Y100bF | n.d. |
| | | A100cV | n.d. |
| | | M100dL | 0.60 |
| | | D101E | 0.19 |
| | | Y102F | 0.62 |

| | | | |
|---|---|---|---|
| * The amino acid position is the position by Kabat numbering. | | | |

### Example 9: Enhancement of activity of humanized 10A11

Optimization of the humanized 10A11 was performed by combining the activity-enhancing mutations found in Example 8 and mutations of N at L28 and G at L29 in amino acid position by Kabat numbering, corresponding to the sequence for deamidation risk, with the humanized framework found in Example 7. As a result, the humanized 10A11 mutant shown in Table 12 has been found.

**[Table 12]**

| Light chain variable region | | Heavy chain variable region | IC50 (nM) |
|---|---|---|---|
| Frame | Mutation | Frame | |
| IGKV4-1 | N53Q | IGHV3-15 T94R | 0.12±0.02 |
| IGKV4-1 | N53Q, N28K | IGHV3-15 T94R | 0.29±0.04 |
| IGKV4-1 | N53Q, G29L | IGHV3-15 T94R | 0.18±0.02 |
| IGKV4-1 | N53Q, G29R | IGHV3-15 T94R | 0.12±0.04 |
| IGKV4-1 | G29R | IGHV3-15 T94R | 0.20±0.05 |
| IGKV3-20 | N53Q | IGHV3-15 T94R | 0.18±0.05 |
| IGKV3-20 | N53Q, N28K | IGHV3-15 T94R | 0.29±0.06 |
| IGKV3-20 | N53Q, G29L | IGHV3-15 T94R | 0.21±0.03 |
| IGKV3-20 | N53Q, G29R | IGHV3-15 T94R | 0.19±0.02 |

| | | | |
|---|---|---|---|
| * The amino acid position is the position by Kabat numbering. | | | |

### Example 9-2: Enhancement of activity of humanized 19D7

Optimization of the humanized 19D7 was performed by introducing point mutations into the humanized framework found in Examples 7-2. As a result, the humanized 19D7 mutant shown in Table 13 has been found.

**[Table 13]**

| Light chain variable region | | Heavy chain variable region | | IC50 (nM) |
|---|---|---|---|---|
| Frame | CDR mutation | Frame | CDR mutation | |
| IGKV3-20 | S25T | IGHV3-15 T94R | None | 0.31±0.04 |
| | S26T | | None | 0.20±0.01 |
| | S27aT | | None | 0.22±0.04 |
| | Q90N | | None | 0.30±0.17 |
| | None | | T31S | 0.26±0.04 |
| | None | | A60V | 0.27±0.04 |
| | None | | K64R | 0.24±0.05 |
| | None | | D65E | 0.27±0.04 |
| | None | | N99Q | 0.22±0.04 |
| | None | | Y102F | 0.31±0.06 |

### Example 10: Anti-tumor activity of anti-human CCR8 antibody in tumor-inoculated human CCR8 knock-in mice (hereinafter referred to as hCCR8-KI (KI/KI) mice)

### (1) Production of hCCR8-KI (KI/KI) mice

The mouse CCR8 gene (Gene ID: 12776) and the human CCR8 gene (Gene ID: 1237) both are composed of two exons, and include the full-length ORF (open reading frame) within the second exon. The full-length ORF (CCDS ID: 23621.1) of the mouse CCR8 gene was removed while the full-length ORF (CCDS ID: 2684.1) of the human CCR8 gene was inserted, thereby hCCR8-KI (KI/KI) mice in which the CCR8 protein expressed is fully humanized were produced.

DNA fragments to be used as homologous recombinantion arms were obtained by PCR amplification of mouse genomic sequences about 2 kb (kilobase) upstream and downstream of the ORF of the mouse CCR8 gene, respectively. The homologous recombinantion arms were designed so that only ORF was removed. The homologous recombinantion arms were seamlessly connected to both sides of the full-length sequence of ORF of the human CCR8 gene, respectively, to produce a targeting vector. The targeting vector was subjected to homologous recombination to produce a hCCR8-KI (KI/+) Balb/c mouse. The produced hCCR8-KI (KI/+) Balb/c mouse was confirmed that the full-length ORF of the mouse CCR8 gene was correctly replaced to the full-length ORF of the human CCR8 gene and there were no insertions or in the ORF and recombination arms, by PCR and DNA nucleotide sequence analysis. The hCCR8-KI (KI/+) Balb/c mice were crossed to produce a hCCR8-KI (KI/KI) mouse.

### (2) Confirmation of human CCR8 expression in intratumoral infiltrating cells in CT26 colorectal cancer inoculated in hCCR8-KI(KI/KI) mice

3.5 x 10⁵ (50 µL) CT26 cells were inoculated on back skin of hCCR8-KI (KI/KI) mice (6 weeks old, female) and tumors were recovered from 5 individuals at 17 day of inoculation (N = 5). Tumor masses of CT26 cells were finely cut with scissors, and tumor-infiltrating cells were prepared with commercially available kits (Tumor Dissociation Kit, mouse, Miltenyi Biotec, and The gentleMACS (TM) Dissociator, Miltenyi Biotec) according to the attached kit protocols.

The prepared cells were passed through a 70 um cell strainer and then washed twice with 10 mM HEPES/HBSS/2% FBS. The cells were then treated with a red blood cell lysing solution (BD Biosciences) for 5 minutes to remove red blood cells, and further washed twice with a 2% FBS/10 mM HEPES/HBSS buffer. Tumor-infiltrating cells were stained by the following method with the antibodies described below.

Infiltrating cells were stained in ice with a Zombie NIR Fixable Viability Kit (BioLegend) reagent for 30 minutes. After one wash with 2% FBS/10 mM HEPES/HBSS, the cells were stained with Bv510-labeled anti-mouse CD45 (30-F11, BioLegend), FITC-labeled anti-mouse CD4 (RM4-4, BioLegend), PE/Cy7-labeled anti-mouse CD8 (53-6.7, BioLegend), PerCP/Cy5.5-labeled anti-mouse TCRβ (H57-597, BioLegend), PE-labeled anti-mouse CD25 (PC61, BioLegend), BV421-labeled anti-human CCR8 antibody (433H, BD Biosciences) (or BV421-labeled isotope control antibody). Staining was performed in ice for 30 minutes. After washing twice with 2% FBS/HEPES/HBSS, the cells were analyzed using a flow cytometer.

Human CCR8 expression in CD45+TCRβ+CD4+CD25+ T cells was analyzed. Negative cell regions were determined by staining with isotype-controlled antibodies, and the frequency of positive in intratumoral infiltrating cells in cancer in 17 days after inoculation was calculated by taking cells becoming positive with anti-human CCR8 antibodies as human CCR8+ cells. As a result, human CCR8 was detected in approximately 47% of CD45+TCRβ+CD4+CD25+ cells in mouse tumors.

### (3) Evaluation of anti-tumor effect of anti-human CCR8 antibody administration in CT26 colon cancer model

On back skinof hCCR8-KI (KI/KI) mice (8 weeks old, female), 4 x 10⁵ (50 uL) CT26colon cancer were inoculated. On days 4 and 11 after tumor inoculation, 100 µg (100 µL) or 200 µg (100 µL) of the humanized 10A11 antibody (light chain variable region: IGKV4-1 N53Q + G29R (SEQ ID NO: 59)/heavy chain variable region: IGHV3-15 T94R (SEQ ID NO: 41)) was administered intravenously (N = 10). As a control, 100 µL of vehicle (phosphate buffered saline) (N = 10) was administered. Tumor sizes were measured 4, 7, 10, 11, 14, 16, 18, 21, 24 days after tumor inoculation. The tumor size (mm³) was measured in long diameter (mm) x short diameter (mm) x short diameter (mm)/2.

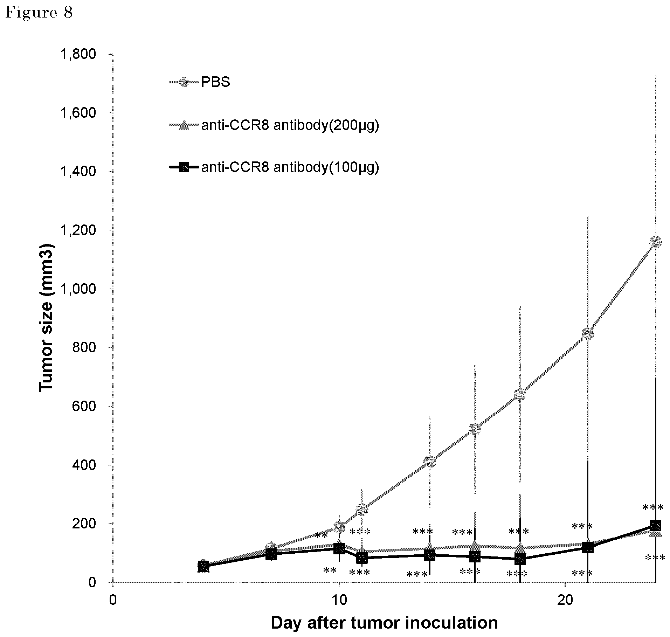

As a result, tumor sizes were significantly smaller in the anti-human CCR8 antibody dosing group at any dose on 11, 14, 16, 18, 21, and 24 days after inoculation (Figure 8, significance levels by Welch's t test were **; p < 0.01 on day 10, and ***; p < 0.001 on day 11 and later, at any dose.). Also in the anti-human CCR8 antibody administration group, individuals indicating complete regression appeared. On day 24, tumors disappeared almost completely in 5 out of 10 mice in the 100 pg anti-human CCR8 antibody administration group, and 6 out of 10 mice in the 200 pg anti-human CCR8 antibody administration group.

### Example 11: Screening of anti-human CCR8 antibodies competing with 10A11

A humanized 10A11 antibody (light chain variable region: IGKV4-1/heavy chain variable region: IGHV3-15 T94R) was used to perform a screening for antibodies competing with the antibody for binding to hCCR8.

In a similar manner to the method described in Example 1, a gene encoding the full length of human CCR8 (UniProtKB/Swiss-Prot: P51685) was used as an antigen and DNA-immunized to A/J Jms Slc female mice to produce a hybridoma. The hybridoma produced was seeded into 96-well plates, and the culture supernatant in 176 wells obtained after 9 days of culture was used to perform a competitive binding test with humanized 10A11 antibodies. The competitive binding test was performed on human CCR8-expressing Expi293 cells produced by the method of Example 1 by mixing the culture supernatant with 10 nM humanized 10A11 antibody or isotype control, allowing to react for 3 hours at room temperature, washing 3 times with PBS, and then detecting with Alexa488-labeled anti-mouse IgG antibody (manufactured by Thermo Fisher Scientific). The antibodies for which the binding of hybridoma-derived antibody was confirmed under the coexistence of isotype control was defined as normal human CCR8-binding antibodies. Among the human CCR8-binding antibodies, an antibody of which the binding signal was significantly reduced under the coexistence of the humanized 10A11 antibody was identified as an antibody competing with the humanized 10A11 antibody. As a result, antibodies of 56 wells in 170 wells were human CCR8-binding antibodies. In the 56 wells, antibodies of 7 wells were antibodies competing with the humanized 10A11 antibody.

Furthermore, of those identified as competing antibodies, three representative clones were cloned, and purified antibodies were obtained from the hybridoma culture supernatants after cloning. For the purified antibodies, the neutralizing activity against human CCL1-human CCR8 was measured by the method of Example 1. As a result, it was shown that all antibodies had neutralizing activity (Table 14) and antibodies competing with the humanized 10A11 antibody were strong neutralizing antibodies.

**[Table 14]**

| Clone | IC50 (nM) |
|---|---|
| 19B10 | 4.5 |
| 6D11 | 3.3 |
| 7F4 | 6.4 |

### Example 12 Epitope analysis of humanized monoclonal antibodies

In a similar manner to Example 3, epitope validation was performed for humanized 10A11 antibody and humanized 19D7 antibody. In addition to the chimeras of human CCR4 and the point mutants at the N-terminal region of human CCR8 produced in Example 3, binding evaluation was also performed for point mutants at loop1 and loop2 regions of human CCR8. The binding evaluation was performed by transiently expressing each mutant in Expi293 cells, and reacting the mutant with an antibody solution of a humanized 10A11 antibody (light chain variable region: IGKV4-1/heavy chain variable region: IGHV3-15 T94R) or a humanized 19D7 antibody (light chain variable region: IGKV3-20/heavy chain variable region: IGHV3-15 T94R) prepared by 8 serially diluting by 3-fold from 20 µg/mL. After reacting at room temperature for 3 hours, it was reacted with Alexa488-labeled Anti-human IgG antibody (Thermo Fisher Scientific) and flow cytometry analysis was performed. The expression amount of each mutant was corrected by tagging the N-terminal of each mutant and detecting with an anti-tag antibody in a similar manner.

The results are shown in Tables 15 and 16. In comparison to human CCR8, a mutant having binding activity of 70% or less was indicated as Δ and a mutant having binding activity of 20% or less was indicated as ×.

**[Table 15]**

| Mutant | h10A11 | h19D7 |
|---|---|---|
| hCCR8 | | |
| hCCR4 | × | × |
| mCCR8 | × | × |
| N-ter hCCR4-hCCR8 | × | × |
| loop1 hCCR4-hCCR8 | Δ | Δ |
| loop2 hCCR4-hCCR8 | Δ | Δ |
| loop3 hCCR4-hCCR8 | | Δ |
| hCCR8 (L5A) | Δ | Δ |
| hCCR8 (D6A) | | |
| hCCR8 (L7A) | | |
| hCCR8 (S8A) | | |
| hCCR8 (T10A) | | Δ |
| hCCR8 (T11A) | | Δ |
| hCCR8 (V12A) | | |
| hCCR8 (T13A) | | Δ |
| hCCR8 (Y15A) | | Δ |
| hCCR8 (Y17A) | × | × |
| hCCR8 (I20A) | Δ | Δ |
| hCCR8 (S22A) | Δ | Δ |
| hCCR8 (S23A) | | Δ |
| hCCR8 (L29A) | | Δ |
| hCCR8 (I30A) | | |
| hCCR8 (Q31A) | | |
| hCCR8 (T32A) | Δ | |
| hCCR8 (N33A) | | |
| hCCR8 (G34A) | | |
| hCCR8 (K35A) | Δ | Δ |
| hCCR8 (Y94A) | | |
| hCCR8 (L95A) | | |
| hCCR8 (L96A) | | |
| hCCR8 (D97A) | Δ | |
| hCCR8 (Q98A) | | |
| hCCR8 (V100A) | | |
| hCCR8 (T103A) | | |

**[Table 16]**

| | | |
|---|---|---|
| hCCR8 (V104A) | | |
| hCCR8 (M105A) | | |
| hCCR8 (K107A) | | |
| hCCR8 (Y172A) | | |
| hCCR8 (Q173A) | | |
| hCCR8 (V174A) | | |
| hCCR8 (A175G) | | |
| hCCR8 (S176A) | | |
| hCCR8 (E177A) | | |
| hCCR8 (D178A) | | |
| hCCR8 (G179A) | | |
| hCCR8 (V180A) | Δ | |
| hCCR8 (L181A) | Δ | Δ |
| hCCR8 (Q182A) | | |
| hCCR8 (C183A) | × | × |
| hCCR8 (Y184A) | | |
| hCCR8 (S185A) | | |
| hCCR8 (F186A) | | |
| hCCR8 (Y187A) | | |
| hCCR8 (N188A) | Δ | Δ |
| hCCR8 (Q189A) | | |
| hCCR8 (Q190A) | | |
| hCCR8 (T191A) | | |
| hCCR8 (L192A) | | |
| hCCR8 (K193A) | | Δ |
| hCCR8 (W194A) | | |
| hCCR8 (K195A) | | |
| hCCR8 (I196A) | | |
| hCCR8 (F197A) | | |
| hCCR8 (T198A) | | |
| hCCR8 (N199A) | | |
| hCCR8 (F200A) | | |

### [INDUSTRIAL APPLICABILITY]

The monoclonal antibody or an antibody fragment thereof of the present invention can be used for detecting CCR8 in a biological sample. Furthermore, the pharmaceutical composition comprising the monoclonal antibody or an antibody fragment thereof of the present invention is very useful as a medicament for the treatment or prevention of CCR8-related diseases.

## Claims

1. A monoclonal antibody or an antibody fragment thereof that binds to CCR8, which recognizes tyrosine at position 17 in the amino acid sequence set forth in SEQ ID NO: 1.

2. The antibody or an antibody fragment thereof according to claim 1, which is a neutralizing antibody.

3. The antibody or an antibody fragment thereof according to claims 1 or 2, which further recognizes one or more of amino acids at positions 94 to 107 in the amino acid sequence set forth in SEQ ID NO: 1.

4. The antibody or an antibody fragment thereof according to any one of claims 1 to 3, which further recognizes one or more of amino acids at positions 172 to 202 in the amino acid sequence set forth in SEQ ID NO: 1.

5. The antibody or an antibody fragment thereof according to any one of claims 1 to 4, which recognizes one or more amino acids of isoleucine at position 20, serine at position 22, lysine at position 35, leucine at position 181, cysteine at position 183, and asparagine at position 188 in the amino acid sequence set forth in SEQ ID NO: 1.

6. The antibody or an antibody fragment thereof according to any one of claims 1 to 5, which is a humanized monoclonal antibody or an antibody fragment thereof.

7. The monoclonal antibody or an antibody fragment thereof that binds to CCR8, having:
1) a light chain variable region including
a CDR1 having the amino acid sequence of SEQ ID NO: 2 optionally having deletion, substitution, insertion and/or addition of one or two amino acids,
a CDR2 having the amino acid sequence of SEQ ID NO: 3 optionally having deletion, substitution, insertion and/or addition of one or two amino acids, and
a CDR3 having the amino acid sequence of SEQ ID NO: 4 optionally having deletion, substitution, insertion and/or addition of one or two amino acids, and
a heavy chain variable region including
a CDR1 having the amino acid sequence of SEQ ID NO: 5 optionally having deletion, substitution, insertion and/or addition of one or two amino acids,
a CDR2 having the amino acid sequence of SEQ ID NO: 6 optionally having deletion, substitution, insertion and/or addition of one or two amino acids, and
a CDR3 having the amino acid sequence of SEQ ID NO: 7 optionally having deletion, substitution, insertion and/or addition of one or two amino acids;
2) a light chain variable region including
a CDR1 having the amino acid sequence of SEQ ID NO: 2 optionally having deletion, substitution, insertion and/or addition of one or two amino acids,
a CDR2 having the amino acid sequence of SEQ ID NO: 3 optionally having deletion, substitution, insertion and/or addition of one or two amino acids, and
a CDR3 having the amino acid sequence of SEQ ID NO: 4 optionally having deletion, substitution, insertion and/or addition of one or two amino acids, and
a heavy chain variable region including
a CDR1 having the amino acid sequence of SEQ ID NO: 8 optionally having deletion, substitution, insertion and/or addition of one or two amino acids,
a CDR2 having the amino acid sequence of SEQ ID NO: 6 optionally having deletion, substitution, insertion and/or addition of one or two amino acids, and
a CDR3 having the amino acid sequence of SEQ ID NO: 7 optionally having deletion, substitution, insertion and/or addition of one or two amino acids;
3) a light chain variable region including
a CDR1 having the amino acid sequence of SEQ ID NO: 2 optionally having deletion, substitution, insertion and/or addition of one or two amino acids,
a CDR2 having the amino acid sequence of SEQ ID NO: 9 optionally having deletion, substitution, insertion and/or addition of one or two amino acids, and
a CDR3 having the amino acid sequence of SEQ ID NO: 10 optionally having deletion, substitution, insertion and/or addition of one or two amino acids, and
a heavy chain variable region including
a CDR1 having the amino acid sequence of SEQ ID NO: 8 optionally having deletion, substitution, insertion and/or addition of one or two amino acids,
a CDR2 having the amino acid sequence of SEQ ID NO: 6 optionally having deletion, substitution, insertion and/or addition of one or two amino acids, and
a CDR3 having the amino acid sequence of SEQ ID NO: 11 optionally having deletion, substitution, insertion and/or addition of one or two amino acids;
4) a light chain variable region including
a CDR1 having the amino acid sequence of SEQ ID NO: 2 optionally having deletion, substitution, insertion and/or addition of one or two amino acids,
a CDR2 having the amino acid sequence of SEQ ID NO: 3 optionally having deletion, substitution, insertion and/or addition of one or two amino acids, and
a CDR3 having the amino acid sequence of SEQ ID NO: 10 optionally having deletion, substitution, insertion and/or addition of one or two amino acids, and
a heavy chain variable region including
a CDR1 having the amino acid sequence of SEQ ID NO: 12 optionally having deletion, substitution, insertion and/or addition of one or two amino acids,
a CDR2 having the amino acid sequence of SEQ ID NO: 6 optionally having deletion, substitution, insertion and/or addition of one or two amino acids, and
a CDR3 having the amino acid sequence of SEQ ID NO: 13 optionally having deletion, substitution, insertion and/or addition of one or two amino acids;
5) a light chain variable region including
a CDR1 having the amino acid sequence of SEQ ID NO: 14 optionally having deletion, substitution, insertion and/or addition of one or two amino acids,
a CDR2 having the amino acid sequence of SEQ ID NO: 15 optionally having deletion, substitution, insertion and/or addition of one or two amino acids, and
a CDR3 having the amino acid sequence of SEQ ID NO: 16 optionally having deletion, substitution, insertion and/or addition of one or two amino acids, and
a heavy chain variable region including
a CDR1 having the amino acid sequence of SEQ ID NO: 17 optionally having deletion, substitution, insertion and/or addition of one or two amino acids,
a CDR2 having the amino acid sequence of SEQ ID NO: 18 optionally having deletion, substitution, insertion and/or addition of one or two amino acids, and
a CDR3 having the amino acid sequence of SEQ ID NO: 19 optionally having deletion, substitution, insertion and/or addition of one or two amino acids; or
6) a light chain variable region including
a CDR1 having the amino acid sequence of SEQ ID NO: 20 optionally having deletion, substitution, insertion and/or addition of one or two amino acids,
a CDR2 having the amino acid sequence of SEQ ID NO: 21 optionally having deletion, substitution, insertion and/or addition of one or two amino acids, and
a CDR3 having the amino acid sequence of SEQ ID NO: 22 optionally having deletion, substitution, insertion and/or addition of one or two amino acids, and
a heavy chain variable region including
a CDR1 having the amino acid sequence of SEQ ID NO: 23 optionally having deletion, substitution, insertion and/or addition of one or two amino acids,
a CDR2 having the amino acid sequence of SEQ ID NO: 24 optionally having deletion, substitution, insertion and/or addition of one or two amino acids, and
a CDR3 having the amino acid sequence of SEQ ID NO: 25 optionally having deletion, substitution, insertion and/or addition of one or two amino acids.

8. The antibody or an antibody fragment thereof according to claim 7, having:
1) a light chain variable region including
a CDR1 having the amino acid sequence of SEQ ID NO: 2,
a CDR2 having the amino acid sequence of SEQ ID NO: 3, and
a CDR3 having the amino acid sequence of SEQ ID NO: 4, and
a heavy chain variable region including
a CDR1 having the amino acid sequence of SEQ ID NO: 5,
a CDR2 having the amino acid sequence of SEQ ID NO: 6, and
a CDR3 having the amino acid sequence of SEQ ID NO: 7,
wherein one or more of the following substitutions are optionally present:
A) substitution of asparagine with lysine at position 10 in the amino acid sequence of SEQ ID NO: 2,
B) substitution of glycine with glutamine, threonine, alanine, lysine, leucine or arginine at position 11 in the amino acid sequence of SEQ ID NO: 2,
C) substitution of asparagine with glutamine at position 4 in the amino acid sequence of SEQ ID NO: 3,
D) substitution of leucine with isoleucine at position 5 in the amino acid sequence of SEQ ID NO: 3,
E) substitution of leucine with isoleucine at position 4 in the amino acid sequence of SEQ ID NO: 4,
F) substitution of tyrosine with phenylalanine at position 6 in the amino acid sequence of SEQ ID NO: 4,
G) substitution of serine with threonine at position 16 in the amino acid sequence of SEQ ID NO: 6, and
H) substitution of aspartic acid with glutamic acid at position 19 in the amino acid sequence of SEQ ID NO: 6;
2) a light chain variable region including
a CDR1 having the amino acid sequence of SEQ ID NO: 2,
a CDR2 having the amino acid sequence of SEQ ID NO: 3, and
a CDR3 having the amino acid sequence of SEQ ID NO: 4, and
a heavy chain variable region including
a CDR1 having the amino acid sequence of SEQ ID NO: 8,
a CDR2 having the amino acid sequence of SEQ ID NO: 6, and
a CDR3 having the amino acid sequence of SEQ ID NO: 7;
3) a light chain variable region including
a CDR1 having the amino acid sequence of SEQ ID NO: 2,
a CDR2 having the amino acid sequence of SEQ ID NO: 9, and
a CDR3 having the amino acid sequence of SEQ ID NO: 10, and
a heavy chain variable region including
a CDR1 having the amino acid sequence of SEQ ID NO: 8,
a CDR2 having the amino acid sequence of SEQ ID NO: 6, and
a CDR3 having the amino acid sequence of SEQ ID NO: 11,
wherein one or more of the following substitutions are optionally present:
A) substitution of serine with threonine at position 2 in the amino acid sequence of SEQ ID NO: 2,
B) substitution of serine with threonine at position 3 in the amino acid sequence of SEQ ID NO: 2,
C) substitution of serine with threonine at position 5 in the amino acid sequence of SEQ ID NO: 2,
D) substitution of glutamine with asparagine at position 2 in the amino acid sequence of SEQ ID NO: 10,
E) substitution of threonine with serine at position 1 in the amino acid sequence of SEQ ID NO: 8,
F) substitution of alanine with valine at position 14 in the amino acid sequence of SEQ ID NO: 6,
G) substitution of lysine with arginine at position 18 in the amino acid sequence of SEQ ID NO: 6,
H) substitution of aspartic acid with glutamic acid at position 19 in the amino acid sequence of SEQ ID NO: 6,
I) substitution of asparagine with glutamine at position 5 in the amino acid sequence of SEQ ID NO: 11, and
J) substitution of tyrosine with phenylalanine at position 12 in the amino acid sequence of SEQ ID NO: 11;
4) a light chain variable region including
a CDR1 having the amino acid sequence of SEQ ID NO: 2,
a CDR2 having the amino acid sequence of SEQ ID NO: 3, and
a CDR3 having the amino acid sequence of SEQ ID NO: 10, and
a heavy chain variable region including
a CDR1 having the amino acid sequence of SEQ ID NO: 12,
a CDR2 having the amino acid sequence of SEQ ID NO: 6, and
a CDR3 having the amino acid sequence of SEQ ID NO: 13;
5) a light chain variable region including
a CDR1 having the amino acid sequence of SEQ ID NO: 14,
a CDR2 having the amino acid sequence of SEQ ID NO: 15, and
a CDR3 having the amino acid sequence of SEQ ID NO: 16, and
a heavy chain variable region including
a CDR1 having the amino acid sequence of SEQ ID NO: 17,
a CDR2 having the amino acid sequence of SEQ ID NO: 18, and
a CDR3 having the amino acid sequence of SEQ ID NO: 19; or
6) a light chain variable region including
a CDR1 having the amino acid sequence of SEQ ID NO: 20,
a CDR2 having the amino acid sequence of SEQ ID NO: 21, and
a CDR3 having the amino acid sequence of SEQ ID NO: 22, and
a heavy chain variable region including
a CDR1 having the amino acid sequence of SEQ ID NO: 23,
a CDR2 having the amino acid sequence of SEQ ID NO: 24, and
a CDR3 having the amino acid sequence of SEQ ID NO: 25.

9. The antibody or an antibody fragment thereof according to claim 8, having:
a light chain variable region including
a CDR1 having the amino acid sequence of SEQ ID NO: 2,
a CDR2 having the amino acid sequence of SEQ ID NO: 3, and
a CDR3 having the amino acid sequence of SEQ ID NO: 4, and
a heavy chain variable region including
a CDR1 having the amino acid sequence of SEQ ID NO: 5,
a CDR2 having the amino acid sequence of SEQ ID NO: 6, and
a CDR3 having the amino acid sequence of SEQ ID NO: 7,
wherein one or more of the following substitutions are present:
A) substitution of asparagine with lysine at position 10 in the amino acid sequence of SEQ ID NO: 2;
B) substitution of glycine with glutamine, threonine, alanine, lysine, leucine or arginine at position 11 in the amino acid sequence of SEQ ID NO: 2;
C) substitution of asparagine with glutamine at position 4 in the amino acid sequence of SEQ ID NO: 3;
D) substitution of leucine with isoleucine at position 5 in the amino acid sequence of SEQ ID NO: 3;
E) substitution of leucine with isoleucine at position 4 in the amino acid sequence of SEQ ID NO: 4;
F) substitution of tyrosine with phenylalanine at position 6 in the amino acid sequence of SEQ ID NO: 4;
G) substitution of serine with threonine at position 16 in the amino acid sequence of SEQ ID NO: 6; and
H) substitution of aspartic acid with glutamic acid at position 19 in the amino acid sequence of SEQ ID NO: 6.

10. The antibody or an antibody fragment thereof according to claim 9, wherein one or more of the following substitutions are present:
A) substitution of asparagine with lysine at position 10 in the amino acid sequence of SEQ ID NO: 2;
B) substitution of glycine with leucine or arginine at position 11 in the amino acid sequence of SEQ ID NO: 2; and
C) substitution of asparagine with glutamine at position 4 in the amino acid sequence of SEQ ID NO: 3.

11. The antibody or an antibody fragment thereof according to claims 9 or 10, wherein
asparagine at position 4 in the amino acid sequence of SEQ ID NO: 3 is substituted with glutamine; and
further, any one of the following substitutions are optionally present:
A) substitution of asparagine with lysine at position 10 in the amino acid sequence of SEQ ID NO: 2; and
B) substitution of glycine with leucine or arginine at position 11 in the amino acid sequence of SEQ ID NO: 2.

12. The antibody or an antibody fragment thereof according to claim 11, wherein asparagine at position 4 in the amino acid sequence of SEQ ID NO: 3 is substituted with glutamine; and glycine at position 11 in the amino acid sequence of SEQ ID NO: 2 is substituted with arginine.

13. The antibody or an antibody fragment thereof according to claim 8, having:
a light chain variable region including
a CDR1 having the amino acid sequence of SEQ ID NO: 2,
a CDR2 having the amino acid sequence of SEQ ID NO: 9, and
a CDR3 having the amino acid sequence of SEQ ID NO: 10, and
a heavy chain variable region including
a CDR1 having the amino acid sequence of SEQ ID NO: 8,
a CDR2 having the amino acid sequence of SEQ ID NO: 6, and
a CDR3 having the amino acid sequence of SEQ ID NO: 11,
wherein one or more of the following substitutions are present:
A) substitution of serine with threonine at position 2 in the amino acid sequence of SEQ ID NO: 2;
B) substitution of serine with threonine at position 3 in the amino acid sequence of SEQ ID NO: 2;
C) substitution of serine with threonine at position 5 in the amino acid sequence of SEQ ID NO: 2;
D) substitution of glutamine with asparagine at position 2 in the amino acid sequence of SEQ ID NO: 10;
E) substitution of threonine with serine at position 1 in the amino acid sequence of SEQ ID NO: 8;
F) substitution of alanine with valine at position 14 in the amino acid sequence of SEQ ID NO: 6;
G) substitution of lysine with arginine at position 18 in the amino acid sequence of SEQ ID NO: 6;
H) substitution of aspartic acid with glutamic acid at position 19 in the amino acid sequence of SEQ ID NO: 6;
I) substitution of asparagine with glutamine at position 5 in the amino acid sequence of SEQ ID NO: 11; and
J) substitution of tyrosine with phenylalanine at position 12 in the amino acid sequence of SEQ ID NO: 11.

14. The antibody or an antibody fragment thereof according to any one of claims 7 to 13, which is a humanized monoclonal antibody or an antibody fragment thereof.

15. The humanized monoclonal antibody or an antibody fragment thereof according to claim 14, having:
a light chain variable region having
the amino acid sequence of SEQ ID NO: 40, 42, 43, 44, 45, or 47, or
an amino acid sequence having 95% or more identity to the amino acid sequence of SEQ ID NO: 40, 42, 43, 44, 45, or 47; and
a heavy chain variable region having
the amino acid sequence of SEQ ID NO: 41 or 46, or
an amino acid sequence having 95% or more identity to the amino acid sequence of SEQ ID NO: 41 or 46.

16. The humanized monoclonal antibody or an antibody fragment thereof according to claims 14 or 15, having:
1) a light chain variable region having the amino acid sequence of SEQ ID NO: 40, and
a heavy chain variable region having the amino acid sequence of SEQ ID NO: 41;
2) a light chain variable region having the amino acid sequence of SEQ ID NO: 42, and
a heavy chain variable region having the amino acid sequence of SEQ ID NO: 41;
3) a light chain variable region having the amino acid sequence of SEQ ID NO: 43, and
a heavy chain variable region having the amino acid sequence of SEQ ID NO: 41;
4) a light chain variable region having the amino acid sequence of SEQ ID NO: 44, and
a heavy chain variable region having the amino acid sequence of SEQ ID NO: 41;
5) a light chain variable region having the amino acid sequence of SEQ ID NO: 45, and
a heavy chain variable region having the amino acid sequence of SEQ ID NO: 41;
6) a light chain variable region having the amino acid sequence of SEQ ID NO: 47, and
a heavy chain variable region having the amino acid sequence of SEQ ID NO: 41;
7) a light chain variable region having the amino acid sequence of SEQ ID NO: 40, and
a heavy chain variable region having the amino acid sequence of SEQ ID NO: 46;
8) a light chain variable region having the amino acid sequence of SEQ ID NO: 42, and
a heavy chain variable region having the amino acid sequence of SEQ ID NO: 46;
9) a light chain variable region having the amino acid sequence of SEQ ID NO: 43, and
a heavy chain variable region having the amino acid sequence of SEQ ID NO: 46;
10) a light chain variable region having the amino acid sequence of SEQ ID NO: 44, and
a heavy chain variable region having the amino acid sequence of SEQ ID NO: 46;
11) a light chain variable region having the amino acid sequence of SEQ ID NO: 45, and
a heavy chain variable region having the amino acid sequence of SEQ ID NO: 46; or
12) a light chain variable region having the amino acid sequence of SEQ ID NO: 47, and
a heavy chain variable region having the amino acid sequence of SEQ ID NO: 46,
wherein one or more of the following substitutions are optionally present:
A) substitution of asparagine with lysine at position 33 in the amino acid sequence of SEQ ID NO: 40, 42, 43, 44, 45 or 47;
B) substitution of glycine with glutamine, threonine, alanine, lysine, leucine or arginine at position 34 in the amino acid sequence of SEQ ID NO: 40, 42, 43, 44, 45 or 47;
C) substitution of asparagine with glutamine at position 58 in the amino acid sequence of SEQ ID NO: 40, 42, 43, 44, 45 or 47;
D) substitution of leucine with isoleucine at position 59 in the amino acid sequence of SEQ ID NO: 40, 42, 43, 44, 45 or 47;
E) substitution of leucine with isoleucine at position 97 in the amino acid sequence of SEQ ID NO: 40, 42, 43, 44, 45 or 47;
F) substitution of tyrosine with phenylalanine at position 99 in the amino acid sequence of SEQ ID NO: 40, 42, 43, 44, 45 or 47;
G) substitution of serine with threonine at position 65 in the amino acid sequence of SEQ ID NO: 41 or 46; and
H) substitution of aspartic acid with glutamic acid at position 68 in the amino acid sequence of SEQ ID NO: 41 or 46.

17. The humanized monoclonal antibody or an antibody fragment thereof according to claim 16, wherein one or more of the following substitutions are present:
A) substitution of asparagine with lysine at position 33 in the amino acid sequence of SEQ ID NO: 40, 42, 43, 44, 45 or 47;
B) substitution of glycine with glutamine, threonine, alanine, lysine, leucine or arginine at position 34 in the amino acid sequence of SEQ ID NO: 40, 42, 43, 44, 45 or 47;
C) substitution of asparagine with glutamine at position 58 in the amino acid sequence of SEQ ID NO: 40, 42, 43, 44, 45 or 47;
D) substitution of leucine with isoleucine at position 59 in the amino acid sequence of SEQ ID NO: 40, 42, 43, 44, 45 or 47;
E) substitution of leucine with isoleucine at position 97 in the amino acid sequence of SEQ ID NO: 40, 42, 43, 44, 45 or 47;
F) substitution of tyrosine with phenylalanine at position 99 in the amino acid sequence of SEQ ID NO: 40, 42, 43, 44, 45 or 47;
G) substitution of serine with threonine at position 65 in the amino acid sequence of SEQ ID NO: 41 or 46; and
H) substitution of aspartic acid with glutamic acid at position 68 in the amino acid sequence of SEQ ID NO: 41 or 46.

18. The humanized monoclonal antibody or an antibody fragment thereof according to claim 17, having
a light chain variable region having the amino acid sequence of SEQ ID NO: 40 or 42, and
a heavy chain variable region having the amino acid sequence of SEQ ID NO: 41,
wherein one or more of the following substitutions are present:
A) substitution of asparagine with lysine at position 33 in the amino acid sequence of SEQ ID NO: 40 or 42;
B) substitution of glycine with glutamine, threonine, alanine, lysine, leucine or arginine at position 34 in the amino acid sequence of SEQ ID NO: 40 or 42;
C) substitution of asparagine with glutamine at position 58 in the amino acid sequence of SEQ ID NO: 40 or 42;
D) substitution of leucine with isoleucine at position 59 in the amino acid sequence of SEQ ID NO: 40 or 42;
E) substitution of leucine with isoleucine at position 97 in the amino acid sequence of SEQ ID NO: 40 or 42;
F) substitution of tyrosine with phenylalanine at position 99 in the amino acid sequence of SEQ ID NO: 40 or 42;
G) substitution of serine with threonine at position 65 in the amino acid sequence of SEQ ID NO: 41; and
H) substitution of aspartic acid with glutamic acid at position 68 in the amino acid sequence of SEQ ID NO: 41.

19. The humanized monoclonal antibody or an antibody fragment thereof according to claim 17 or 18, having
a light chain variable region having the amino acid sequence of SEQ ID NO: 40 or 42, and
a heavy chain variable region having the amino acid sequence of SEQ ID NO: 41,
wherein one or more of the following substitutions are present:
A) substitution of asparagine with lysine at position 33 in the amino acid sequence of SEQ ID NO: 40 or 42;
B) substitution of glycine with leucine or arginine at position 34 in the amino acid sequence of SEQ ID NO: 40 or 42; and
C) substitution of asparagine with glutamine at position 58 in the amino acid sequence of SEQ ID NO: 40 or 42.

20. The humanized monoclonal antibody or an antibody fragment thereof according to any one of claim 17 to 19, having:
a light chain variable region having the amino acid sequence of SEQ ID NO: 40 or 42, and
a heavy chain variable region having the amino acid sequence of SEQ ID NO: 41,
wherein asparagine at position 58 in the amino acid sequence of SEQ ID NO: 40 or 42 is substituted with glutamine, and
further, any one of the following substitutions are optionally present:
A) substitution of asparagine with lysine at position 33 in the amino acid sequence of SEQ ID NO: 40 or 42; and
B) substitution of glycine with leucine or arginine at position 34 in the amino acid sequence of SEQ ID NO: 40 or 42.

21. The humanized monoclonal antibody or an antibody fragment thereof according to claim 20, having
a light chain variable region having the amino acid sequence of SEQ ID NO: 42, and
a heavy chain variable region having the amino acid sequence of SEQ ID NO: 41,
wherein asparagine at position 58 in the amino acid sequence of SEQ ID NO: 42 is substituted with glutamine, and glycine at position 34 in the amino acid sequence of SEQ ID NO: 42 is substituted with arginine.

22. The humanized monoclonal antibody or an antibody fragment thereof according to claim 20, having
a light chain variable region having the amino acid sequence of SEQ ID NO: 59, and a heavy chain variable region having the amino acid sequence of SEQ ID NO: 41.

23. The humanized monoclonal antibody or an antibody fragment thereof according to claim 14, having:
a light chain variable region having
the amino acid sequence of SEQ ID NO: 54, 55, or, 56, or
an amino acid sequence having 95% or more identity to the amino acid sequence of SEQ ID NO: 54, 55, or, 56; and
a heavy chain variable region having
the amino acid sequence of SEQ ID NO: 57 or 58, or
an amino acid sequence having 95% or more identity to the amino acid sequence of SEQ ID NO: 57 or 58.

24. The humanized monoclonal antibody or an antibody fragment thereof according to claims 14 or 23, having
1) a light chain variable region having the amino acid sequence of SEQ ID NO: 54, and
a heavy chain variable region having the amino acid sequence of SEQ ID NO: 57;
2) a light chain variable region having the amino acid sequence of SEQ ID NO: 55, and
a heavy chain variable region having the amino acid sequence of SEQ ID NO: 57;
3) a light chain variable region having the amino acid sequence of SEQ ID NO: 56, and
a heavy chain variable region having the amino acid sequence of SEQ ID NO: 57;
4) a light chain variable region having the amino acid sequence of SEQ ID NO: 54, and
a heavy chain variable region having the amino acid sequence of SEQ ID NO: 58;
5) a light chain variable region having the amino acid sequence of SEQ ID NO: 55, and
a heavy chain variable region having the amino acid sequence of SEQ ID NO: 58;
6) a light chain variable region having the amino acid sequence of SEQ ID NO: 56, and
a heavy chain variable region having the amino acid sequence of SEQ ID NO: 58,
wherein one or more of the following substitutions are optionally present:
A) substitution of serine with threonine at position 25 in the amino acid sequence of SEQ ID NO: 54, 55 or 56;
B) substitution of serine with threonine at position 26 in the amino acid sequence of SEQ ID NO: 54, 55 or 56;
C) substitution of serine with threonine at position 28 in the amino acid sequence of SEQ ID NO: 54, 55 or 56;
D) substitution of glutamine with asparagine at position 95 in the amino acid sequence of SEQ ID NO: 54, 55 or 56;
E) substitution of threonine with serine at position 31 in the amino acid sequence of SEQ ID NO: 57 or 58;
F) substitution of alanine with valine at position 63 in the amino acid sequence of SEQ ID NO: 57 or 58;
G) substitution of lysine with arginine at position 67 in the amino acid sequence of SEQ ID NO: 57 or 58;
H) substitution of aspartic acid with glutamic acid at position 68 in the amino acid sequence of SEQ ID NO: 57 or 58;
I) substitution of asparagine with glutamine at position 99 in the amino acid sequence of SEQ ID NO: 57 or 58; and
J) substitution of tyrosine with phenylalanine at position 105 in the amino acid sequence of SEQ ID NO: 57 or 58.

25. The humanized monoclonal antibody or an antibody fragment thereof according to claim 24, having a light chain variable region having the amino acid sequence of SEQ ID NO: 56, and a heavy chain variable region having the amino acid sequence of SEQ ID NO: 57, wherein one or more of the following substitutions are optionally present:
A) substitution of serine with threonine at position 25 in the amino acid sequence of SEQ ID NO: 56;
B) substitution of serine with threonine at position 26 in the amino acid sequence of SEQ ID NO: 56;
C) substitution of serine with threonine at position 28 in the amino acid sequence of SEQ ID NO: 56;
D) substitution of glutamine with asparagine at position 95 in the amino acid sequence of SEQ ID NO: 56;
E) substitution of threonine with serine at position 31 in the amino acid sequence of SEQ ID NO: 57;
F) substitution of alanine with valine at position 63 in the amino acid sequence of SEQ ID NO: 57;
G) substitution of lysine with arginine at position 67 in the amino acid sequence of SEQ ID NO: 57;
H) substitution of aspartic acid with glutamic acid at position 68 in the amino acid sequence of SEQ ID NO: 57;
I) substitution of asparagine with glutamine at position 99 in the amino acid sequence of SEQ ID NO: 57; and
J) substitution of tyrosine with phenylalanine at position 105 in the amino acid sequence of SEQ ID NO: 57.

26. The humanized monoclonal antibody or an antibody fragment thereof according to any one of claims 14 to 25, further having:
a light chain constant region having the amino acid sequence of SEQ ID NO: 52, and
a heavy chain constant region having the amino acid sequence of SEQ ID NO: 53,
wherein lysine is optionally added to the C-terminal end of SEQ ID NO: 53.

27. A pharmaceutical composition comprising the antibody or an antibody fragment thereof according to any one of claims 1 to 26.

28. A pharmaceutical composition comprising the antibody or an antibody fragment thereof according to any one of claims 1 to 26, for use in inhibiting a binding of CCR8 to a CCR8 ligand.

29. A pharmaceutical composition comprising the antibody or an antibody fragment thereof according to any one of claims 1 to 26, for use as a neutralizing antibody.

30. A pharmaceutical composition comprising the monoclonal antibody or an antibody fragment thereof according to any one of claims 1 to 26 that binds to CCR8, for use in recognizing tyrosine at position 17 in the amino acid sequence set forth in SEQ ID NO: 1.

31. The pharmaceutical composition according to claim 30, for use in inhibiting a binding of CCR8 to a CCR8 ligand.

32. The pharmaceutical composition according to any one of claims 27 to 31, wherein the antibody has ADCC activity.

33. The pharmaceutical composition according to any one of claims 27 to 31, wherein the antibody is an IgG antibody.

34. The pharmaceutical composition according to any one of claims 27 to 33, for use in treating cancer.

35. A polynucleotide encoding a light chain variable region or a heavy chain variable region of the antibody according to any one of claims 7 to 24.

36. An expression vector comprising the polynucleotide according to claim 35.
